# EUROPÄISCHE PATENTSCHRIFT

(11) **EP 4 321 724 B1**
(45) Veröffentlichungstag und Bekanntmachung des Hinweises auf die Patenterteilung: **14.10.2026**
(21) Anmeldenummer: 23219420.9
(22) Anmeldetag: 28.02.2018
(51) Int. Cl.: E06B 9/78, A41D 13/06, A43C 11/16

(54) **VERSCHLUSSVORRICHTUNG MIT EINEM WICKELELEMENT**
LOCKING DEVICE WITH A WINDING ELEMENT
DISPOSITIF DE FERMETURE DOTÉ D'UN ÉLÉMENT D'ENROULEMENT

(30) Priorität: 28.02.2017 DE 102017203263; 14.11.2017 DE 102017220304; 23.01.2018 DE 102018201019
(43) Veröffentlichungstag der Anmeldung: 14.02.2024
(60) Teilanmeldung: 26203229.5
(62) Dokumentnummer(n) der früheren Anmeldung(en) nach Art. 76 EPÜ: 18710796.6 / 3 589 153
(73) Patentinhaber: Fidlock GmbH, 30659 Hannover (DE)
(72) Erfinder: FIEDLER, Joachim, 30519 Hannover (DE); BOTKUS, Breido, 30175 Hannover (DE); BUETTNER, Heiko, 30659 Hannover (DE)
(74) Vertreter: Maikowski & Ninnemann Patentanwälte Partnerschaft mbB

(56) Entgegenhaltungen:
- WO-A1-2015/006616
- DE-A1- 102012 218 344
- US-A1- 2012 004 587
- US-A1- 2016 058 130
- US-A1- 2016 354 223

## Beschreibung

Die Erfindung betrifft eine Verschlussvorrichtung nach dem Oberbegriff des Anspruchs 1.

Eine derartige Verschlussvorrichtung umfasst ein erstes Verschlussteil und ein zweites Verschlussteil, die entlang einer Schließrichtung aneinander angesetzt werden können, in einer Schließstellung aneinander gehalten sind und zum Öffnen der Verschlussvorrichtung voneinander gelöst werden können.

Eine derartige Verschlussvorrichtung dient generell zum Verbinden zweier Teile miteinander. Beispielsweise kann eine solche Verschlussvorrichtung einen Verschluss für einen Behälter, zum Beispiel eine Tasche oder einen Rucksack, bereitstellen. Eine solche Verschlussvorrichtung kann beispielsweise aber auch als Verschluss für einen Schuh, z.B. einen Sportschuh, dienen. Ganz generell kann die Verschlussvorrichtung zum belastbaren Verbinden zweier beliebiger Baugruppen miteinander dienen.

Wünschenswert kann hierbei sein, dass eine solche Verschlussvorrichtung nicht nur zum lösbaren Verbinden zweier Teile miteinander verwendet werden kann, sondern auch ein Spannen ermöglicht. Beispielsweise bei einem Verschluss für einen Rucksack oder bei einem Verschluss für einen Schuh kann wünschenswert sein, dass Teile zum einen aneinander angesetzt, zum anderen aber auch zueinander gespannt werden können.

Eine Spanneinrichtung mit einem auf ein Wickelelement aufwickelbaren Zugelement ist beispielsweise in der WO 2015/006616 A1, die den Oberbegriff des Anspruchs 1 offenbart, beschrieben.

Andere Bauformen von Wickeleinrichtungen sind aus der US 2016/354223 A1, der DE 10 2012 218 344 A1 und der US 2012/004587 A1 bekannt.

Aufgabe der vorliegenden Erfindung ist es, eine Verschlussvorrichtung zur Verfügung zu stellen, die einerseits ein lösbares Verbinden von Baugruppen miteinander, andererseits aber auch ein Spannen der Baugruppen zueinander ermöglicht.

Diese Aufgabe wird durch einen Gegenstand mit den Merkmalen des Anspruchs 1 gelöst. Demnach weist das zweite Verschlussteil ein Wickelelement auf, an dem ein Zugelement anordbar ist und das relativ zum ersten Verschlussteil zum Aufwickeln des Zugelements auf das Wickelelement in eine Wickelrichtung verdrehbar ist.

Mit der vorgeschlagenen Verschlussvorrichtung werden ein Verschluss zum lösbaren Verbinden zweier Teile miteinander und eine Spanneinrichtung miteinander kombiniert. Einerseits weist die Verschlussvorrichtung zwei Verschlussteile auf, die entlang einer Schließrichtung aneinander angesetzt werden können und in einer Schließstellung aneinandergehalten sind, sodass den Verschlussteilen zugeordnete Baugruppen über die Verschlussteile und deren Halt aneinander miteinander verbunden sind und durch Trennen der Verschlussteile voneinander die Baugruppen auch wieder voneinander gelöst werden können. Andererseits weist das zweite Verschlussteil ein Wickelelement auf, an dem ein Zugelement angeordnet werden kann. Das Wickelelement kann beispielsweise die Form einer zylindrischen Rolle aufweisen und eine Wickelrille tragen, in der das Zugelement aufgenommen werden kann. Durch Verdrehen des Wickelelements kann somit das Zugelement aufgewickelt und damit gespannt werden.

Während das erste Verschlussteil an einer ersten Baugruppe angeordnet sein kann, kann das zweite Verschlussteil über das Zugelement mit einer zweiten Baugruppe verbunden sein, wobei durch Aufwickeln des Zugelements auf das Wickelelement die erste Baugruppe und die zweite Baugruppe zueinander gespannt werden können.

Bei dem Zugelement kann es sich beispielsweise um ein biegeschlaffes Element handeln, dass (ausschließlich) zum Übertragen von Zugkräften geeignet ist. Bei dem Zugelement kann es sich beispielsweise um ein Seil, ein Gurt, ein Band, einen Riemen, eine Kette oder ein (elektrisch leitfähiges) Kabel handeln.

Das Zugelement kann beispielsweise mit zwei Enden an dem Wickelelement befestigt werden, sodass durch Verdrehen des Wickelelements das Zugelement mit seinen zwei Enden auf das Wickelelement aufgewickelt werden kann. Denkbar und möglich ist aber auch, dass lediglich ein Ende des Zugelements an dem Wickelelement befestigt ist, um durch Verdrehen des Wickelelements lediglich dieses eine Ende aufzuwickeln. Denkbar und möglich ist zudem auch, dass ein Binnenabschnitt des Zugelements an dem Wickelelement angeordnet ist, um durch Verdrehen des Wickelelements das Zugelement aufzuwickeln. Und denkbar ist, dass mehrere unterschiedliche Zugelemente an dem Wickelelement angeordnet sind und über das Wickelelement aufgewickelt werden können.

Das Wickelelement ist in eine Wickelrichtung zu dem ersten Verschlussteil verdrehbar, um das Zugelement aufzuwickeln. Vorzugsweise ist die Wickelrichtung hierbei um die Schließrichtung gerichtet, sodass das Wickelelement um die Schließrichtung relativ zu dem ersten Verschlussteil verdrehbar ist. Die Verschlussteile können somit entlang der Schließrichtung aneinander angesetzt werden, und das Wickelelement kann um die Schließrichtung verdreht werden, um den Verschlussteilen zugeordnete Baugruppen zueinander zu verspannen.

In einer Ausgestaltung kann das Wickelelement in der Schließstellung relativ zu dem ersten Verschlussteil verdrehbar sein. Vorzugsweise ist das Wickelelement hierbei in der Schließstellung in die Wickelrichtung relativ zu dem ersten Verschlussteil verdrehbar, nicht aber entgegen der Wickelrichtung. Dies ermöglicht ein Aufwickeln des Zugelements auf das Wickelelement, wenn die Verschlussteile aneinander angesetzt sind. Nach Aufwickeln des Zugelements auf das Wickelelement verbleibt das Wickelelement in seiner eingenommenen Stellung. Eine Rückbewegung entgegen der Wickelrichtung zum Abwickeln des Zugelements ist gesperrt.

In anderer Ausgestaltung kann demgegenüber vorgesehen sein, dass bei aneinander angesetzten Verschlussteilen ein Verdrehen des Wickelelements zu dem ersten Verschlussteil nicht mehr möglich ist. In diesem Fall ist somit eine Verdrehbewegung sowohl in die Wickelrichtung als auch entgegen der Wickelrichtung gesperrt, wenn die Verschlussteile aneinander angesetzt sind.

In einer Ausgestaltung weist das Wickelelement eine Verzahnungseinrichtung auf, die in der Schließstellung mit einer Verzahnungseinrichtung des ersten Verschlussteils in Eingriff steht. Eine solche Verzahnungseinrichtung kann z.B. durch eine entlang der Wickelrichtung umlaufende, zum Beispiel sägezahnförmige Verzahnung gebildet sein. Eine solche Verzahnungseinrichtung kann aber auch durch einzelne Verzahnungselemente, die keine durchgehende Verzahnung bilden, gebildet sein.

Mittels solcher Verzahnungseinrichtungen kann insbesondere eine Art Freilauf bereitgestellt werden, der ein Verdrehen des Wickelelements zu dem ersten Verschlussteil in die Wickelrichtung ermöglicht, wenn die Verschlussteile aneinander angesetzt sind und sich somit in der Schließstellung befinden, eine Bewegung entgegen der Wickelrichtung jedoch sperrt. Bei Verdrehen des Wickelelements zu dem ersten Verschlussteil gleitet die Verzahnungseinrichtung des Wickelelements über die Verzahnungseinrichtung des ersten Verschlussteils, sodass ein rastendes Bewegen des Wickelelements zu dem ersten Verschlussteil in die Wickelrichtung möglich ist. Bei einer Belastung entgegen der Wickelrichtung gelangen Verzahnungselemente der Verzahnungseinrichtungen jedoch derart miteinander in Eingriff, dass eine Bewegung gesperrt ist und das Wickelelement somit in seiner gerade eingenommenen Stellung gehalten wird.

Die Verzahnungseinrichtungen können beispielsweise in axialer Richtung miteinander in Eingriff stehen. Bei einem Verdrehen des Wickelelements in die Wickelrichtung zu dem ersten Verschlussteil gleiten die Verzahnungseinrichtungen übereinander, beispielsweise indem sägezahnförmige Verzahnungselemente aufeinander aufgleiten. Sind das erste Verschlussteil und das zweite Verschlussteil verdrehbar zueinander gelagert und axial aneinander geführt, kann dies einhergehen mit einer (geringfügigen) axialen Bewegung des ersten Verschlussteils zu dem zweiten Verschlussteil.

Alternativ kann vorgesehen sein, dass zumindest eine der Verzahnungseinrichtungen zumindest ein Verzahnungselement aufweist, das bei Verdrehen des Wickelelements in die Wickelrichtung quer zur Wickelrichtung beiseite gedrängt werden kann. In diesem Fall kommt es somit nicht zu einer axialen Bewegung zwischen den Verschlussteilen, sondern die Verzahnungselemente einer der Verzahnungseinrichtungen werden beiseite gedrängt, wenn das Wickelelement in die Wickelrichtung relativ zu dem ersten Verschlussteil verdreht wird. Dies kann insbesondere sinnvoll sein, wenn das erste Verschlussteil und das zweite Verschlussteil in der Schließstellung mechanisch miteinander verrastet sind und somit nicht axial gegeneinander bewegt werden können.

Generell können das erste Verschlussteil und das zweite Verschlussteil in der Schließstellung zum Halten der Verschlussteile entgegen der Schließrichtung aneinander mechanisch miteinander verrastet sein. Hierzu kann eines der Verschlussteile beispielsweise eine Rasteinrichtung mit zumindest einem verstellbaren Rastelement aufweisen, das in einer verrasteten Stellung in einen Rasteingriff des anderen Verschlussteils eingreift und dadurch die Verschlussteile entgegen der Schließrichtung aneinander hält. Über die Rasteinrichtung wird somit eine mechanische Verrastung zwischen den Verschlussteilen hergestellt, wenn die Verschlussteile aneinander angesetzt sind. Über die Rasteinrichtung werden die Verschlussteile entgegen einer zur Schließrichtung entgegengesetzten Belastung aneinander gehalten, sodass ein Abnehmen der Verschlussteile voneinander nicht ohne Lösen der Verrastung möglich ist.

Vorzugsweise gelangt die Rasteinrichtung bei Ansetzen der Verschlussteile aneinander selbsttätig in die verrastete Stellung. Bei Ansetzen der Verschlussteile aneinander verrasten die Verschlussteile somit selbsttätig miteinander, sodass der Halt der Verschlussteile aneinander in der Schließstellung gesichert ist. Das Wickelelement kann hierbei gegebenenfalls trotz der Verrastung in die Wickelrichtung zu dem ersten Verschlussteil drehbar sein, sodass das Zugelement bei aneinander angesetzten Verschlussteilen auf das Wickelelement aufgewickelt werden kann.

Die Rasteinrichtung kann ein oder mehrere Rastelemente aufweisen. Diese sind beispielsweise in Richtung ihrer verrasteten Stellung federvorgespannt, sodass die Rastelemente bei Ansetzen der Verschlussteile aneinander vorzugsweise selbsttätig in Eingriff mit dem zugeordneten Rasteingriff des anderen Verschlussteils gelangen.

Um die Verrastung zwischen den Verschlussteile zu lösen und die Verschlussteile zum Öffnen der Verschlussvorrichtung voneinander trennen zu können, weist die Rasteinrichtung vorzugsweise ein Bedienelement auf, dass betätigt werden kann, um das zumindest eine Rastelement außer Eingriff mit dem Rasteingriff zu bringen. Das Bedienelement kann beispielsweise durch Drücken zu betätigen sein und weist beispielsweise ein Auflaufelement auf, das ausgebildet ist, bei Betätigung des Bedienelements auf ein Auflaufelement an einem das zumindest eine Rastelement in Richtung der verrasteten Stellung vorspannenden Vorspannelement aufzulaufen, um das zumindest eine Rastelement dadurch zu verstellen. Das Vorspannelement kann beispielsweise durch einen Federring gebildet sein, an dem einerseits die Rastelemente und andererseits Auflaufelemente geformt sind. Durch Auflaufen des Auflaufelements des Bedienelements auf die Auflaufelemente des Vorspannelements wird das ringförmige Vorspannelement verformt, sodass die Rastelemente bewegt und außer Eingriff mit dem Rasteingriff des anderen Verschlussteils gebracht werden.

Das Wickelelement kann beispielsweise einstückig mit einem Eingriffselement des zweiten Verschlussteils geformt sein, sodass das zweite Verschlussteil mit seinem Wickelelement im ganzen bewegt werden kann. Durch Verdrehen des zweiten Verschlussteils mit dem daran angeordneten Wickelelement kann somit das Zugelement auf das Wickelelement aufgewickelt werden, um das Zugelement in geeigneter Weise zu spannen. In diesem Fall sind die Verschlussteile beispielsweise drehbar aneinander gelagert, indem ein Zylinderbund eines der Verschlussteile in das andere der Verschlussteile eingreift und darüber eine Lagerung bereitstellt.

Der Zylinderbund kann hierbei axial an dem anderen Verschlussteil geführt sein, sodass die Verschlussteile axial zueinander verstellt und beispielsweise axial entlang der Schließrichtung aneinander angesetzt werden können.

In einer Ausgestaltung weist das zweite Verschlussteil ein Gehäuseelement auf, das in der Schließstellung an dem ersten Verschlussteil angeordnet ist und an dem das Wickelelement drehbar gelagert ist. In diesem Fall kann das Gehäuseelement beispielsweise drehfest an das erste Verschlussteil anzusetzen sein und ist beispielsweise, in der Schließstellung der Verschlussvorrichtung, fest an dem ersten Verschlussteil gehalten. Das Wickelelement ist zu dem Gehäuseelement drehbar, sodass durch Verdrehen des Wickelelements das Zugelement in die Wickelrichtung auf das Wickelelement aufgewickelt und somit gespannt werden kann.

In einer Ausgestaltung weist das zweite Verschlussteil eine Sperrbaugruppe aus, die in einem gesperrten Zustand das Wickelelement derart relativ zu dem Gehäuseelement sperrt, dass das Wickelelement in die Wickelrichtung, nicht aber entgegen der Wickelrichtung zu dem Gehäuseelement verdrehbar ist.

Die Sperrbaugruppe dient somit dazu, ein Verdrehen des Wickelelements relativ zu dem Gehäuseelement in die Wickelrichtung zuzulassen, ein Zurückdrehen des Wickelelements entgegen der Wickelrichtung aber zu sperren. Über die Sperrbaugruppe kann somit das Wickelelement nach Art eines Freilaufs in die Wickelrichtung verdreht werden, um das Zugelement aufzuwickeln, wobei nach Verdrehen das Wickelelement in seiner eingenommenen Stellung verbleibt und somit an dem Zugelement wirkende Zugkräfte über die Sperrbaugruppe aufgenommen werden können.

Vorzugsweise ist die Sperrbaugruppe aus dem gesperrten Zustand entsperrbar. Dies ermöglicht, das Wickelelement freizugeben, sodass das Wickelelement auch entgegen der Wickelrichtung zu dem Gehäuseelement verdreht werden kann, um das Zugelement von dem Wickelelement abzuwickeln. Durch Entsperren der Sperrbaugruppe kann das Zugelement somit entspannt werden, indem das Zugelement von dem Wickelelement abgewickelt wird.

In einer Ausgestaltung kann vorgesehen sein, dass die Sperrbaugruppe beim Öffnen der Verschlussvorrichtung selbsttätig entsperrt wird. Werden die Verschlussteile voneinander getrennt und dazu das Gehäuseelement des zweiten Verschlussteils von dem ersten Verschlussteil abgenommen, gelangt die Sperrbaugruppe selbsttätig in ihren entsperrten Zustand, sodass das Wickelelement freigegeben wird und somit das Zugelement von dem Wickelelement abgewickelt werden kann.

In einer Ausgestaltung weist die Sperrbaugruppe ein Betätigungselement auf, dass beispielsweise als Handgriff ausgestaltet sein kann und das drehbar an dem Gehäuseelement angeordnet ist. Über das Betätigungselement kann das Wickelelement zu verdrehen sein, wobei hierzu das Betätigungselement, abhängig vom Zustand der Sperrbaugruppe, in Wirkverbindung mit dem Wickelelement oder außer Wirkverbindung von dem Wickelelement ist.

Das Betätigungselement und das Gehäuseelement können beispielsweise über eine Freilaufeinrichtung miteinander wirkverbundenen sein. Über die Freilaufeinrichtung ist ein Verdrehen des Betätigungselements in die Wickelrichtung zu dem Gehäuseelement möglich, ein Verdrehen entgegen der Wickelrichtung jedoch gesperrt. Ist das Betätigungselement mit dem Wickelelement in Wirkverbindung, kann über das Betätigungselement somit das Wickelelement in die Wickelrichtung verdreht werden, nicht aber entgegen der Wickelrichtung.

In einer Ausgestaltung weist die Freilaufeinrichtung eine Verzahnung an dem Gehäuseelement oder dem Betätigungselement und zumindest ein verstellbares Sperrelement, zum Beispiel in Form einer Sperrklinke, an dem jeweils anderen Element auf. Die Verzahnung kann beispielsweise als Innenverzahnung an einem Zylinderbund des Gehäuseelements geformt sein, wobei in diesem Fall z.B. ein oder mehrere bewegliche (z.B. schwenkbare) Sperrelemente an dem Betätigungselement angeordnet sind. Bei einem Verdrehen des Betätigungselements in die Wickelrichtung gleiten die Sperrelemente über die Verzahnung, sodass ein Verdrehen des Betätigungselements in die Wickelrichtung möglich, ein umgekehrtes Verdrehen entgegen der Wickelrichtung jedoch durch den Eingriff der Sperrelemente in die Verzahnung gesperrt ist und das Betätigungselement damit nicht entgegen der Wickelrichtung zu dem Gehäuseelement verdreht werden kann.

Denkbar und möglich ist hierbei, dass das Betätigungselement zum Beispiel durch ein axiales Verstellen gegenüber dem Gehäuseelement entsperrt werden kann, indem die Sperrelemente außer Eingriff von der Verzahnung gebracht werden.

Um die Wirkverbindung zwischen dem Betätigungselement und dem Wickelelement herzustellen, kann das Betätigungselement eine erste Verzahnungseinrichtung aufweisen, während das Wickelelement eine zweite Verzahnungseinrichtung trägt. Die Verzahnungseinrichtungen können beispielsweise jeweils sägezahnförmig sein, wobei der Eingriff derart ist, dass das Wickelelement bei einem Verdrehen des Betätigungselements in die Wickelrichtung mitgenommen und somit ebenfalls in die Wickelrichtung zu dem Gehäuseelement verdreht wird. Bei Belastung an dem Wickelelement durch über das Zugelement eingeleitete Zugkräfte, die zu einer Drehmomentbelastung entgegen der Wickelrichtung führen, wird das Wickelelement, wenn die Verzahnungseinrichtungen in Eingriff miteinander stehen, über das Betätigungselement in Position zu dem Gehäuseelement gehalten, sodass das Zugelement nicht von dem Wickelelement abgewickelt werden kann.

Um das Wickelelement zum Abwickeln des Zugelements freizugeben, kann das Betätigungselement axial zu dem Gehäuseelement verstellbar sein, sodass die Verzahnungseinrichtungen am Betätigungselement einerseits und am Wickelelement andererseits außer Eingriff gelangen und somit das Wickelelement nicht länger über das Betätigungselement in Position gehalten wird. Das Wickelelement kann somit entgegen der Wickelrichtung zu dem Gehäuseelement verdreht und damit das Zugelement von dem Wickelelement abgewickelt werden.

Das Gehäuseelement ist, wenn die Verschlussteile aneinander angesetzt sind, an dem ersten Verschlussteil gehalten. Das Gehäuseelement kann hierbei, in der Schließstellung, mechanisch fest mit dem ersten Verschlussteil verbunden sein, beispielsweise indem das Gehäuseelement mit dem ersten Verschlussteil verrastet ist.

In einer Ausgestaltung kann das erste Verschlussteil beispielsweise zumindest ein erstes Hinterschnittelement aufweisen, während das Gehäuseelement des zweiten Verschlussteils zumindest ein zweites Hinterschnittelement umfasst. Bei Ansetzen der Verschlussteile aneinander gelangen die Hinterschnittelemente miteinander in Eingriff, sodass die Verschlussteile in der Schließstellung entgegen der Schließrichtung aneinander gehalten sind.

Die Hinterschnittelemente sind beispielsweise starr an einem Grundkörper des ersten Verschlussteils und an dem Gehäuseelement des zweiten Verschlussteils ausgebildet. Nach Ansetzen der Verschlussteile in die Schließrichtung aneinander sind die Hinterschnittelemente in einer Eingriffsrichtung quer zur Schließrichtung miteinander in Eingriff zu bringen, sodass die Hinterschnittelemente ineinander eingreifen und eine entgegen der Schließrichtung belastbare Verbindung zwischen den Verschlussteilen herstellen.

In einer Ausgestaltung weist das erste Verschlussteil zumindest zwei erste Hinterschnittelemente auf, die quer zur Schließrichtung versetzt zueinander an einem Grundkörper des ersten Verschlussteils angeordnet sind. Ebenso kann das Gehäuseelement des zweiten Verschlussteils zumindest zwei zweite Hinterschnittelemente aufweisen, die quer zur Schließrichtung versetzt zueinander an dem Gehäuseelement des zweiten Verschlussteils angeordnet sind und in der Schließstellung den ersten Hinterschnittelementen des ersten Verschlussteils zugeordnet sind und mit diesen in Eingriff stehen. Über eine Anordnung mehrerer Hinterschnittelemente an jedem Verschlussteil kann in der Schließstellung eine hochfeste, mechanisch belastbare Verbindung zwischen den Verschlussteilen geschaffen werden.

Beispielsweise können die ersten Hinterschnittelemente des ersten Verschlussteils sich diametral zur Drehachse des Wickelelements gegenüberstehen, und ebenso können sich die zweiten Hinterschnittelemente des Gehäuseelements des zweiten Verschlussteils diametral zur Drehachse des Wickelelements gegenüberstehen. Beidseitig des Wickelelements sind somit Hinterschnittelemente angeordnet, sodass in der Schließstellung zwischen den Verschlussteilen wirkende Kräfte wirkungsvoll aufgenommen und abgeleitet werden können.

Die Hinterschnittelemente können jeweils beispielsweise eine Bogenform oder eine V-Form aufweisen. Unter einer Bogenform soll hierbei jede gekrümmte Form verstanden werden, die beispielsweise auch abschnittsweise gerade erstreckte Abschnitte aufweisen kann und somit lediglich abschnittsweise gekrümmt ist. Eine V-Form kann beispielsweise durch zwei Hinterschnittelemente gebildet sein, die winklig zueinander angeordnet sind und somit eine V-Form ausbilden.

In einer Ausgestaltung weist eines der Verschlussteile ein Blockierelement auf, das zum Sichern des Eingriffs zwischen dem zumindest einen ersten Hinterschnittelement und dem zumindest einen zweiten Hinterschnittelement in der Schließstellung der Verschlussteile dient. Ein solches Blockierelement kann beispielsweise am ersten Verschlussteil angeordnet sein und wirkt in der Schließstellung derart blockierend zwischen den Verschlussteilen, dass der Eingriff zwischen den Hinterschnittelementen gesichert ist und die Hinterschnittelemente insbesondere nicht entgegen der Eingriffsrichtung außer Eingriff voneinander gelangen können.

Das Blockierelement kann beispielsweise, in einer Blockierstellung, einem Bauteil des anderen Verschlussteils zugewandt sein, sodass darüber die Hinterschnittelemente in Eingriff gehalten werden. Werden die Verschlussteile entgegen der Eingriffsrichtung belastet, so verhindert das Blockierelement, dass die Hinterschnittelemente außer Eingriff voneinander gelangen, sodass darüber der Halt der Verschlussteile aneinander gesichert ist.

Vorzugsweise ist das Blockierelement in Richtung seiner Blockierstellung federvorgespannt. Bei Schließen der Verschlussvorrichtung gelangt das Blockierelement somit selbsttätig in seine Blockierstellung, sodass in der Schließstellung die Verschlussteile in ihrer Verbindung zueinander gesichert sind.

Zum Öffnen kann das Blockierelement beispielsweise aus seiner Blockierstellung heraus verstellbar sein. Durch zum Beispiel manuelle Einwirkung auf das Blockierelement wird die Blockierung der Verschlussteile zueinander somit aufgehoben, sodass die Verschlussteile durch Trennen der Hinterschnittelemente voneinander gelöst werden können.

Alternativ kann auch vorgesehen sein, das Bauteil des anderen Verschlussteils, dem das Blockierelement in der Blockierstellung zugewandt ist, zu verstellen, sodass darüber die blockierende Wirkung des Blockierelements aufgehoben wird. Ist das Blockierelement beispielsweise an dem ersten Verschlussteil angeordnet, kann es sich bei dem Bauteil zum Beispiel um das Betätigungselement des zweiten Verschlussteils handeln, das zum Beispiel axial zu dem Gehäuseelement verstellt werden kann, um dadurch zum einen die Wirkverbindung zwischen dem Betätigungselement und dem Wickelelement aufzuheben und zum anderen das Betätigungselement aus seiner blockierenden Gegenüberlage zum Blockierelement zu bringen.

Die Verschlussvorrichtung ist magnetisch ausgebildet. Hierzu weisen das erste Verschlussteil und das zweite Verschlussteil jeweils zumindest ein Magnetelement auf, die sich bei Ansetzen der Verschlussteile aneinander zum Schließen der Verschlussvorrichtung magnetisch anziehend gegenüberstehen und das Schließen der Verschlussvorrichtung somit magnetisch unterstützen.

Ein Magnetelement kann hierbei durch einen Permanentmagneten oder auch durch einen magnetischen Anker, zum Beispiel aus einem ferromagnetischen Material, gebildet sein. Beispielsweise kann eines der Verschlussteile einen Permanentmagneten aufweisen, das magnetisch anziehend mit einem magnetischen Anker des anderen Verschlussteils zusammenwirkt. Denkbar ist aber auch, dass beide Verschlussteile jeweils einen Permanentmagneten oder auch eine Anordnung von mehreren Permanentmagneten aufweisen, die sich bei Ansetzen der Verschlussteile aneinander mit ungleichnamigen Polen gegenüberstehen und ein Ansetzen somit durch magnetische Anziehung unterstützen.

Die Betätigung des Betätigungselements kann händisch durch Verdrehen des Betätigungselements erfolgen. Denkbar und möglich sind aber auch Ausgestaltungen, bei denen ein Elektromotor zum Antreiben des Betätigungselements vorgesehen ist. Ein solcher Elektromotor kann beispielsweise an einer mit dem ersten Verschlussteil verbundenen Baugruppe ortsfest angeordnet sein und kann beispielsweise über ein geeignetes Getriebeelement, zum Beispiel eine Antriebsschnecke, mit einer Verzahnung des Betätigungselements in Eingriff gelangen, wenn sich die Verschlussvorrichtung in ihrer Schließstellung befindet. Über den Elektromotor kann somit das Betätigungselement verdreht werden.

Denkbar und möglich ist alternativ, die Verzahnungseinrichtung des ersten Verschlussteils elektromotorisch anzutreiben, um durch Verdrehen der Verzahnungseinrichtung des ersten Verschlussteils das Wickelelement zu verdrehen.

Am ersten Verschlussteil und am zweiten Verschlussteil können, in einer Ausgestaltung, jeweils ein oder mehrere elektrische Kontaktelemente angeordnet sein, sodass bei Schließen der Verschlussvorrichtung ein elektrischer Kontakt zwischen den Verschlussteilen hergestellt wird.

In einer weiteren Ausgestaltung kann die Verschlussvorrichtung ein Windenausgangselement aufweisen, zum Beispiel in Form einer Öse, die an dem zweiten Verschlussteil angeordnet und als zu dem Wickelelement und dem Betätigungselement zusätzliches Bauteil ausgebildet sein kann. Das Windenausgangselement kann beispielsweise frei gegenüber dem Wickelelement und/oder dem Betätigungselement verdrehbar sein und führt das Zugelement mit Bezug auf das Wickelelement, sodass das Zugelement in definierter Weise in das Wickelelement einläuft. Dies verhindert ein unkontrolliertes Abwickeln des Zugelements vom Wickelelement und insbesondere ein Verknoten des Zugelements beim Abwickeln.

Die hier beschriebene Verschlussvorrichtung ermöglicht ein lösbares Verbinden von Verschlussteilen in Kombination mit einer Spannmöglichkeit für ein Zugelement. Dies ermöglicht zum Beispiel, bei getrennten Verschlussteilen das Zugelement auf Zug vorzuspannen, um sodann die Verschlussvorrichtung zu schließen und in der Schließstellung der Verschlussvorrichtung durch Verdrehen des Wickelelements das Zugelement aufzuwickeln und nachzuspannen. Beispielsweise bei einem Schuh kann auf diese Weise das Zugelement (nach Art eines Schnürsenkels) bei getrennter Verschlussvorrichtung händisch durch Zug am Zugelement vorgespannt und sodann, bei geschlossener Verschlussvorrichtung, nachgespannt werden.

Zudem ermöglicht das Trennen der Verschlussteile, dass das mit dem Wickelelement verbundene Zugelement um einen Gegenstand herum gelegt werden kann, um mittels der Verschlussvorrichtung eine Baugruppe an einer anderen festzulegen. So kann das Zugelement bei geöffneter Verschlussvorrichtung beispielsweise um einen Mast oder auch einen Rahmen, zum Beispiel einen Fahrradrahmen, herum gelegt werden, um die Verschlussvorrichtung sodann zu schließen und das Zugelement zu spannen, sodass auf diese Weise eine Baugruppe an dem Mast oder dem Rahmen festgelegt werden kann.

Eine Verschlussvorrichtung der hier beschriebenen Art kann in ganz unterschiedlicher Weise Verwendung finden. So kann eine Verschlussvorrichtung der hier beschriebenen Art bei Taschen oder anderen Behältnissen wie Rücksäcken, Kisten oder Containern, bei Schuhen (insbesondere Sportschuhen wie Wanderschuhen, Skistiefeln oder dergleichen), bei Helmen, insbesondere Sporthelmen, oder bei medizinischen Hilfsmitteln wie zum Beispiel medizinischen Stützschienen oder dergleichen zum Einsatz kommen.

Beispielsweise können über eine Verschlussvorrichtung der hier beschriebenen Art Gurte an Säcken oder Taschen gespannt werden (sogenannter Kompressionsgurte). Ein Gürtel oder ein Hüftgurt eines Rucksacks oder Schulranzens kann über eine solche Verschlussvorrichtung geschlossen und gespannt werden. Und eine solche Verschlussvorrichtung kann an einer Kabeltrommel zum Aufwickeln eines elektrischen Kabels, wie zum Beispiel eines Kopfhörer- oder Ladekabels, zum Einsatz kommen.

Bei einem Helm kann über eine Verschlussvorrichtung der hier beschriebenen Art ein Gurt gespannt werden, oder ein Gegenstand kann an dem Helm befestigt werden, zum Beispiel eine Schutzbrille (wie eine Skibrille) oder dergleichen.

Eine solche Verschlussvorrichtung kann auch zum Verstauen und Befestigen von Zubehör oder Taschen in oder an Fahrzeugen (Fahrräder, PKW, LKW, Schiffe, Flugzeuge etc.) dienen, zum Beispiel als Spannvorrichtung an einem Fahrradgepäckträger.

Konkret kann eine solche Verschlussvorrichtung beispielsweise an einer um einen Fahrradrahmen spannbaren Halterung zum Festlegen einer Baugruppe, zum Beispiel einer Trinkflasche oder eines Behälters an dem Fahrradrahmen, zum Einsatz kommen.

Zudem kann eine solche Verschlussvorrichtung zum Spannen von Planen und Tüchern jeder Art dienen, zum Beispiel zum Spannen von Zeltplanen oder auch zum Spannen eines Sonnenschutzes.

Auch militärische Anwendungen sind denkbar und möglich. So kann eine Verschlussvorrichtung zum Spannen und Verstauen von Waffen und Munition verwendet werden.

Eine Verschlussvorrichtung der beschriebenen Art kann auch bei einem Tourniquet-Abbindesystem zum Einsatz kommen zum Abbinden stark blutender Wunden bei einem Patienten.

Der der Erfindung zugrunde liegende Gedanke soll nachfolgend anhand der in den Figuren dargestellten Ausführungsbeispiele näher erläutert werden. Es zeigen:
- Fig. 1A: eine Ansicht eines ersten Ausführungsbeispiels einer Verschlussvorrichtung;
- Fig. 1B: eine andere perspektivische Ansicht der Verschlussvorrichtung;
- Fig. 2A: eine Explosionsansicht der Verschlussvorrichtung;
- Fig. 2B: eine andere Explosionsansicht der Verschlussvorrichtung;
- Fig. 3A: die Verschlussvorrichtung vor Ansetzen der Verschlussteile aneinander;
- Fig. 3B: die Verschlussvorrichtung in einer Seitenansicht;
- Fig. 3C: eine Schnittansicht entlang der Linie A-A gemäß Fig. 3B;
- Fig. 3D: eine Schnittansicht entlang der Linie B-B gemäß Fig. 3B;
- Fig. 4A: eine Ansicht der Verschlussvorrichtung vor Ansetzen der Verschlussteile aneinander, bei entsperrtem Wickelelement;
- Fig. 4B: eine Seitenansicht der Verschlussvorrichtung;
- Fig. 4C: eine Schnittansicht entlang der Linie A-A gemäß Fig. 4B;
- Fig. 4D: eine Schnittansicht entlang der Linie B-B gemäß Fig. 4B;
- Fig. 5A: eine Ansicht der Verschlussvorrichtung bei Ansetzen der Verschlussteile aneinander;
- Fig. 5B: eine Seitenansicht der Anordnung gemäß Fig. 5A;
- Fig. 5C: eine Schnittansicht entlang der Linie A-A gemäß Fig. 5B;
- Fig. 5D: eine Schnittansicht entlang der Linie B-B gemäß Fig. 5B;
- Fig. 6A: eine Ansicht der Verschlussvorrichtung bei weiterem Ansetzen der Verschlussteile aneinander;
- Fig. 6B: eine Seitenansicht der Anordnung gemäß Fig. 6A;
- Fig. 6C: eine Schnittansicht entlang der Linie A-A gemäß Fig. 6B;
- Fig. 6D: eine Schnittansicht entlang der Linie B-B gemäß Fig. 6B;
- Fig. 7A: eine Ansicht der Verschlussvorrichtung bei weiterem Ansetzen der Verschlussteile aneinander;
- Fig. 7B: eine Seitenansicht der Anordnung gemäß Fig. 7A;
- Fig. 7C: eine Schnittansicht entlang der Linie A-A gemäß Fig. 7B;
- Fig. 7D: eine Schnittansicht entlang der Linie B-B gemäß Fig. 7B;
- Fig. 8A: eine Ansicht der Verschlussvorrichtung in einer Schließstellung bei aneinander angesetzten Verschlussteile;
- Fig. 8B: eine Seitenansicht der Anordnung gemäß Fig. 8A;
- Fig. 8C: eine Schnittansicht entlang der Linie A-A gemäß Fig. 8B;
- Fig. 8D: eine Schnittansicht entlang der Linie B-B gemäß Fig. 8B;
- Fig. 9A: eine Ansicht der Verschlussvorrichtung beim Öffnen;
- Fig. 9B: eine Seitenansicht der Anordnung gemäß Fig. 9A;
- Fig. 9C: eine Schnittansicht entlang der Linie A-A gemäß Fig. 9B;
- Fig. 9D: eine Schnittansicht entlang der Linie B-B gemäß Fig. 9B;
- Fig. 10A: eine Ansicht der Verschlussvorrichtung beim weiteren Öffnen;
- Fig. 10B: eine Seitenansicht der Anordnung gemäß Fig. 10A;
- Fig. 10C: eine Schnittansicht entlang der Linie A-A gemäß Fig. 10B;
- Fig. 10D: eine Schnittansicht entlang der Linie B-B gemäß Fig. 10B;
- Fig. 11A: eine Ansicht eines anderen Ausführungsbeispiels einer Verschlussvorrichtung;
- Fig. 11B: eine andere Ansicht der Verschlussvorrichtung;
- Fig. 12A: eine Draufsicht auf die Verschlussvorrichtung;
- Fig. 12B: eine Seitenansicht der Verschlussvorrichtung bei voneinander getrennten Verschlussteilen;
- Fig. 13A: eine Explosionsansicht der Verschlussvorrichtung;
- Fig. 13B: eine andere Explosionsansicht der Verschlussvorrichtung;
- Fig. 14A: eine Ansicht der Verschlussvorrichtung in einer Schließstellung;
- Fig. 14B: eine Seitenansicht der Anordnung gemäß Fig. 14A;
- Fig. 14C: eine Schnittansicht entlang der Linie A-A gemäß Fig. 14B;
- Fig. 14D: eine Teilschnittansicht der Verschlussvorrichtung;
- Fig. 15A: eine Ansicht der Verschlussvorrichtung beim Verdrehen eines zweiten Verschlussteils mit einem daran angeordneten Wickelelement zu einem ersten Verschlussteil;
- Fig. 15B: eine Seitenansicht der Anordnung gemäß Fig. 15A;
- Fig. 15C: eine Schnittansicht entlang der Linie A-A gemäß Fig. 15B;
- Fig. 15D: eine Teilschnittansicht der Verschlussvorrichtung;
- Fig. 16A: eine Ansicht der Verschlussvorrichtung beim weiteren Verdrehen des zweiten Verschlussteils relativ zum ersten Verschlussteil;
- Fig. 16B: eine Seitenansicht der Anordnung gemäß Fig. 16A;
- Fig. 16C: eine Schnittansicht entlang der Linie A-A gemäß Fig. 16B;
- Fig. 16D: eine Teilschnittansicht der Verschlussvorrichtung;
- Fig. 17A: eine Ansicht der Verschlussvorrichtung bei voneinander getrennten Verschlussteilen;
- Fig. 17B: eine Seitenansicht der Anordnung gemäß Fig. 17A;
- Fig. 17C: eine Schnittansicht entlang der Linie A-A gemäß Fig. 17B;
- Fig. 17D: eine Teilschnittansicht der Verschlussvorrichtung;
- Fig. 18A: eine Ansicht eines anderen Ausführungsbeispiels einer Verschlussvorrichtung;
- Fig. 18B: eine andere Ansicht der Verschlussvorrichtung;
- Fig. 19A: eine Ansicht eines wiederum anderen Ausführungsbeispiels einer Verschlussvorrichtung;
- Fig. 19B: eine andere Ansicht der Verschlussvorrichtung;
- Fig. 20A: eine Explosionsansicht der Verschlussvorrichtung;
- Fig. 20B: eine andere Explosionsansicht der Verschlussvorrichtung;
- Fig. 21A: eine Ansicht der Verschlussvorrichtung vor Ansetzen der Verschlussteile aneinander;
- Fig. 21B: eine teilweise geschnittene Draufsicht auf die Anordnung gemäß Fig. 21A;
- Fig. 21C: eine Schnittansicht entlang der Linie L-L gemäß Fig. 21B;
- Fig. 21D: eine Schnittansicht entlang der Linie M-M gemäß Fig. 21B;
- Fig. 22A: eine Ansicht der Verschlussvorrichtung beim Schließen;
- Fig. 22B: eine teilweise geschnittene Draufsicht auf die Anordnung gemäß Fig. 22A;
- Fig. 22C: eine Schnittansicht entlang der Linie F-F gemäß Fig. 22B;
- Fig. 22D: eine Schnittansicht entlang der Linie G-G gemäß Fig. 22B;
- Fig. 23A: eine Ansicht der Verschlussvorrichtung beim weiteren Schließen;
- Fig. 23B: eine teilweise geschnittene Draufsicht auf die Anordnung gemäß Fig. 23A;
- Fig. 23C: eine Schnittansicht entlang der Linie H-H gemäß Fig. 23B;
- Fig. 23D: eine Schnittansicht entlang der Linie I-I gemäß Fig. 23B;
- Fig. 24A: eine Ansicht der Verschlussvorrichtung in einer Schließstellung;
- Fig. 24B: eine teilweise geschnittene Draufsicht auf die Anordnung gemäß Fig. 24A;
- Fig. 24C: eine Schnittansicht entlang der Linie B-B gemäß Fig. 24B;
- Fig. 24D: eine Schnittansicht entlang der Linie C-C gemäß Fig. 24B;
- Fig. 25A: eine Ansicht der Verschlussvorrichtung beim Verdrehen der Verschlussteile zueinander;
- Fig. 25B: eine teilweise geschnittene Draufsicht auf die Anordnung gemäß Fig. 25A;
- Fig. 25C: eine Schnittansicht entlang der Linie J-J gemäß Fig. 25B;
- Fig. 25D: eine Schnittansicht entlang der Linie K-K gemäß Fig. 25B;
- Fig. 26A: eine Ansicht der Anordnung bei Betätigen eines Bedienelements zum Öffnen der Verschlussvorrichtung;
- Fig. 26B: eine teilweise geschnittene Draufsicht auf die Anordnung gemäß Fig. 26A;
- Fig. 26C: eine Schnittansicht entlang der Linie D-D gemäß Fig. 26B;
- Fig. 26D: eine Schnittansicht entlang der Linie E-E gemäß Fig. 26B;
- Fig. 27A: eine perspektivische Ansicht eines weiteren Ausführungsbeispiels einer Verschlussvorrichtung, in einer Schließstellung der Verschlussvorrichtung;
- Fig. 27B: eine Seitenansicht der Anordnung gemäß Fig. 27A;
- Fig. 27C: eine Draufsicht auf die Verschlussvorrichtung;
- Fig. 27D: eine Schnittansicht entlang der Linie A-A gemäß Fig. 27C;
- Fig. 28: eine Schnittansicht entlang der Linie A-A gemäß Fig. 27C eines modifizierten Ausführungsbeispiels;
- Fig. 29A: eine schematische Ansicht eines Ausführungsbeispiels von Verzahnungseinrichtungen;
- Fig. 29B: eine perspektivische Ansicht der Verzahnungseinrichtungen gemäß Fig. 29A;
- Fig. 30A: eine schematische Ansicht eines anderen Ausführungsbeispiels von Verzahnungseinrichtungen;
- Fig. 30B: eine perspektivische Ansicht der Verzahnungseinrichtungen gemäß Fig. 30A;
- Fig. 31A: eine schematische Ansicht eines wiederum anderen Ausführungsbeispiels von Verzahnungseinrichtungen;
- Fig. 31B: eine perspektivische Ansicht der Verzahnungseinrichtungen gemäß Fig. 31A;
- Fig. 32A: eine schematische Ansicht eines wiederum anderen Ausführungsbeispiels von Verzahnungseinrichtungen;
- Fig. 32B: eine perspektivische Ansicht der Verzahnungseinrichtungen gemäß Fig. 32A;
- Fig. 33A: eine schematische Ansicht eines wiederum anderen Ausführungsbeispiels von Verzahnungseinrichtungen;
- Fig. 33B: eine perspektivische Ansicht der Verzahnungseinrichtungen gemäß Fig. 33A;
- Fig. 34A: eine Ansicht eines Ausführungsbeispiel einer Verschlussvorrichtung, darstellend insbesondere eine Verzahnungseinrichtung am ersten Verschlussteil;
- Fig. 34B: eine Ansicht des Ausführungsbeispiels gemäß Fig. 34A, darstellend eine Verzahnungseinrichtung am zweiten Verschlussteil;
- Fig. 34C: eine Draufsicht auf die Verschlussvorrichtung;
- Fig. 34D: eine Schnittansicht entlang der Linie A-A gemäß Fig. 34C;
- Fig. 35: eine Ansicht eines Ausführungsbeispiels einer Anwendung der Verschlussvorrichtung zum Spannen eines Schuhs;
- Fig. 36: eine Ansicht eines anderen Ausführungsbeispiels einer Anwendung der Verschlussvorrichtung zum Spannen eines Schuhs;
- Fig. 37: eine Ansicht eines wiederum anderen Ausführungsbeispiels einer Anwendung der Verschlussvorrichtung zum Spannen eines Schuhs;
- Fig. 38: eine Ansicht des Ausführungsbeispiels gemäß Fig. 37 beim Spannen des Schuhs;
- Fig. 39: eine Ansicht eines wiederum anderen Ausführungsbeispiels einer Anwendung der Verschlussvorrichtung zum Spannen eines Schuhs;
- Fig. 40: eine Ansicht eines wiederum anderen Ausführungsbeispiels einer Anwendung der Verschlussvorrichtung zum Spannen eines Schuhs;
- Fig. 41: eine Ansicht eines wiederum anderen Ausführungsbeispiels einer Anwendung der Verschlussvorrichtung zum Spannen eines Schuhs;
- Fig. 42: eine Ansicht eines Ausführungsbeispiels der Verschlussvorrichtung zum Spannen eines Hosenbeins;
- Fig. 43: eine Ansicht eines Ausführungsbeispiels einer Anwendung der Verschlussvorrichtung zum Verspannen eines Hosenbeins mit einem Schuh;
- Fig. 44: eine Ansicht eines anderen Ausführungsbeispiels einer Anwendung der Verschlussvorrichtung zum Spannen eines Hosenbeins an einem Schuh;
- Fig. 45: eine Ansicht eines Ausführungsbeispiels einer Anwendung der Verschlussvorrichtung zum Schließen eines Bekleidungsstücks;
- Fig. 46: eine Ansicht des Ausführungsbeispiels gemäß Fig. 45, bei geschlossenem Bekleidungsstück;
- Fig. 47: eine Ansicht eines anderen Ausführungsbeispiels einer Anwendung der Verschlussvorrichtung zum Schließen eines Bekleidungsstücks;
- Fig. 48: eine Ansicht eines Ausführungsbeispiels einer Anwendung der Verschlussvorrichtung zum Schließen und Spannen eines medizinischen Hilfsmittels;
- Fig. 49: eine Ansicht des Ausführungsbeispiels gemäß Fig. 48, in geschlossenem und gespanntem Zustand;
- Fig. 50: eine Ansicht eines anderen Ausführungsbeispiels einer Anwendung der Verschlussvorrichtung zum Schließen und Spannen eines medizinischen Hilfsmittels;
- Fig. 51: eine Ansicht des Ausführungsbeispiels gemäß Fig. 50 in geschlossenem Zustand;
- Fig. 52: eine Ansicht eines Ausführungsbeispiels einer Anwendung der Verschlussvorrichtung zum Schließen und Spannen eines anderen medizinischen Hilfsmittels;
- Fig. 53: eine Ansicht eines Ausführungsbeispiels einer Anwendung der Verschlussvorrichtung zum Schließen und Spannen eines Riemens eines Helms;
- Fig. 54: eine Ansicht des Ausführungsbeispiels gemäß Fig. 53 bei geschlossenem Riemen;
- Fig. 55: eine Ansicht eines Ausführungsbeispiels einer Anwendung der Verschlussvorrichtung zum Schließen und Spannen eines Gurts eines Helms;
- Fig. 56: eine Ansicht des Ausführungsbeispiels gemäß Fig. 55, bei geschlossenem Gurt;
- Fig. 57: eine Ansicht eines Ausführungsbeispiel einer Anwendung der Verschlussvorrichtung zum Aufhängen einer Lampe;
- Fig. 58: eine Ansicht eines Ausführungsbeispiels einer Anwendung der Verschlussvorrichtung zum Aufhängen eines Bildes;
- Fig. 59: eine Ansicht eines Ausführungsbeispiels einer Anwendung der Verschlussvorrichtung zum Anschließen einer Lampe an eine Steckdose;
- Fig. 60: eine Ansicht des Ausführungsbeispiels gemäß Fig. 59, angeschlossen an eine Steckdose;
- Fig. 61: eine Ansicht des Ausführungsbeispiels gemäß Fig. 60, bei gespanntem Kabel;
- Fig. 62: eine Ansicht eines Ausführungsbeispiels einer Anwendung der Verschlussvorrichtung zum Spannen eines Rollos;
- Fig. 63: eine Ansicht eines anderen Ausführungsbeispiels einer Anwendung der Verschlussvorrichtung zum Spannen eines Rollos;
- Fig. 64: eine Ansicht eines Ausführungsbeispiels einer Anwendung der Verschlussvorrichtung zum Spannen eines Gurts an einem Gepäckstück;
- Fig. 65: eine Ansicht eines Ausführungsbeispiels einer Anwendung der Verschlussvorrichtung zum Spannen eines Zugelements an einem Rucksack;
- Fig. 66: eine Ansicht eines Ausführungsbeispiels einer Anwendung der Verschlussvorrichtung zum Befestigen eines Gegenstands an einem Fahrrad;
- Fig. 67: eine Ansicht eines anderen Ausführungsbeispiels einer Anwendung der Verschlussvorrichtung zum Befestigen eines Gegenstands an einem Fahrrad;
- Fig. 68: eine Ansicht eines Ausführungsbeispiels einer Anwendung der Verschlussvorrichtung zum Festlegen eines Gegenstands im Kofferraum eines Kraftfahrzeugs;
- Fig. 69: eine Ansicht eines weiteren Ausführungsbeispiels einer Verschlussvorrichtung mit einem Zugelement in Form eines Gurts;
- Fig. 70: eine Frontalansicht der Verschlussvorrichtung gemäß Fig. 69;
- Fig. 71: eine Seitenansicht der Verschlussvorrichtung;
- Fig. 72: eine Draufsicht auf die Verschlussvorrichtung;
- Fig. 73: eine Schnittansicht entlang der Linie A-A gemäß Fig. 72;
- Fig. 74A: eine Ansicht der Verschlussvorrichtung zum Verbinden zweier Gurtenden miteinander;
- Fig. 74B: eine Ansicht der Verschlussvorrichtung in geschlossenem Zustand;
- Fig. 75: eine Ansicht eines anderen Ausführungsbeispiels einer Verschlussvorrichtung;
- Fig. 76: eine Ansicht eines weiteren Ausführungsbeispiels einer Verschlussvorrichtung zum Verbinden zweier Gurtenden miteinander;
- Fig. 77: eine Ansicht der Verschlussvorrichtung in geschlossener Stellung;
- Fig. 78A: eine Draufsicht auf die Verschlussvorrichtung, mit einem Spannhebel in einer Stellung zum Ansetzen der Verschlussteile aneinander;
- Fig. 78B: eine vergrößerte Ansicht im Ausschnitt A gemäß Fig. 78A;
- Fig. 79A: eine Draufsicht auf die Verschlussvorrichtung beim Spannen eines Zugelements; und
- Fig. 79B: eine vergrößerte Ansicht im Ausschnitt A gemäß Fig. 79A.

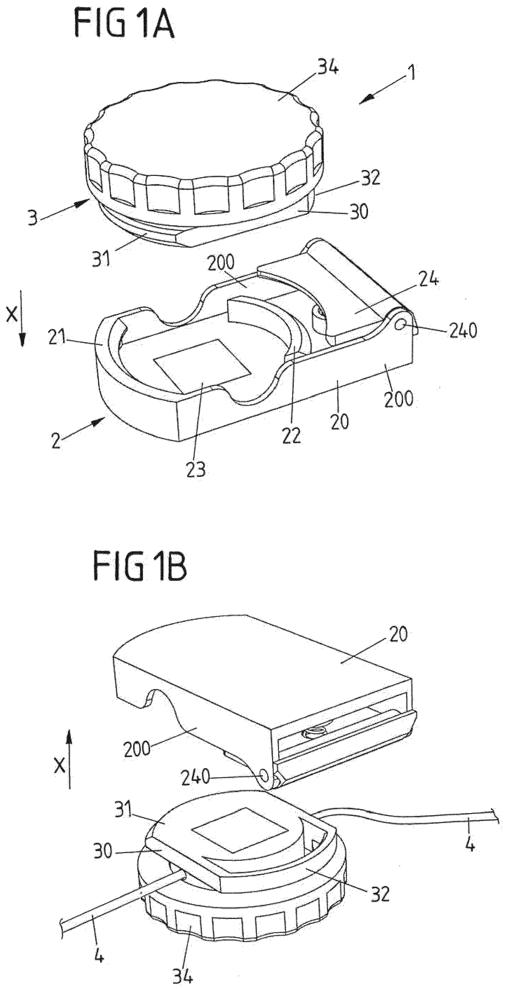

Fig. 1A, 1B bis 10A-10D zeigen ein erstes Ausführungsbeispiel einer Verschlussvorrichtung 1, die ein erstes Verschlussteil 2 und ein zweites Verschlussteil 3 aufweist. Die Verschlussteile 2, 3 können entlang einer Schließrichtung X aneinander angesetzt werden und sind in einer Schließstellung aneinander gehalten, sodass in der Schließstellung eine mechanisch feste Verbindung zwischen den Verschlussteile 2, 3 geschaffen ist.

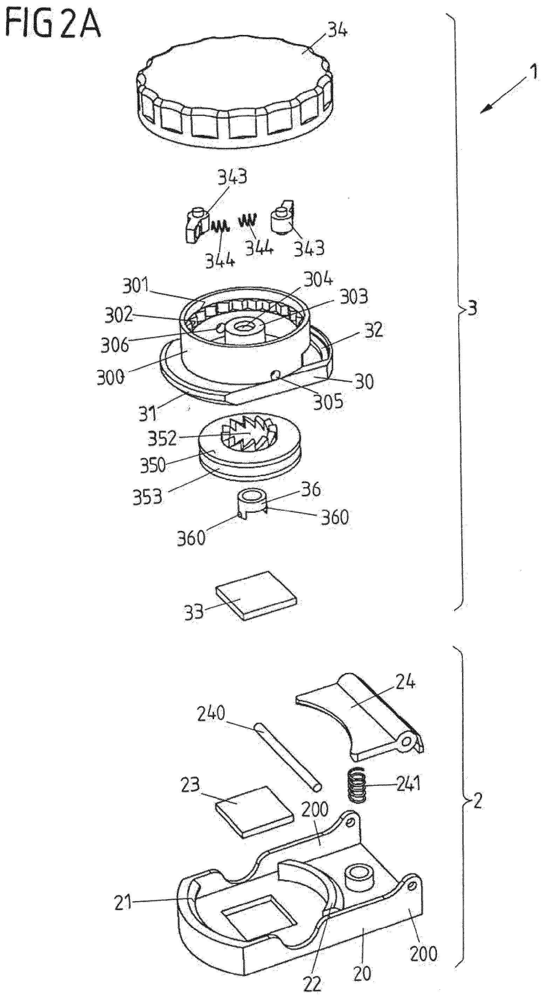

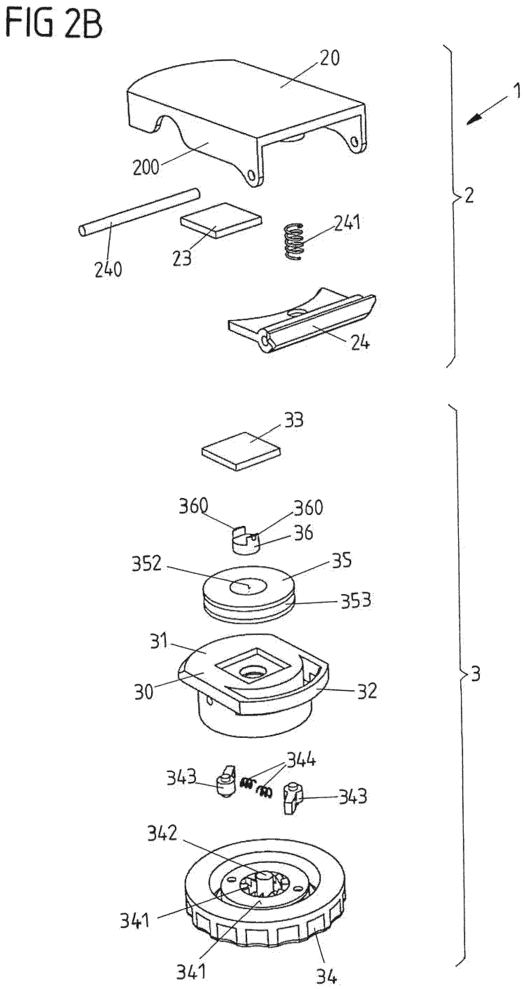

Das erste Verschlussteil 2 weist, wie insbesondere aus den Explosionsansichten gemäß Fig. 2A und 2B ersichtlich, einen Grundkörper 20 auf, an dem zwei starre Hinterschnittelemente 21, 22 mit jeweils bogenförmiger Gestalt angeordnet sind. An dem Grundkörper 20 ist zudem ein Blockierelement 24 angeordnet, das über ein Achselement 240 verschwenkbar zwischen Seitenwandungen 200 des Grundkörpers 20 gelagert ist und über ein Federelement 241 in eine Blockierstellung federvorgespannt ist, wie dies nachstehend noch erläutert werden soll.

Das zweite Verschlussteil 3 weist ein Gehäuseelement 30 auf, an dem zwei starre Hinterschnittelemente 31, 32 angeordnet sind, die den Hinterschnittelementen 21, 22 des ersten Verschlussteils 2 zugeordnet sind und in der Schließstellung mechanisch mit diesen Hinterschnittelementen 21, 22 in Eingriff stehen.

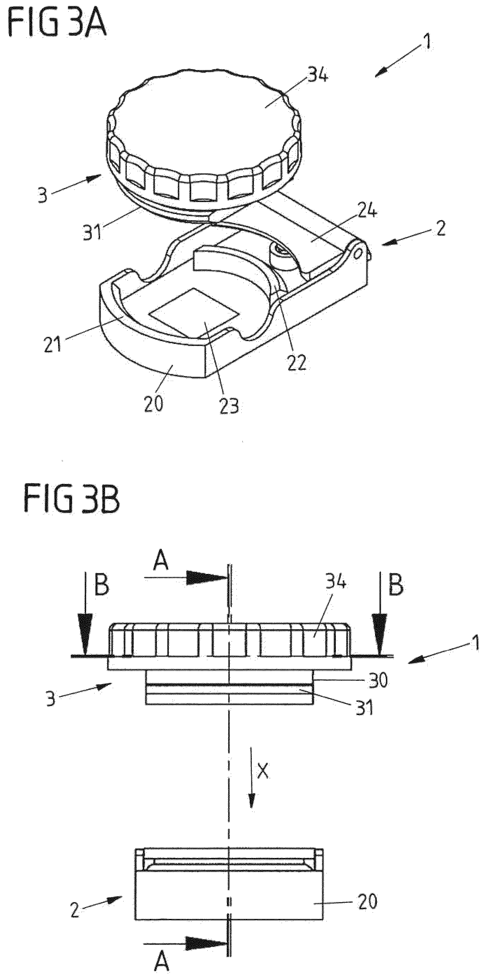

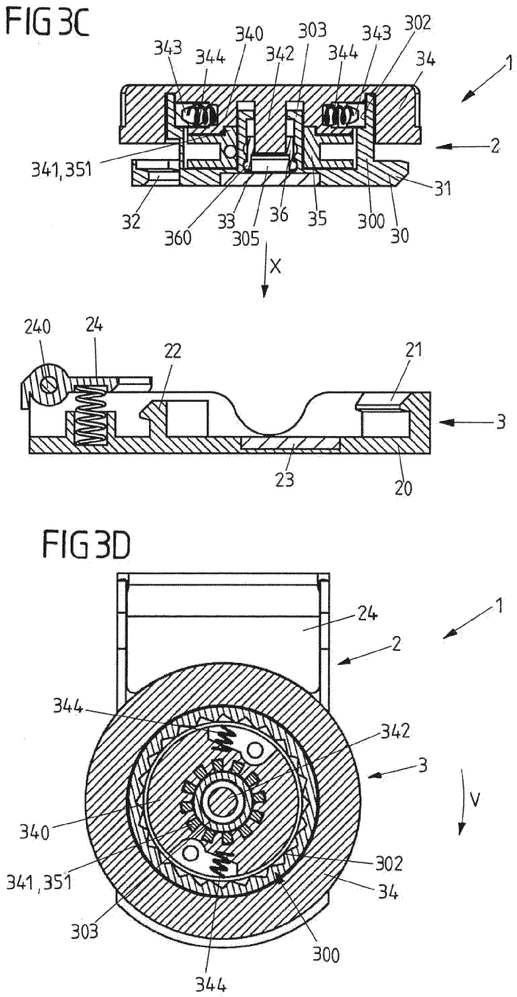

An dem Gehäuseelement 30 ist ein Zylinderbund 300 ausgebildet, der einen Innenraum 301 einfasst, in dem ein Wickelelement 35 um einen Lagerdom 303 drehbar gelagert ist. Ein Betätigungselement 34 in Form eines Griffelements greift mit einem Körper 340 in den Innenraum 301 ein und durchgreift mit einem Zapfen 342 eine Öffnung 304 des Lagerdoms 303 und ist innerhalb des Lagerdoms 303 mit einem Rastelement 36 verbunden (siehe zum Beispiel die Schnittansicht gemäß Fig. 3C).

Innen umlaufend an dem Zylinderbund 300 ist eine Verzahnung 302 gebildet, die mit Sperrelementen 343 zusammenwirkt, die über Federelemente 344 vorgespannt und innerhalb des Körpers 340 das Betätigungselements 34 beweglich gelagert sind (siehe zum Beispiel die Schnittansicht gemäß Fig. 3C). Wie nachstehend noch erläutert werden soll, bildet die Verzahnung 302 zusammen mit den Sperrelementen 343 einen Freilauf aus, der ein Verdrehen des Betätigungselements 34 zu dem Gehäuseelement 30 in eine Wickelrichtung V ermöglicht, eine entgegengesetzte Drehbewegung entgegen der Wickelrichtung V jedoch sperrt.

Das Betätigungselement 34 weist an einem stirnseitigen, dem Wickelelement 35 zugewandten Abschnitt des Körpers 340 eine Verzahnung 342 auf, die einer Verzahnung 351 des Wickelelements 35 zugeordneten ist und über die das Betätigungselement 34 in Wirkverbindung mit dem Wickelelement 35 bringbar ist. Besteht eine Wirkverbindung zwischen dem Betätigungselement 34 und dem Wickelelement 35, kann durch ein Verdrehen des Betätigungselements 34 das Wickelelement 35 verdreht werden, sodass ein Zugelement 4 (siehe Fig. 1B) in eine Rille 353 an einem Wickelkörper 350 des Wickelelements 35 gewickelt werden kann, um das über Öffnungen 305 in den Innenraum 301 hineingeführte Zugelement 4 auf den Wickelkörper 350 aufzuwickeln.

Das erste Verschlussteil 2 weist ein an dem Grundkörper 20 befestigtes, erstes Magnetelement 23 auf. Das zweite Verschlussteil 3 weist ein zweites Magnetelement 33 auf, das an dem Gehäuseelement 30 angeordnet ist und magnetisch mit dem ersten Magnetelement 23 des ersten Verschlussteils 2 zusammenwirkt. Die Magnetelemente 23, 33 können jeweils durch einen Permanentmagneten ausgebildet sein und sich mit ungleichnamigen Polen gegenüberstehen und somit magnetisch anziehend beim Ansetzen der Verschlussteile 2, 3 zusammenwirken. Alternativ kann eines der Magnetelemente 23, 33 durch einen Permanentmagneten verwirklicht sein, während das andere der Magnetelemente 33, 23 durch einen magnetischen Anker, zum Beispiel aus einem ferromagnetischen Material, gebildet ist und somit eine magnetische Anziehung zwischen den Magnetelementen 23, 33 besteht.

Fig. 3A-3D bis 10A-10D zeigen die Verschlussvorrichtung 1 in unterschiedlichen Stellungen.

Fig. 3A-3D zeigen die Verschlussvorrichtung 1 in einer geöffneten Stellung bei voneinander getrennten Verschlussteilen 2, 3. Wie aus der Schnittansicht gemäß Fig. 3C ersichtlich, steht in der dargestellten Stellung das Betätigungselement 34 über seine Verzahnung 341 mit der zugeordneten Verzahnung 351 des Wickelelements 35 in Eingriff, sodass eine Wirkverbindung zwischen dem Betätigungselement 34 und dem Wickelelement 35 besteht. In dieser axialen Stellung wird das (axial zu dem Gehäuseelement 30 verstellbare) Betätigungselement 34 durch rastenden Eingriff von Rastvorsprüngen 360 des Rastelements 36 in eine Rastnut 305 innerhalb des Lagerdoms 303 des Gehäuseelements 30 gehalten.

Wird das Betätigungselement 34 in eine Wickelrichtung V zu dem Gehäuseelement 30 verdreht, wird aufgrund der Wirkverbindung zwischen dem Betätigungselement 34 und dem Wickelelement 35 das Wickelelement 35 mit verdreht, sodass ein an dem Wickelelement 35 angeordnetes Zugelement 4 auf den Wickelkörper 350 des Wickelelements 35 aufgewickelt wird. Ein Verdrehen des Betätigungselements 34 ist hierbei möglich, weil die Rastelemente 343 bei Verdrehen des Betätigungselements 34 in die Wickelrichtung V über die Innenverzahnung 302 an dem Zylinderbund 300 des Gehäuseelements 30 gleiten und somit ein Verdrehen des Betätigungselements 34 in die Wickelrichtung V nicht gesperrt ist.

Nach einem Verdrehen verbleiben sowohl das Betätigungselement 34 als auch das damit wirkverbundene Wickelelement 35 in der eingenommenen Stellung. An dem Zugelement 4 wirkenden Zugkräfte können über das Wickelelement 35 und das Betätigungselement 34 über die nunmehr sperrend wirkenden Sperrelemente 343 in das Gehäuseelement 30 eingeleitet und dort aufgenommen werden.

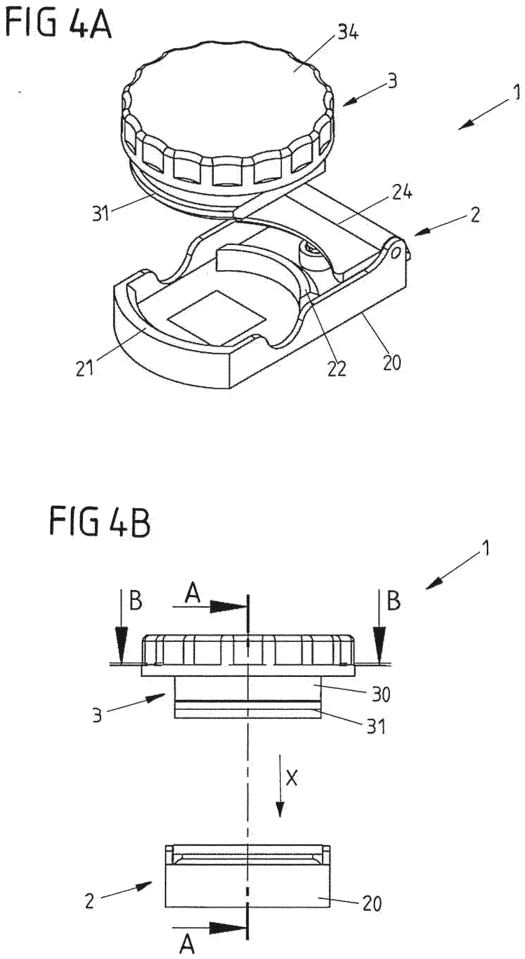

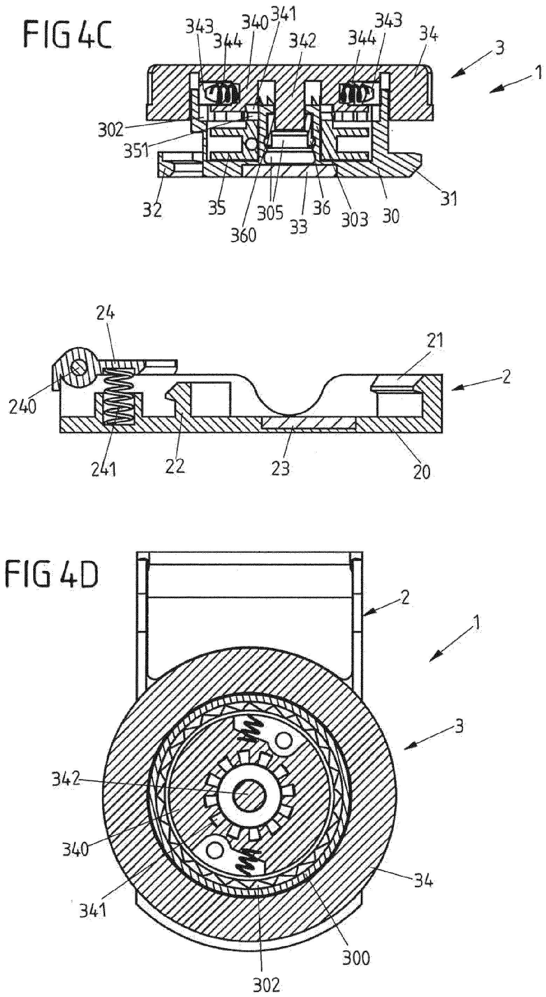

Um die Spannung an dem Zugelement 4 zu lösen, kann das Betätigungselement 34, wie dies in Fig. 4A-4D dargestellt ist, axial zu dem Lagerdom 303 des Gehäuseelements 30 verstellt werden, wodurch - wie aus Fig. 4C ersichtlich - die Rastelemente 360 in Eingriff mit einer oberen Rastnut 305 innerhalb des Lagerdoms 303 gelangen und somit das Betätigungselement 34 nunmehr in einer axial oberen Stellung zu dem Gehäuseelement 30 verrastet. Durch die axiale Bewegung des Betätigungselements 34 wird die Wirkverbindung zwischen den Verzahnungen 341, 351 des Betätigungselements 34 und des Wickelelements 35 aufgehoben, sodass das Betätigungselement 34 und das Wickelelement 35 außer Eingriff gelangen. Das Wickelelement 35 ist somit nicht mehr gesperrt und kann entgegen der Wickelrichtung V zu dem Gehäuseelement 30 bewegt werden, sodass das Zugelement 4 von dem Wickelelement 35 abgewickelt werden kann.

Durch axiale Bewegung des Betätigungselements 34 werden zudem die Sperrelemente 343 an dem Körper 340 axial relativ zu der Innenverzahnung 302 an dem Zylinderbund 300 des Gehäuseelements 30 verstellt, sodass darüber die Sperrwirkung zwischen dem Betätigungselement 34 und dem Gehäuseelement 30 aufgehoben wird. Auch das Betätigungselement 34 kann somit gegebenenfalls frei (auch entgegen der Wickelrichtung V) zu dem Gehäuseelement 30 verdreht werden.

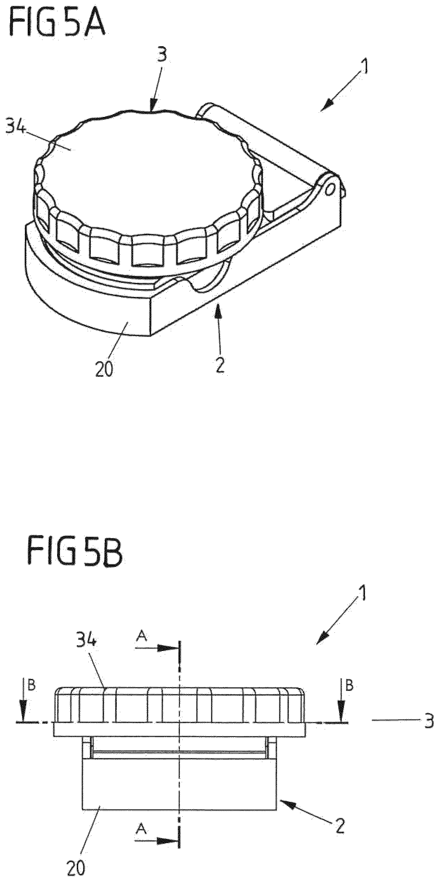

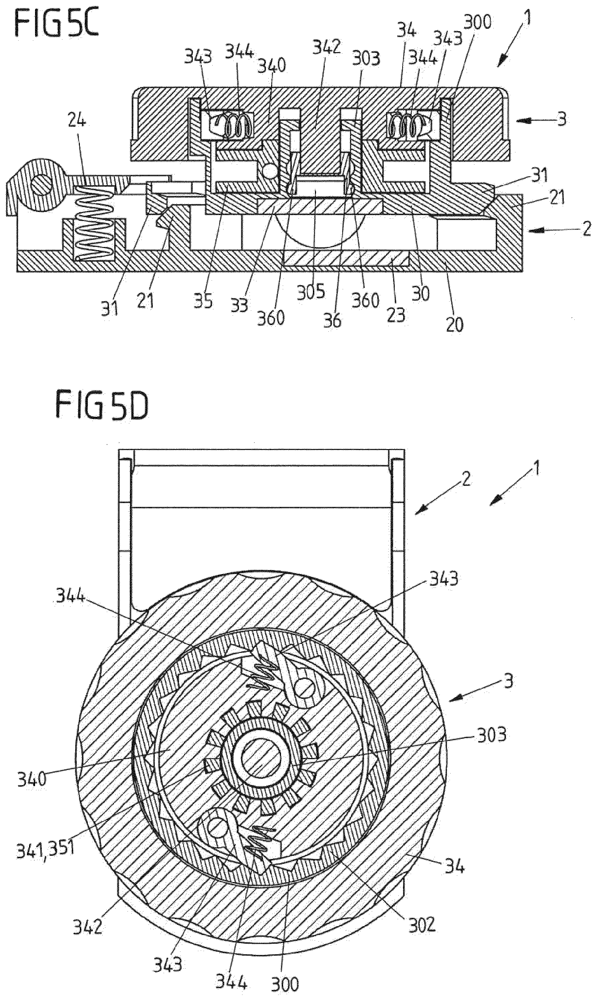

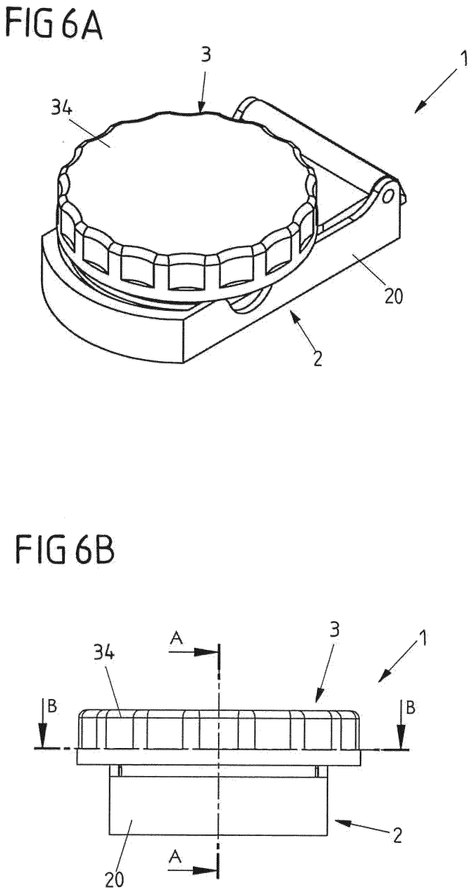

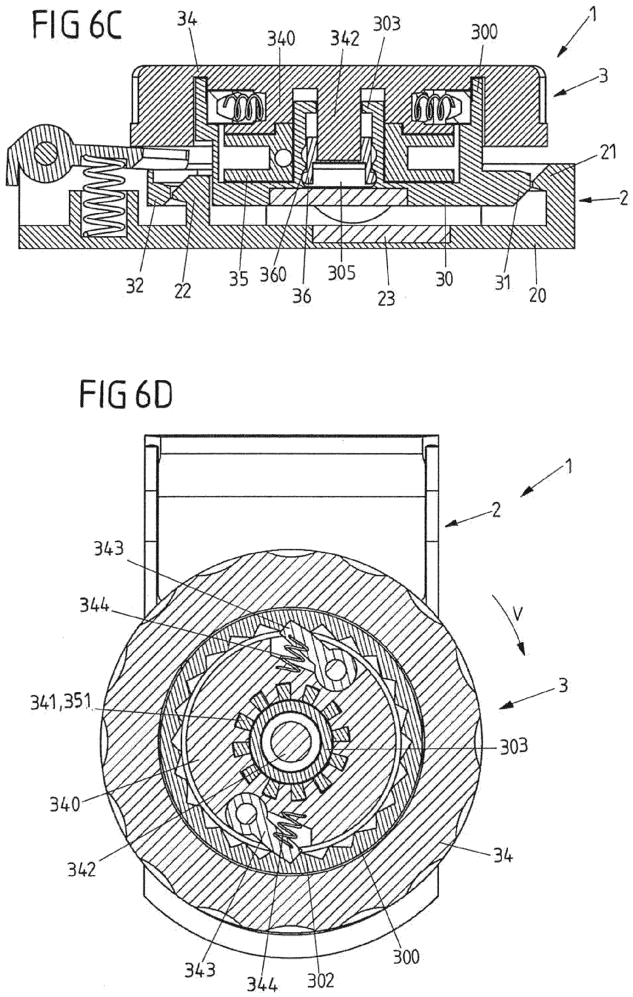

Fig. 5A-5D und 6A-6D zeigen die Verschlussvorrichtung 1 beim Ansetzen der Verschlussteile 2, 3 aneinander. Beim Ansetzen der Verschlussteile 2, 3 aneinander laufen die Hinterschnittelemente 21, 22, 31, 32 am Grundkörper 20 des ersten Verschlussteils 2 und an dem Gehäuseelement 30 des zweiten Verschlussteils 3 aufeinander auf und werden dadurch seitlich derart zueinander versetzt, dass die Hinterschnittelemente 21, 22, 31, 32 in die Schließrichtung X aneinander vorbei bewegt werden können, um in Eingriff miteinander gebracht zu werden.

Das Ansetzen der Verschlussteile 2, 3 wird hierbei durch Wirkung der Magnetelemente 23, 33 magnetisch unterstützt, sodass das Ansetzen weitestgehend selbsttätig erfolgen kann.

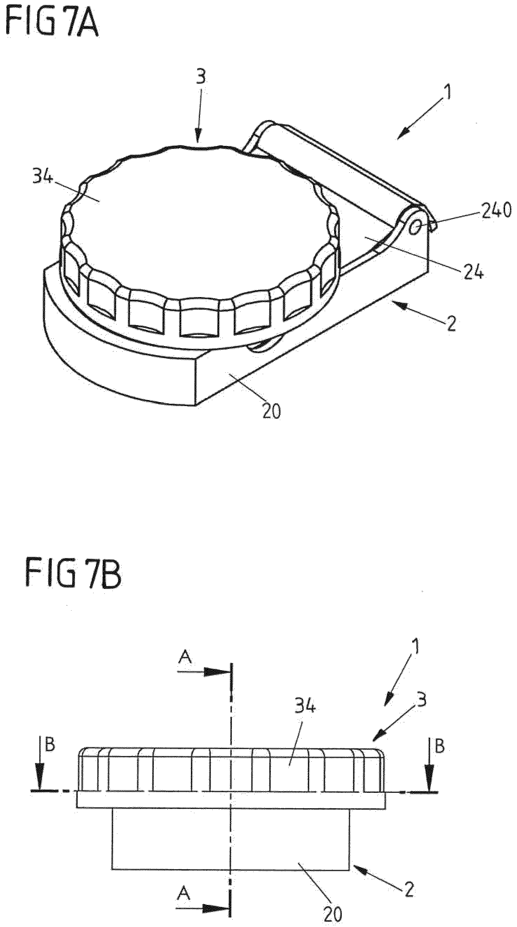

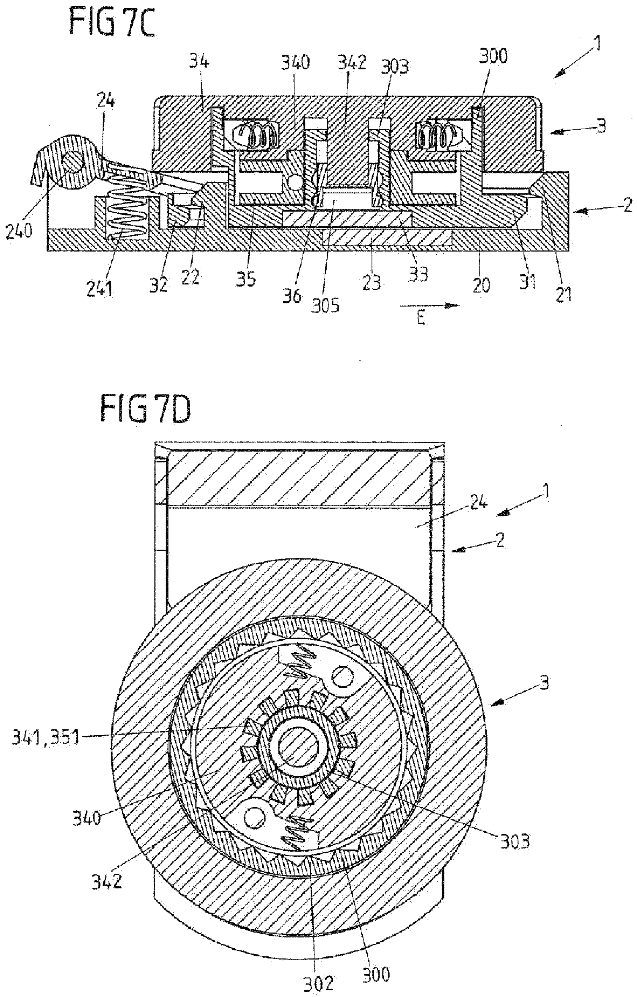

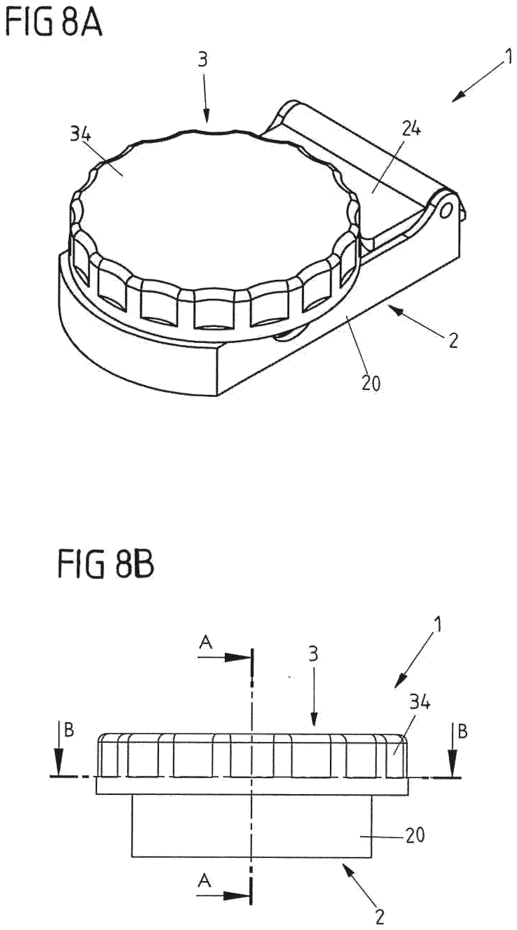

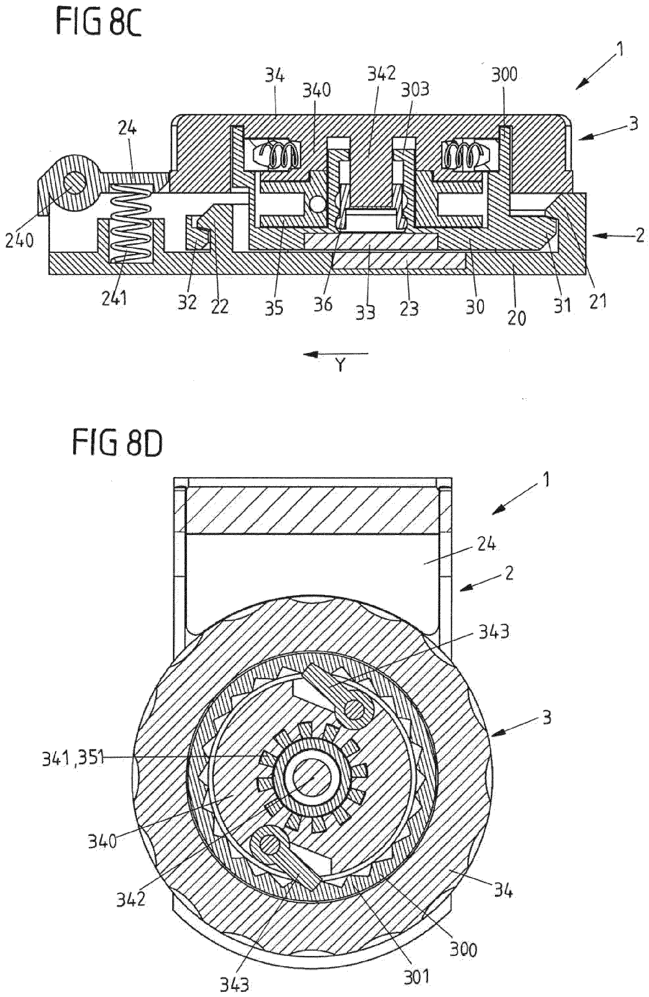

Sind die Hinterschnittelemente 21, 22, 31, 32 aneinander vorbei bewegt worden und sind der Grundkörper 20 des ersten Verschlussteils 2 und das Gehäuseelement 30 des zweiten Verschlussteils 3 in Anlage miteinander gelangt, wie dies in Fig. 7A bis 7D dargestellt ist, so sind die Magnetelemente 23, 33 zueinander versetzt. Es wirkt somit eine Querkraft zwischen den Magnetelementen 23, 33, die die Hinterschnittelemente 21, 22, 31, 32 in eine Eingriffsrichtung E miteinander in Eingriff zieht, wie dies in Fig. 8A bis 8D dargestellt ist. Die Verschlussteile 2, 3 gelangen somit in die in Fig. 8A bis 8D dargestellte Schließstellung und sind in der Schließstellung mechanisch miteinander verbunden und aneinander gehalten.

Wie insbesondere aus Fig. 7A bis 7D ersichtlich, wirkt das Betätigungselement 34 beim Ansetzen der Verschlussteile 2, 3 aneinander auf das Blockierelement 24 ein und lenkt dieses entgegen der Federvorspannung des Federelements 241 aus (siehe insbesondere die Schnittansicht gemäß Fig. 7C). Sind die Hinterschnittelemente 21, 22, 31, 32 miteinander in Eingriff gebracht worden, so wird das Blockierelement 24 aufgrund der Federvorspannung des Federelements 241 zurück in seine Ausgangsstellung bewegt und gelangt in Gegenüberlage zu einem umfänglichen Rand des Betätigungselements 34, sodass darüber eine Bewegung des zweiten Verschlussteils 3 gegenüber dem ersten Verschlussteil 2 in eine Öffnungsrichtung Y (entgegen der Eingriffsrichtung E) gesperrt ist und darüber die Hinterschnittelemente 21, 22, 31, 32 formschlüssig in Eingriff miteinander gehalten werden.

In der Schließstellung kann durch Verdrehen des Betätigungselements 34 in die Wickelrichtung V das Wickelelement 35 verdreht und somit das Zugelement 4 auf das Wickelelement 35 aufgewickelt werden. Das Zugelement 4 kann somit gespannt werden, sodass eine mit dem ersten Verschlussteil 2 verbundene erste Baugruppe und eine über das Zugelement 4 mit dem zweiten Verschlussteil 3 verbundene zweite Baugruppe auf Zug belastet aneinander festgelegt werden können.

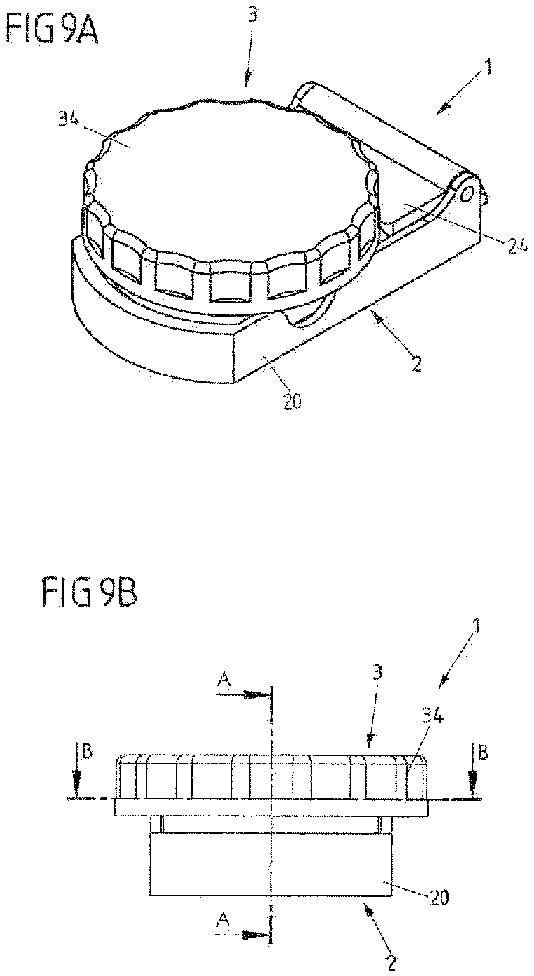

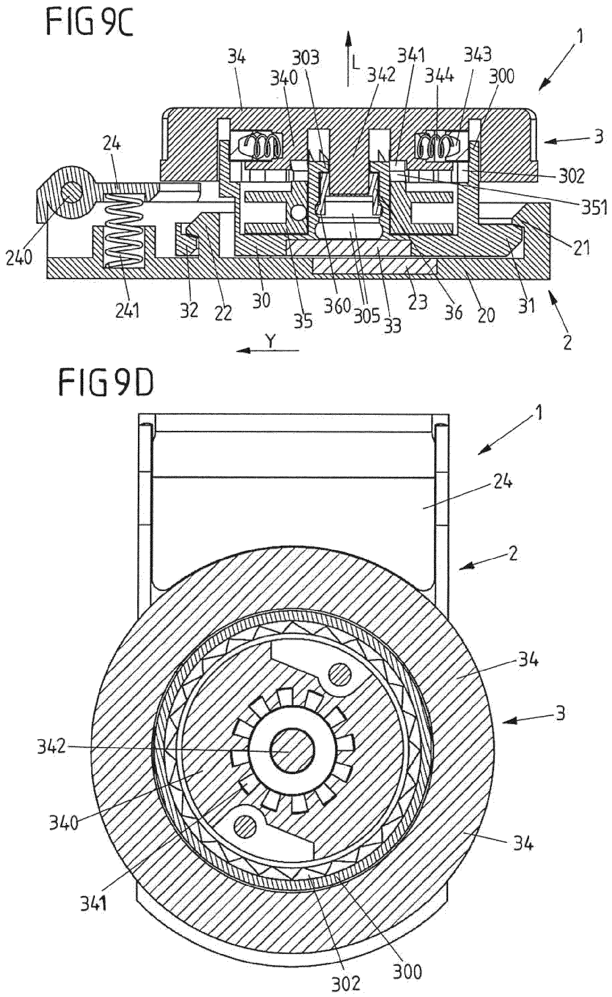

Soll die Verschlussvorrichtung 1 geöffnet werden, so kann das Betätigungselement 34 in eine Löserichtung L axial zu dem Gehäuseelement 30 des zweiten Verschlussteils 3 verstellt werden, wie dies in Fig. 9A-9D dargestellt ist. Dadurch gelangt das Betätigungselement 34 außer Eingriff von dem Wickelelement 35 (die Verzahnungen 341, 351 werden voneinander getrennt), sodass das Wickelelement 35 frei gegenüber dem Gehäuseelement 30 verdreht und insbesondere auch entgegen der Wickelrichtung V bewegt werden kann, um das Zugelement 4 von dem Wickelelement 35 abzuwickeln.

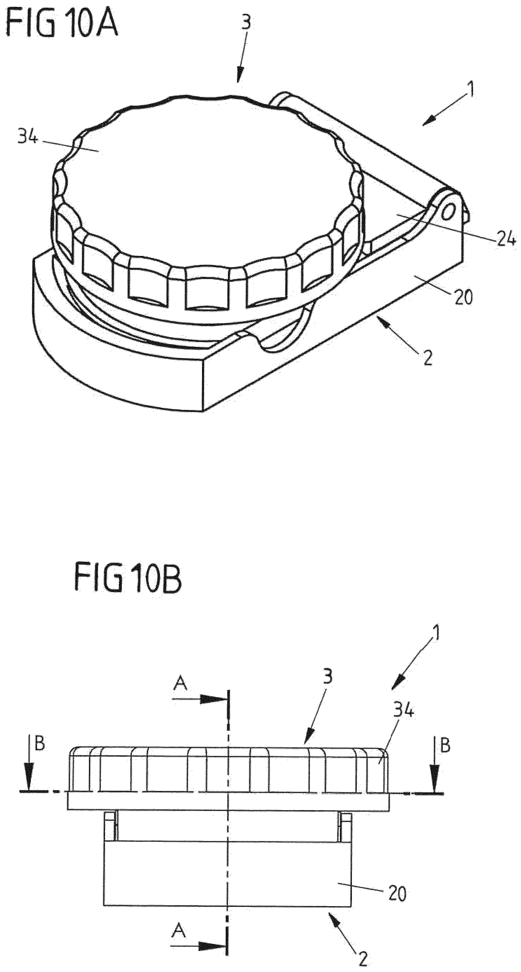

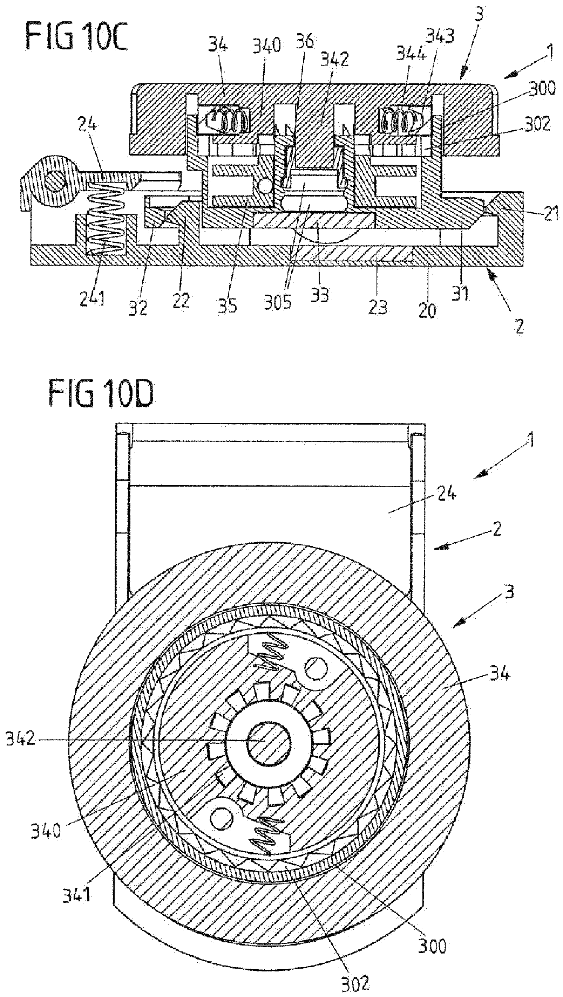

Durch axiale Bewegung des Betätigungselements 34 in die Löserichtung L gelangt das Betätigungselement 34 auch aus seiner Gegenüberlage mit dem Blockierelement 24 heraus. Die Hinterschnittelemente 21, 22, 31, 32 können somit außer Eingriff voneinander gebracht werden, sodass die Verschlussteile 2, 3, wie dies in Fig. 10A-10D dargestellt ist, voneinander getrennt werden können.

Das Lösen der Verschlussteile 2, 3 voneinander kann auch bei gespanntem Zugelement 4, also bei Wirkverbindung zwischen dem Betätigungselement 34 und dem Wickelelement 35 erfolgen. Hierzu kann das Blockierelement 24 manuell betätigt werden, indem es entgegen der Federvorspannung 241 nach unten gedrückt wird, sodass die blockierende Gegenüberlage des Blockierelements 24 mit dem Betätigungselement 34 aufgehoben wird.

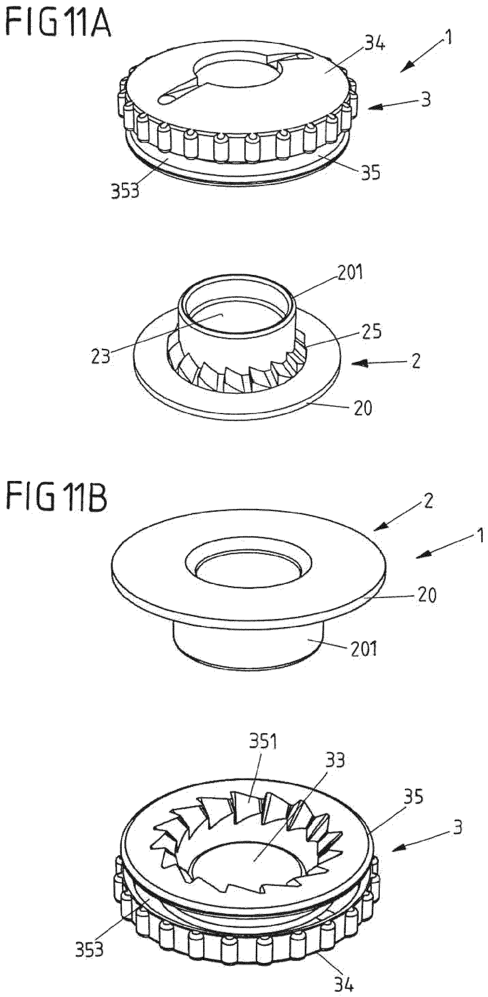

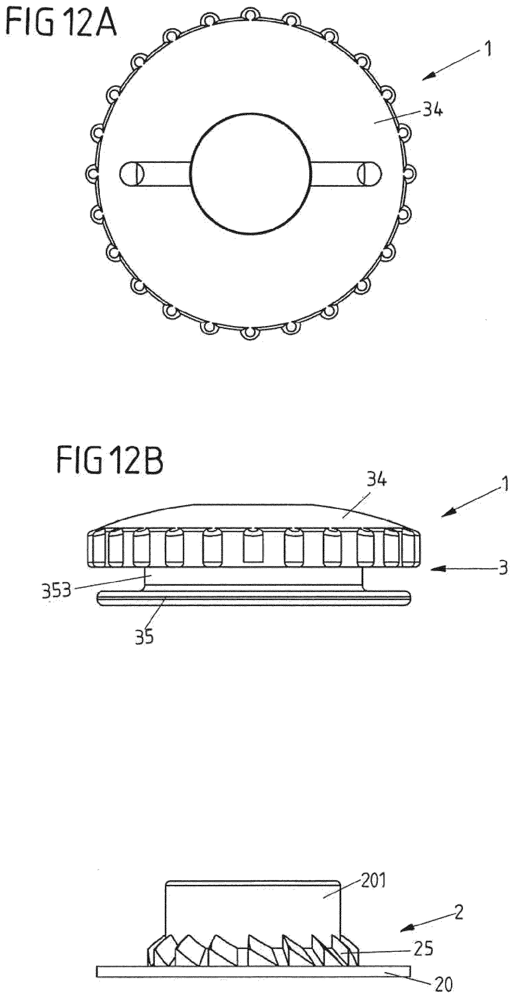

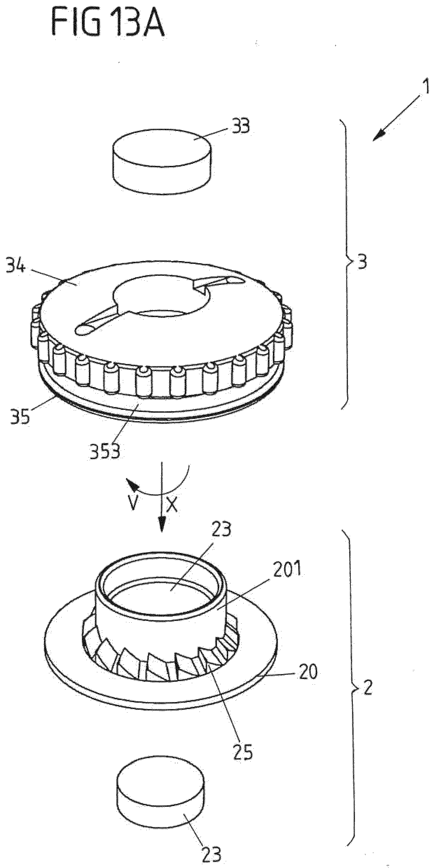

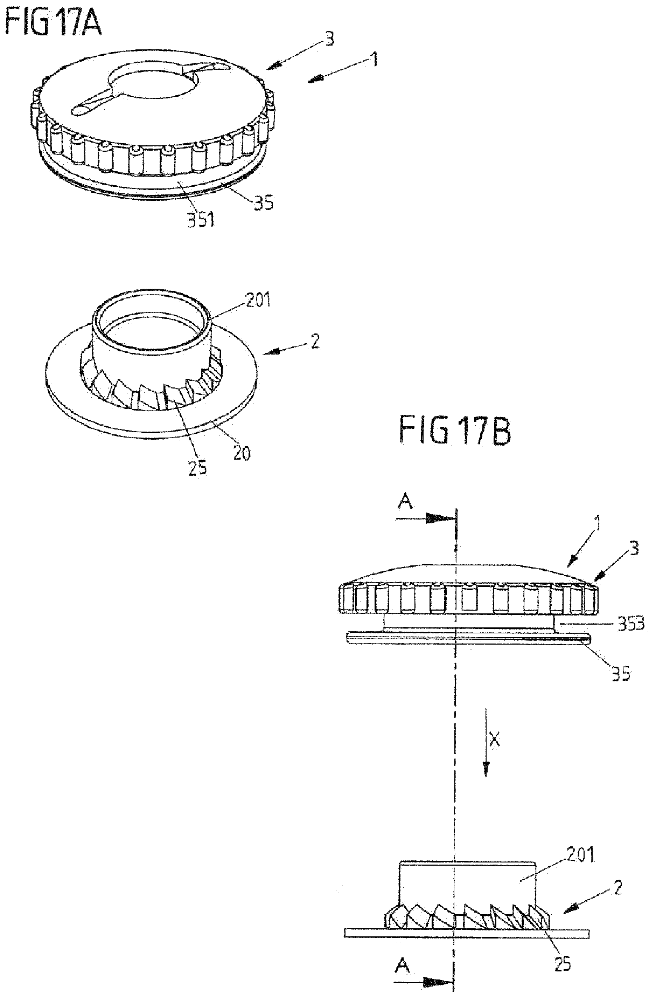

Fig. 11A, 11B bis 17A-17B zeigen ein zweites Ausführungsbeispiel einer Verschlussvorrichtung 1, bei der Verschlussteile 2, 3 entlang einer Schließrichtung X (siehe zum Beispiel Fig. 13A) aneinander angesetzt werden können und in einer Schließstellung aneinander gehalten sind.

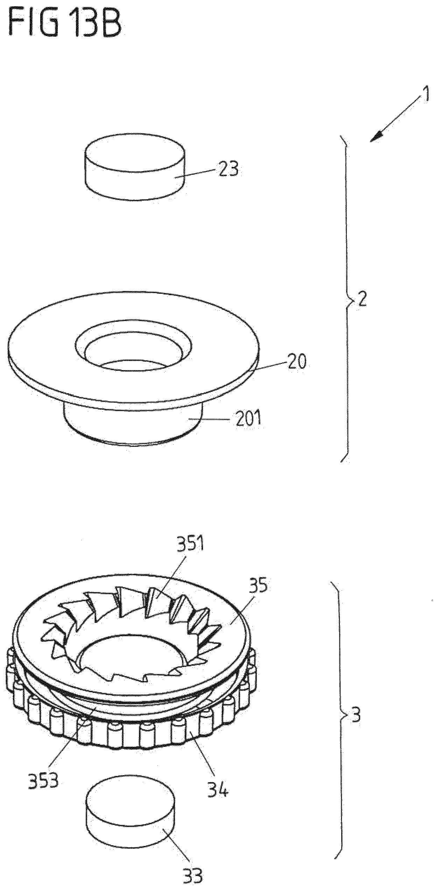

Wie aus den Explosionsansichten gemäß Fig. 13A und 13B ersichtlich, weist das erste Verschlussteil 2 einen Grundkörper 20 auf, an dem ein Zylinderbund 201 geformt ist. Um den Zylinderbund 201 läuft eine Verzahnung 25 um, deren Zähne eine Sägezahnform aufweisen. An dem Grundkörper 20 ist zudem ein Magnetelement 23 angeordnet.

Das zweite Verschlussteil 3 weist ein Betätigungselement 34 auf, mit dem ein Wickelelement 35 fest, beispielsweise einstückig, verbunden ist. Das Wickelelement 35 weist eine Rille 353 auf, in der ein Zugelement 4 zum Aufwickeln auf das Wickelelement 35 aufgenommen werden kann. An dem Betätigungselement 34 ist ein Magnetelement 33 befestigt, das magnetisch anziehend mit dem Magnetelemente 23 am ersten Verschlussteil 2 zusammenwirkt.

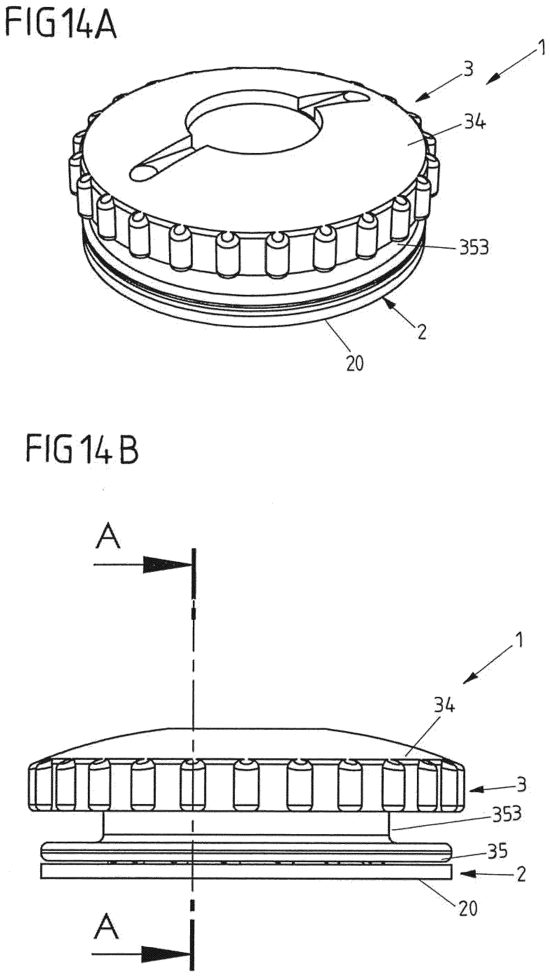

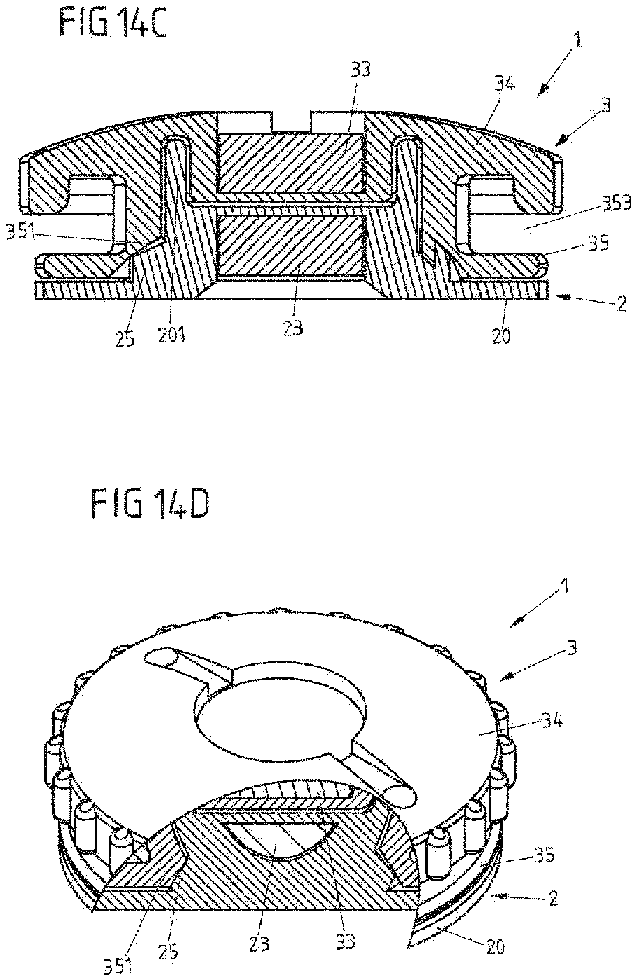

An einer dem ersten Verschlussteil 2 zugewandten Seite ist eine Verzahnung 351 an dem Wickelelement 35 geformt. Bei Ansetzen der Verschlussteile 2, 3 aneinander gelangt diese Verzahnung 351 des Wickelelements 35 mit der Verzahnung 25 am Grundkörper 20 des ersten Verschlussteils 2 in Eingriff, wie dies in Fig. 14A-14D dargestellt ist.

Bei aneinander angesetzten Verschlussteilen 2, 3 greift zudem der Zylinderbund 301 in das Betätigungselement 34 ein und ist darüber verdrehbar und axial bewegbar an dem Betätigungselement 34 gelagert.

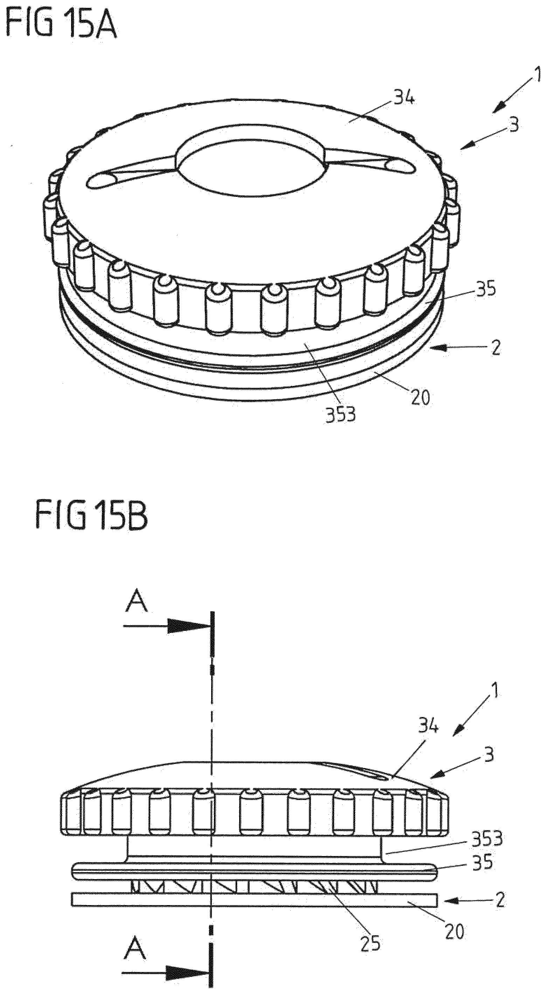

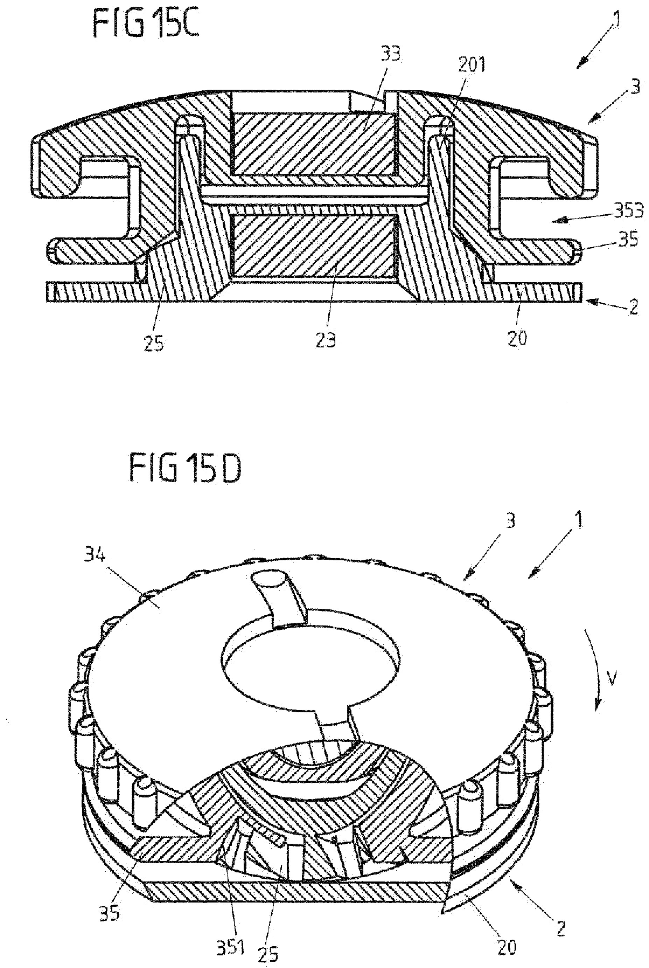

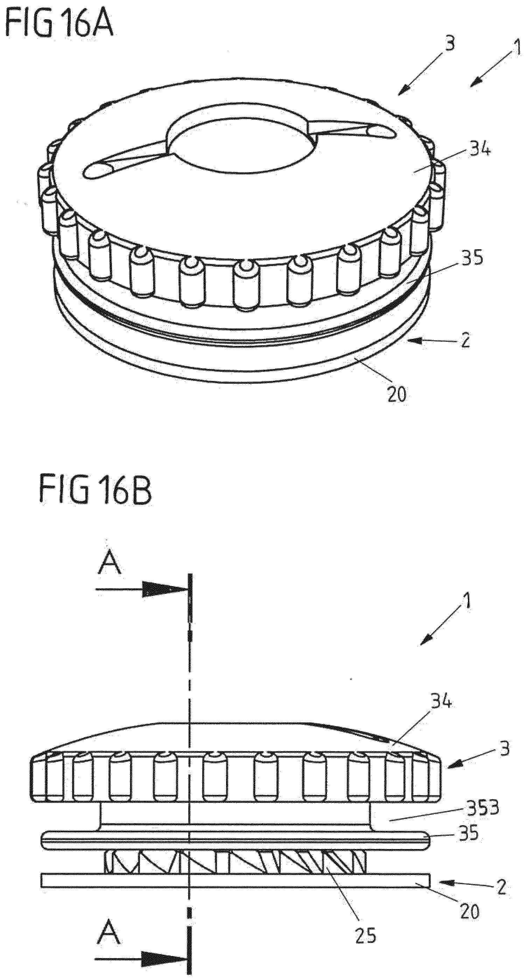

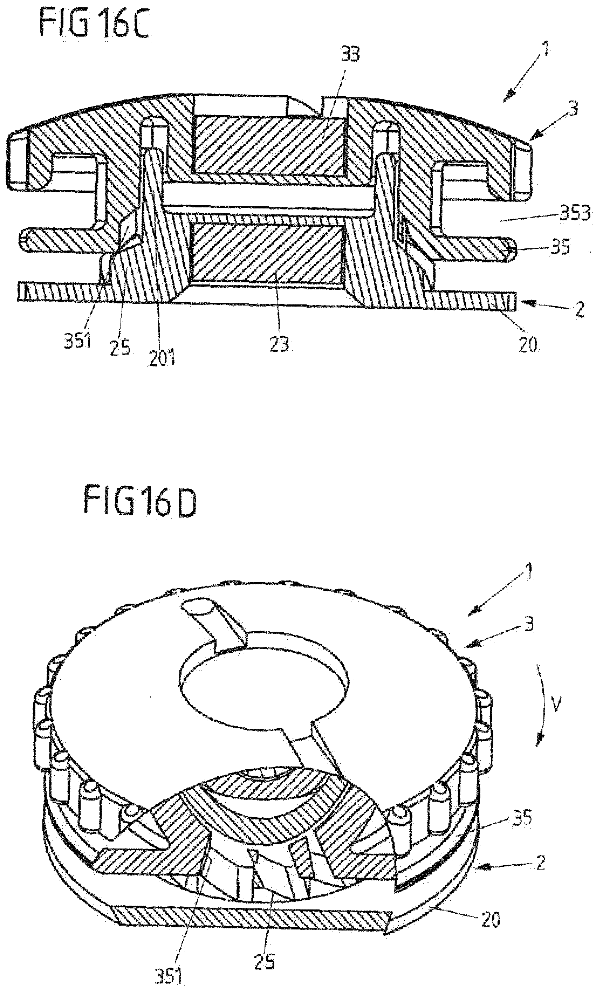

Sowohl die Verzahnung 351 des Wickelelements 35 als auch die Verzahnung 25 des ersten Verschlussteils 2 weisen eine Sägezahnform auf. Dies ermöglicht, wie in Fig. 15A-15D und 16A-16D dargestellt, das zweite Verschlussteil 3 mit dem Wickelelement 35 und dem Betätigungselement 34 in eine Wickelrichtung V gegenüber dem ersten Verschlussteil 2 zu bewegen, wobei dabei die Zähne der Verzahnungen 25, 351 aufeinander aufgleiten und übereinander hinweg bewegt werden, unter axialer Auslenkung der Verschlussteile 2, 3 zueinander. Die Verzahnungen 25, 351 stellen somit eine Art Freilauf zur Verfügung, der bei aneinander angesetzten Verschlussteilen 2, 3 ein Verdrehen des Wickelelements 35 in die Wickelrichtung V ermöglicht, um ein an dem Wickelelement 35 angeordnetes Zugelement 4 zu spannen, eine Bewegung entgegen der Wickelrichtung V hingegen sperrt, sodass das Zugelement 4 bei aneinander angesetzten Verschlussteilen 2, 3 nicht abgewickelt werden kann.

Aufgrund der magnetischen Wechselwirkung der Magnetelemente 23, 33 werden die Verschlussteile 2, 3 aneinander gehalten und gelangen, nach einem Verdrehen, stets in eine Stellung, in der die Verzahnungen 25, 351 formschlüssig miteinander in Eingriff stehen.

Zum Lösen der Verschlussteile 2, 3 voneinander können die Verschlussteile 2, 3 entgegen der Schließrichtung X voneinander abgenommen werden. Dadurch wird auch die Wechselwirkung zwischen den Verzahnungen 25, 351 aufgehoben, sodass das Zugelement 4 von dem Wickelelement 35 abgewickelt werden kann.

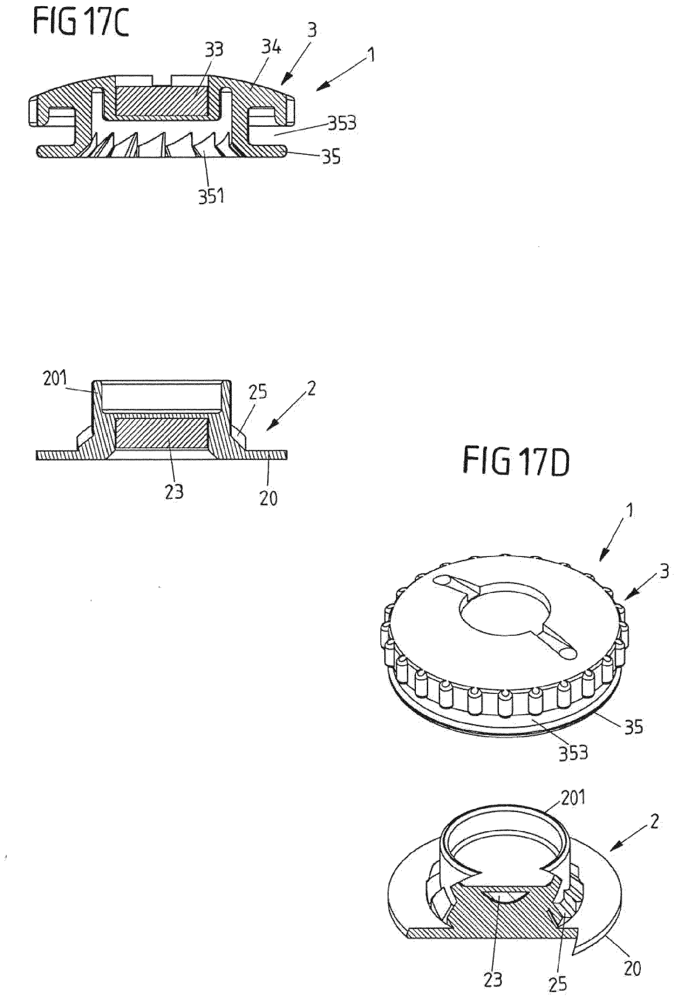

Bei dem Ausführungsbeispiel gemäß Fig. 11A, 11B bis 17A-17D wird das zweite Verschlussteil 3 im Ganzen mit dem fest mit dem Betätigungselement 34 verbundenen Wickelelement 35 verdreht. Es ergibt sich eine sehr einfache Verschlussvorrichtung 1.

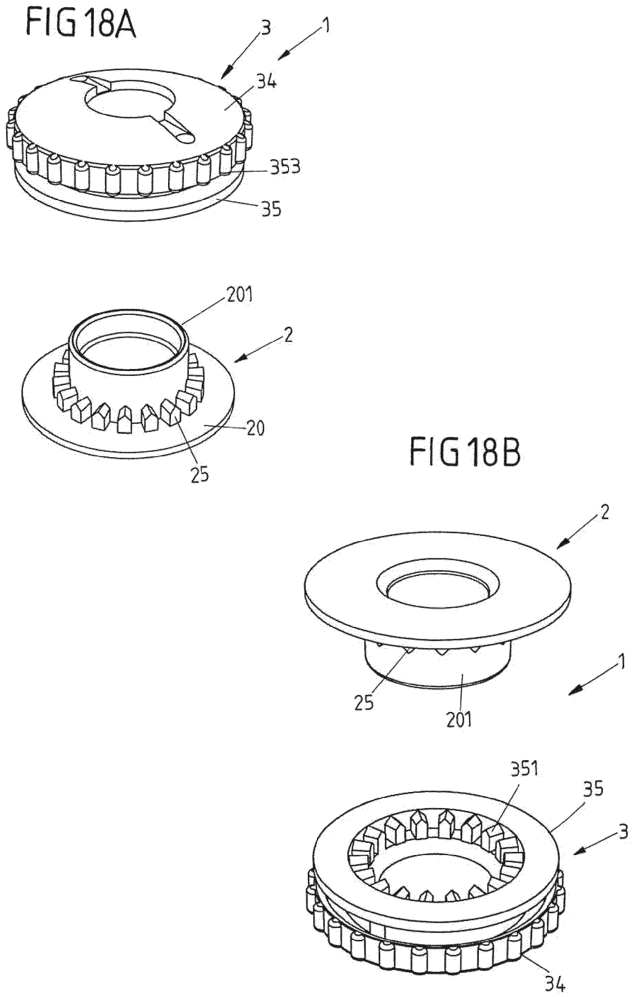

Bei dem Ausführungsbeispiel gemäß Fig. 11A, 11B bis 17A-17D ist das zweite Verschlussteil 3 in der Schließstellung gegenüber dem ersten Verschlussteil 2 in die Wickelrichtung V verdrehbar. Bei einem in Fig. 18A, 18B dargestellten Ausführungsbeispiel sind demgegenüber Verzahnungen 25, 351 an dem ersten Verschlussteil 2 einerseits und an dem zweiten Verschlussteil 3 andererseits derart ausgebildet, dass ein Verdrehen der Verschlussteile 2, 3 gegeneinander (egal in welche Richtung) nicht möglich ist, wenn die Verschlussteile 2, 3 aneinander angesetzt sind, sondern über den Eingriff der Verzahnungen 25, 351 gesperrt ist.

Bei diesem Ausführungsbeispiel ist ein Spannen des Zugelements 4 durch Aufwickeln auf das Wickelelement 35 somit nur vor Ansetzen der Verschlussteile 2, 3 aneinander möglich. Ebenso kann das Zugelement 4 erst nach Abnehmen der Verschlussteile 2, 3 voneinander wieder entspannt werden.

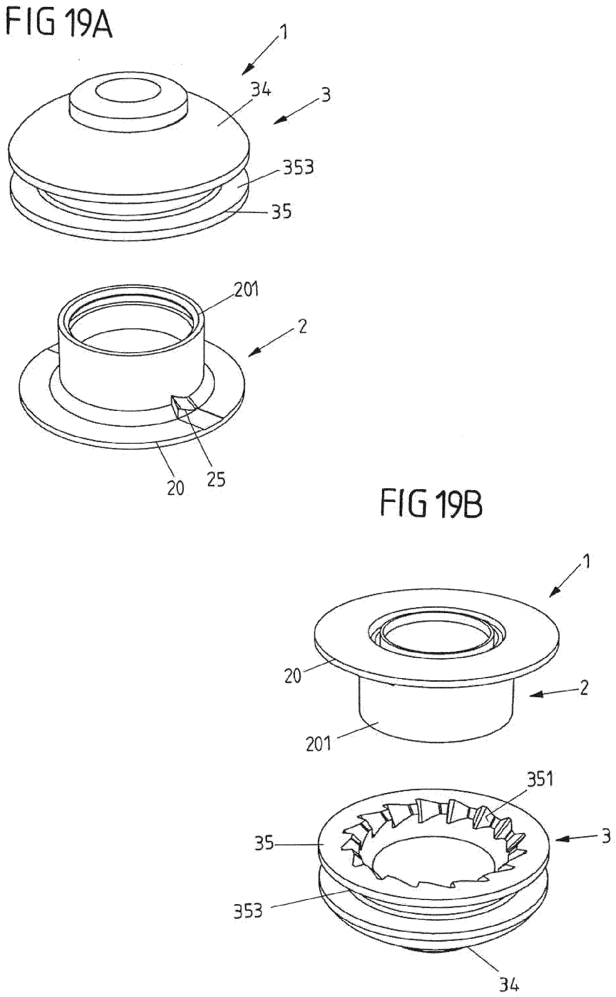

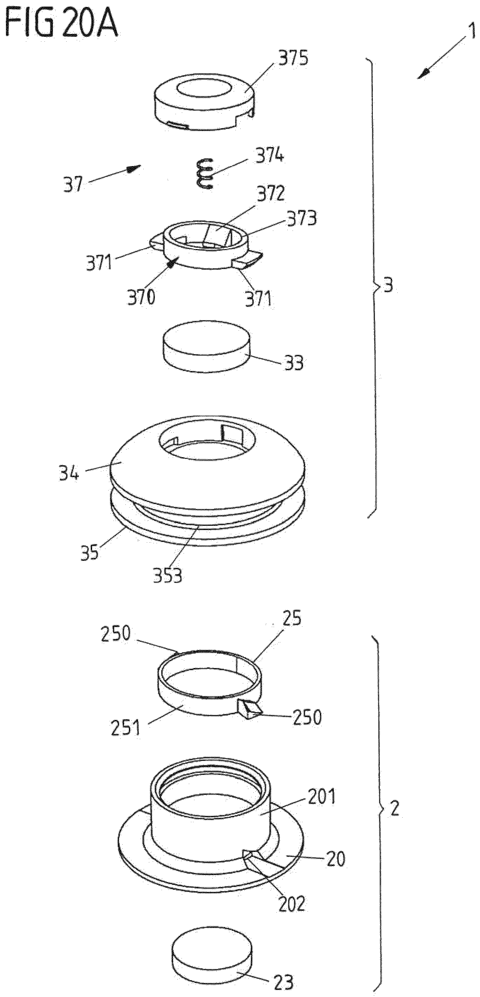

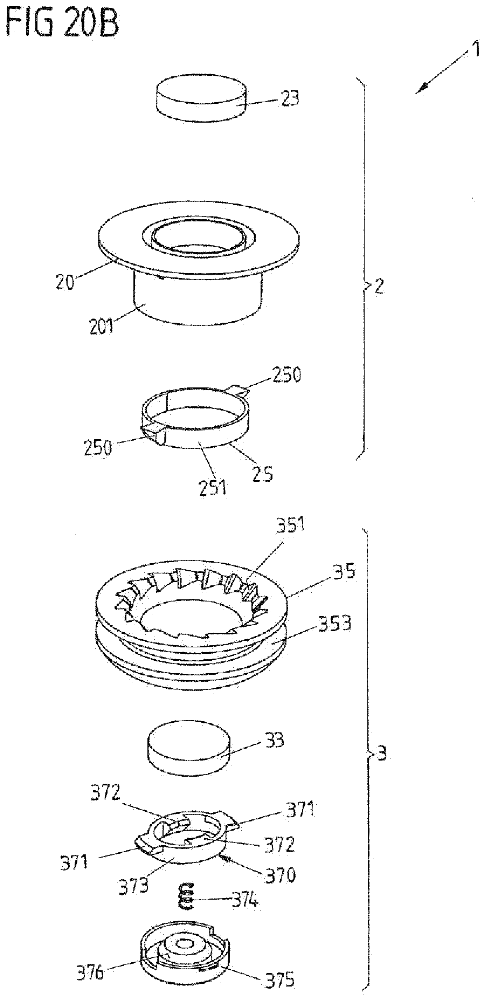

Bei einem in Fig. 19A, 19B bis 26A-26D dargestellten Ausführungsbeispiel weist ein erstes Verschlussteil 2 einen Grundkörper 20 auf, an dem ein Zylinderbund 201 geformt ist, der eine Verzahnungseinrichtung 25 mit über einen Ring 251 miteinander verbundenen Verzahnungselementen 250 einfasst. In montierter Stellung ragen die Verzahnungselemente 250 durch Aussparungen 202 in dem Zylinderbund 201 radial nach außen und können in Wirkverbindung mit einer Verzahnung 351 an einem Wickelelement 35 eines zweiten Verschlussteils 3 gebracht werden.

Das zweite Verschlussteil 3 weist ein Betätigungselement 34 auf, das fest mit dem Wickelelement 35 verbunden ist. An dem Betätigungselement 34 ist eine Rasteinrichtung 37 angeordnet, die ein Rastelement 370 mit über ein ringförmiges Vorspannelement verbundenen Rastvorsprüngen 371 aufweist und über ein Bedienelement 375 zu bedienen ist.

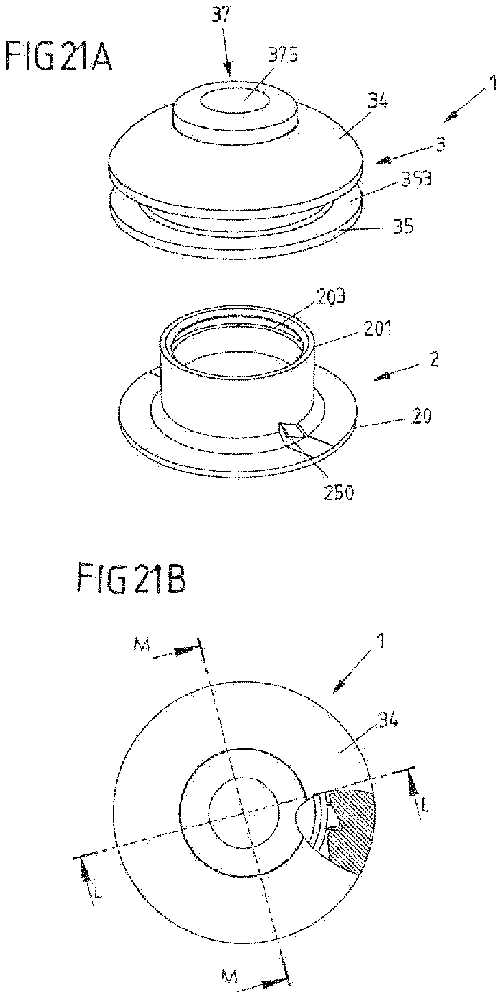

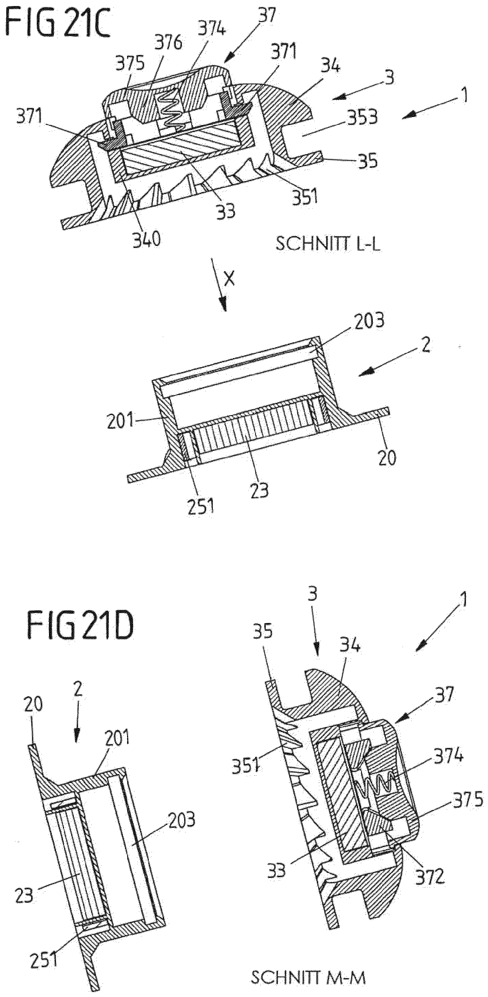

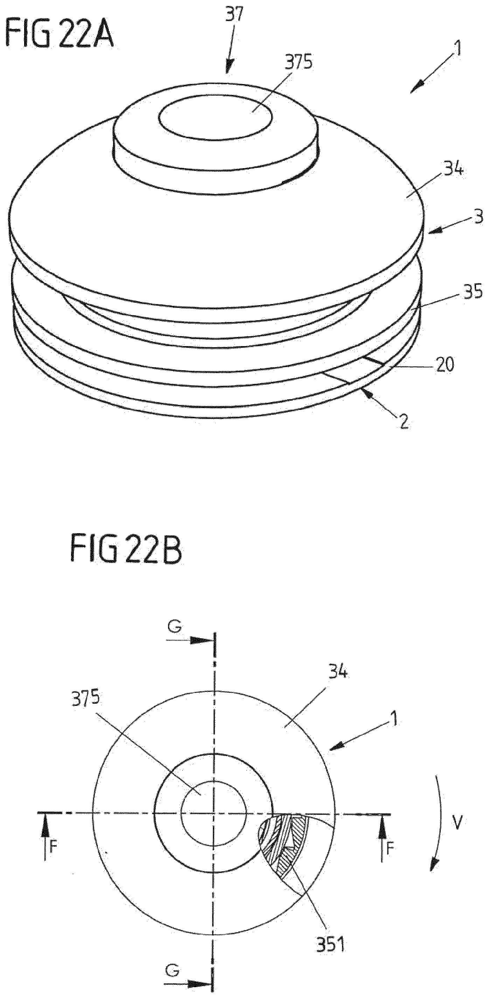

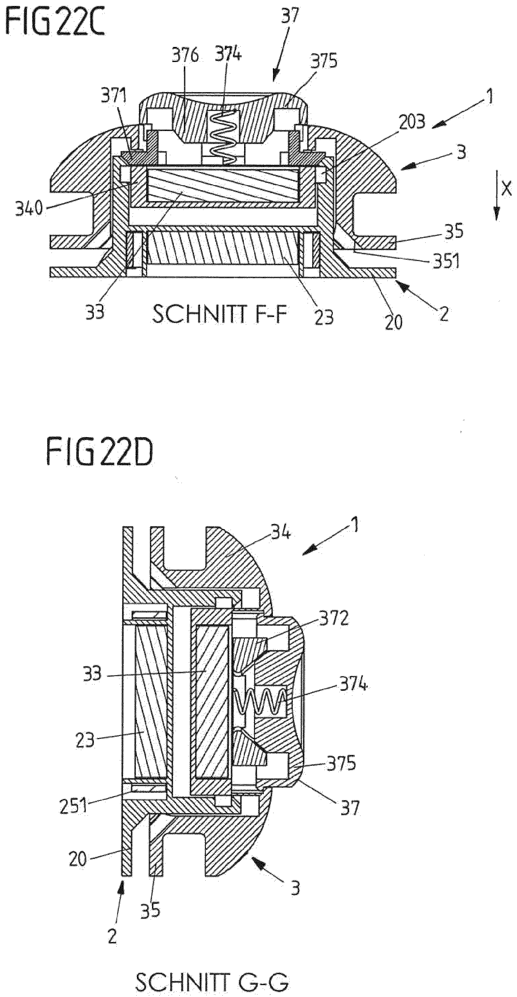

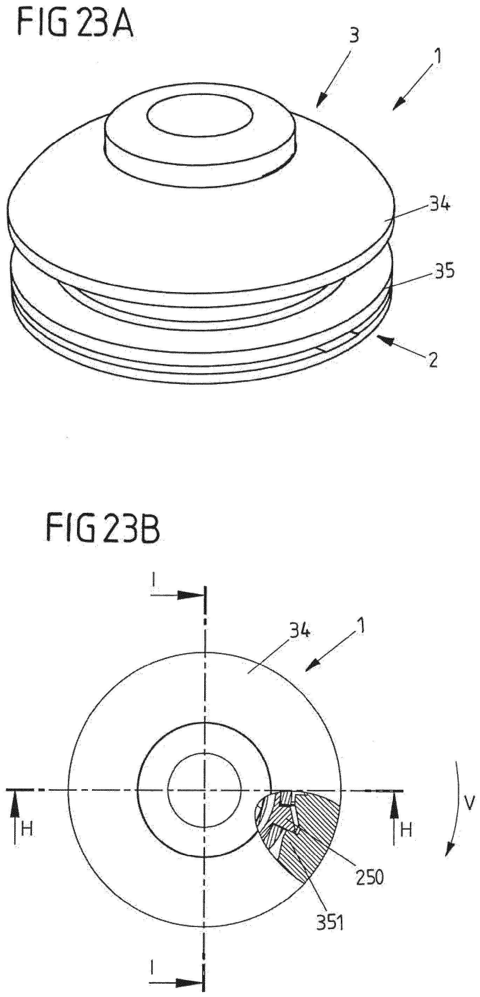

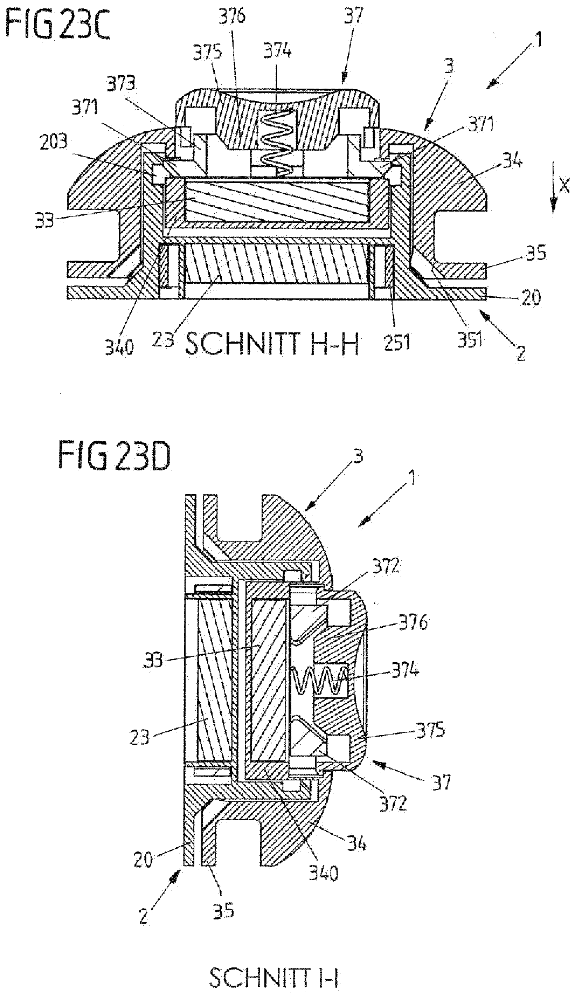

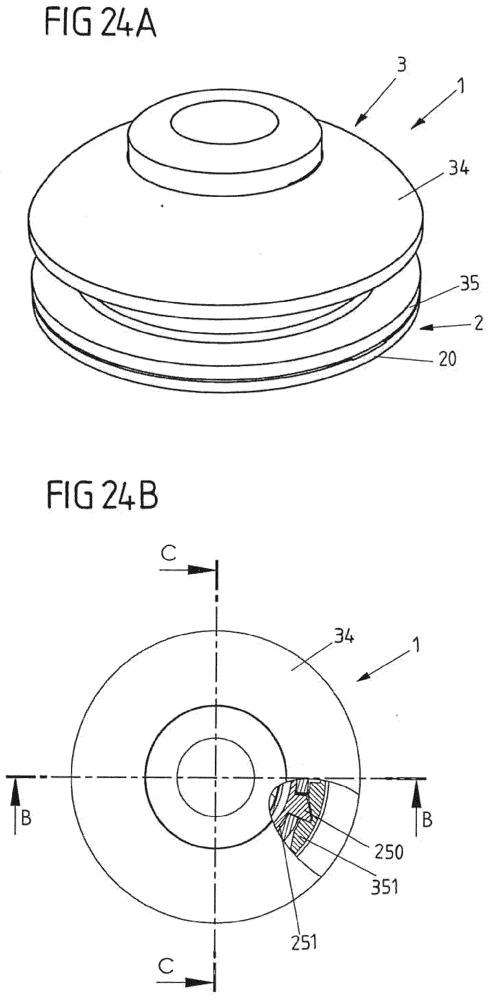

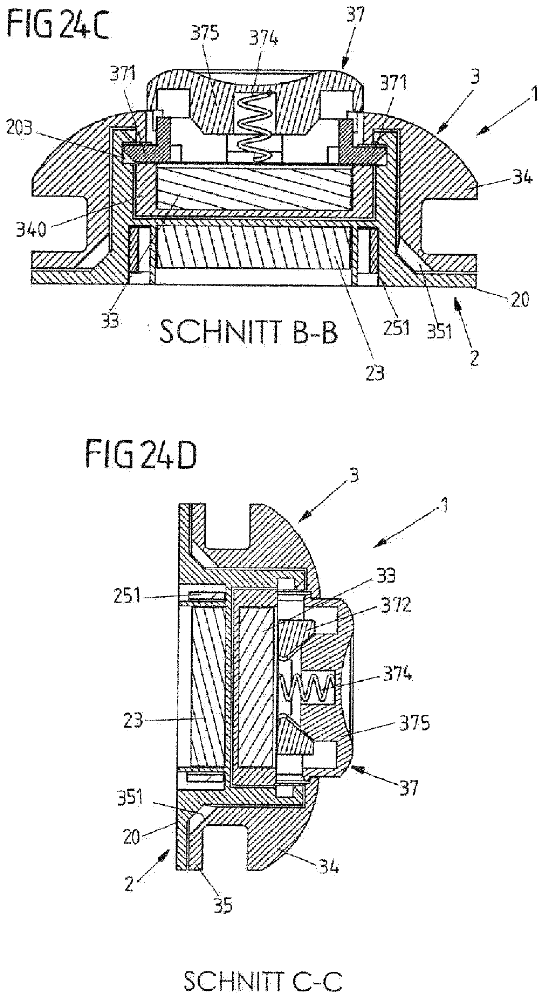

Wie zum Beispiel der Schnittansicht gemäß Fig. 21C zu entnehmen, ist die Rasteinrichtung 37 in einem Körper 340 des Betätigungselements 34 aufgenommen und steht mit ihren Rastvorsprüngen 371 radial von dem Körper 340 vor. Bei Ansetzen der Verschlussteile 2, 3 aneinander, wie dies in Fig. 22A-22D dargestellt ist, greift der Zylinderbund 201 des ersten Verschlussteils 2 axial in das Betätigungselement 34 ein und umgreift den Körper 340 des Betätigungselements 34, sodass, wie aus Fig. 23A-23D und 24A-24D ersichtlich ist, die Rastvorsprünge 371 radial nach innen beiseite gedrängt werden und schließlich, in der Schließstellung der Verschlussvorrichtung 1 gemäß Fig. 24A-24D, mit einem Rasteingriff 203 in Form einer innen am Zylinderbund 201 umlaufenden Ringnut in Eingriff gelangen.

In der Schließstellung sind die Verschlussteile 2, 3 somit miteinander verrastet, sodass die Verschlussteile 2, 3 axial über die Rasteinrichtung 37 in Position zueinander gehalten werden.

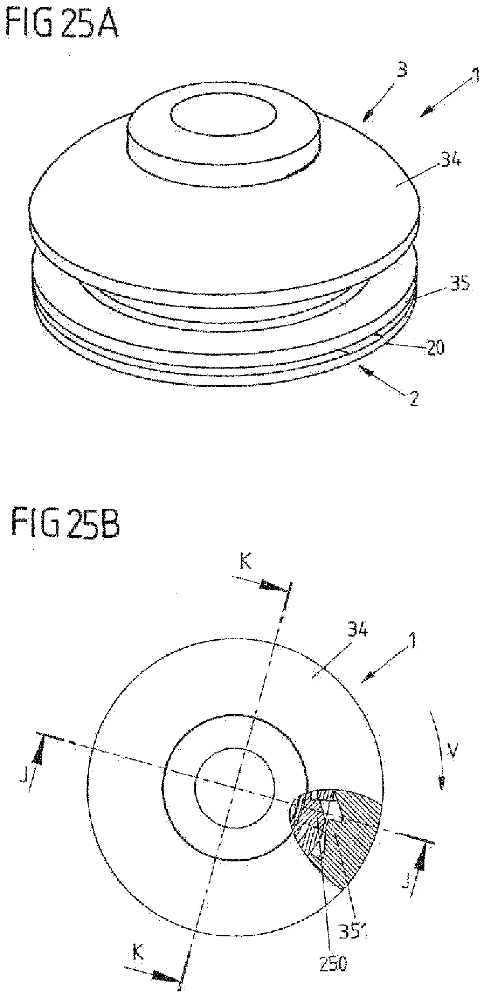

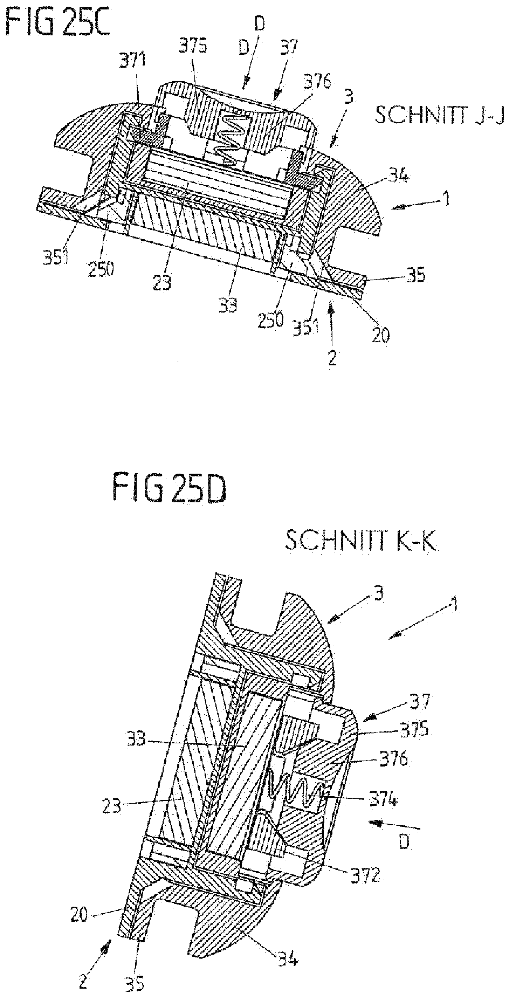

Bei Ansetzen der Verschlussteile 2, 3 gelangen die Verzahnungen 25, 351, wie beispielsweise aus der Teilschnittansicht gemäß Fig. 24B ersichtlich, miteinander in Eingriff. Das Betätigungselement 34 ist zusammen mit dem Wickelelement 35 hierbei in eine Wickelrichtung V zu dem ersten Verschlussteil 2 bewegbar, wobei dabei - wie aus Fig. 25A-25D ersichtlich - die Verzahnungselemente 250 durch die Zähne der Verzahnung 351 des Wickelelements 35 beiseite gedrängt werden und dadurch radial nach innen in den Zylinderbund 201 hineingedrückt werden.

Das Wickelelement 35 kann somit in die Wickelrichtung V zum Aufwickeln des Zugelements 4 verdreht werden. Eine Verdrehbewegung des Wickelelements 35 zu dem ersten Verschlussteil 2 entgegen der Wickelrichtung V ist jedoch aufgrund des Eingriffs der Verzahnungselemente 250 auf Seiten des ersten Verschlussteils 2 mit der Verzahnung 351 auf Seiten des zweiten Verschlussteils 3 gesperrt, sodass ein Abwickeln des Zugelements 4 von dem Wickelelement 35 nicht möglich ist.

Bei einem Verdrehen des Wickelelements 35 zu dem ersten Verschlussteil 2 bleiben das erste Verschlussteil 2 und das zweite Verschlussteil 3 aufgrund der Verrastung über die Rasteinrichtung 37 axial in Position zueinander. Das Verdrehen des Wickelelements 35 ist dabei möglich über die radiale Verdrängung der Verzahnungselemente 250 der Verzahnung 25 des ersten Verschlussteils 2.

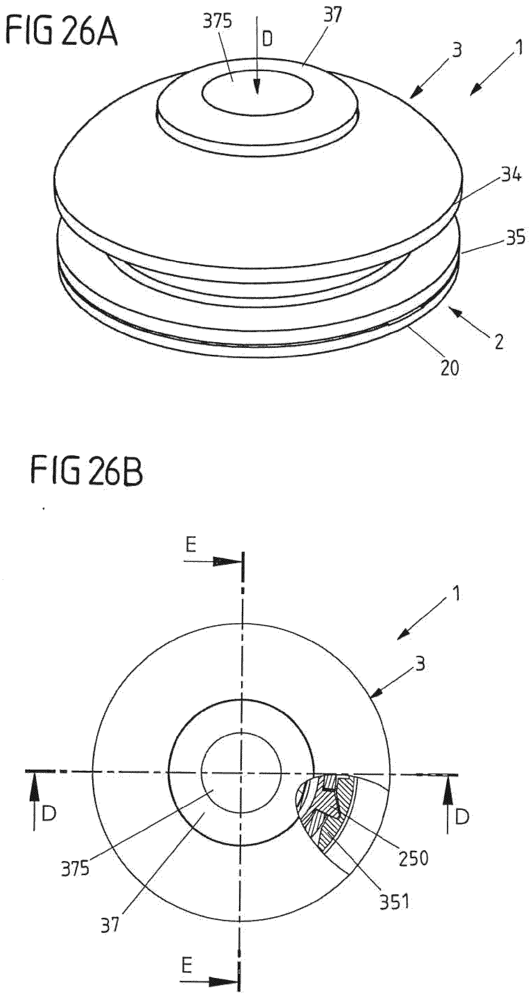

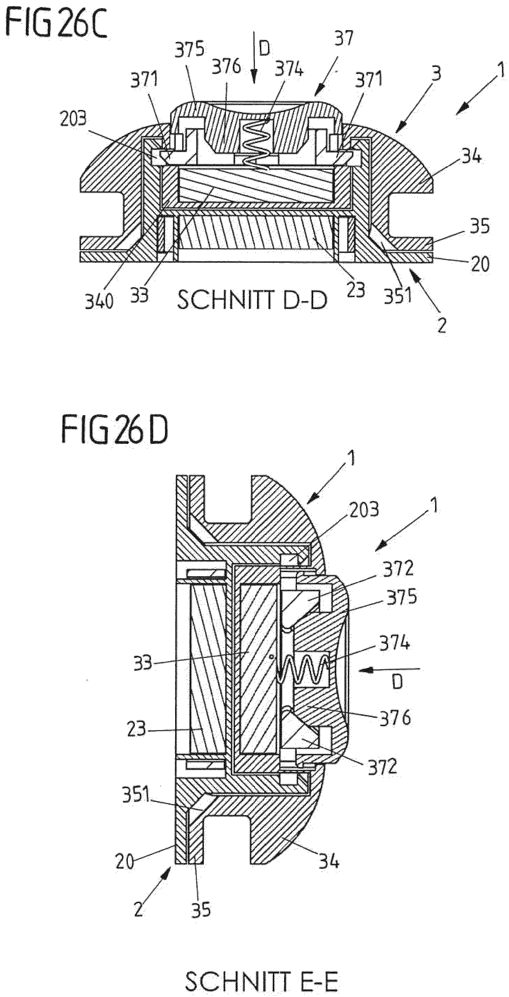

Zum Lösen der Verschlussteile 2, 3 voneinander kann ein Nutzer händisch auf das Bedienelement 375 am Betätigungselement 34 drücken (Betätigungsrichtung D in Fig. 26A). Dadurch läuft das Bedienelement 375 mit einem Auflaufelement 376, an dem Auflaufschrägen gebildet sind, auf angeschrägte Auflaufelemente 372, die an dem ringförmigen Vorspannelement 373 geformt sind, auf und verformt auf diese Weise das Vorspannelement 373. Dadurch werden, wie aus Fig. 26C ersichtlich, die Rastvorsprünge 371 radial nach innen gezogen, sodass der Eingriff zwischen den Rastelementen 371 und dem Rasteingriff 203 am Zylinderbund 201 aufgehoben wird und die Verschlussteile 2, 3 axial entgegen der Schließrichtung X voneinander abgenommen werden können.

Auch bei dem Ausführungsbeispiel gemäß Fig. 19A, 19B bis 26A-26D weist jedes Verschlussteil 2, 3 ein Magnetelement 23, 33 auf, die beim Ansetzen der Verschlussteile 2, 3 magnetisch anziehend zusammenwirken und somit ein Ansetzen der Verschlussteile 2, 3 aneinander magnetisch unterstützen.

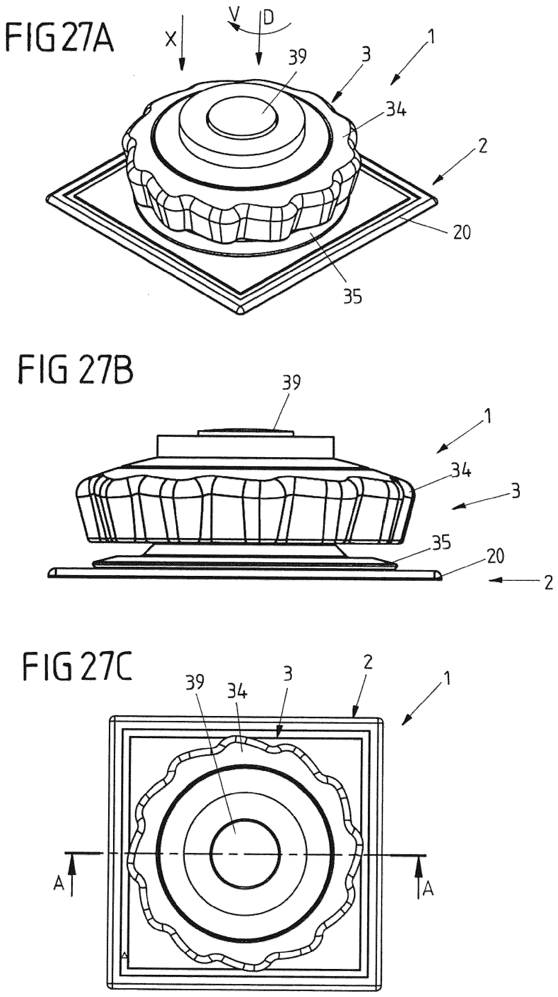

Fig. 27A bis 27D zeigen ein anderes Ausführungsbeispiel, das mit Blick auf die zwischen dem ersten Verschlussteil 2 und dem Wickelelement 35 wirkenden Verzahnungen 25, 351 ähnlich wie das vorangehend anhand von Fig. 11A, 11B bis 17A-17B beschriebene Ausführungsbeispiel ausgestaltet ist, sodass diesbezüglich auf die vorangehenden Erläuterungen verwiesen werden soll.

Das Ausführungsbeispiel gemäß Fig. 27A bis 27D weist eine Rasteinrichtung 37 auf, die an dem zweiten Verschlussteil 3 angeordnet ist und dazu dient, die Verschlussteile 2, 3 in der Schließstellung derart miteinander zu verrasten, dass das zweite Verschlussteil 3 entgegen der Schließrichtung X rastend und somit formschlüssig an dem ersten Verschlussteil 2 gehalten ist.

Das Verwenden einer solchen Rasteinrichtung 37 ermöglicht eine nicht magnetische Ausgestaltung der Verschlussvorrichtung 1. Grundsätzlich kann somit bei der Verschlussvorrichtung 1 auf Magnetelemente verzichtet werden. Denkbar und möglich ist aber auch, zusätzlich zu der Rasteinrichtung 37 auch Magnetelemente zu verwenden.

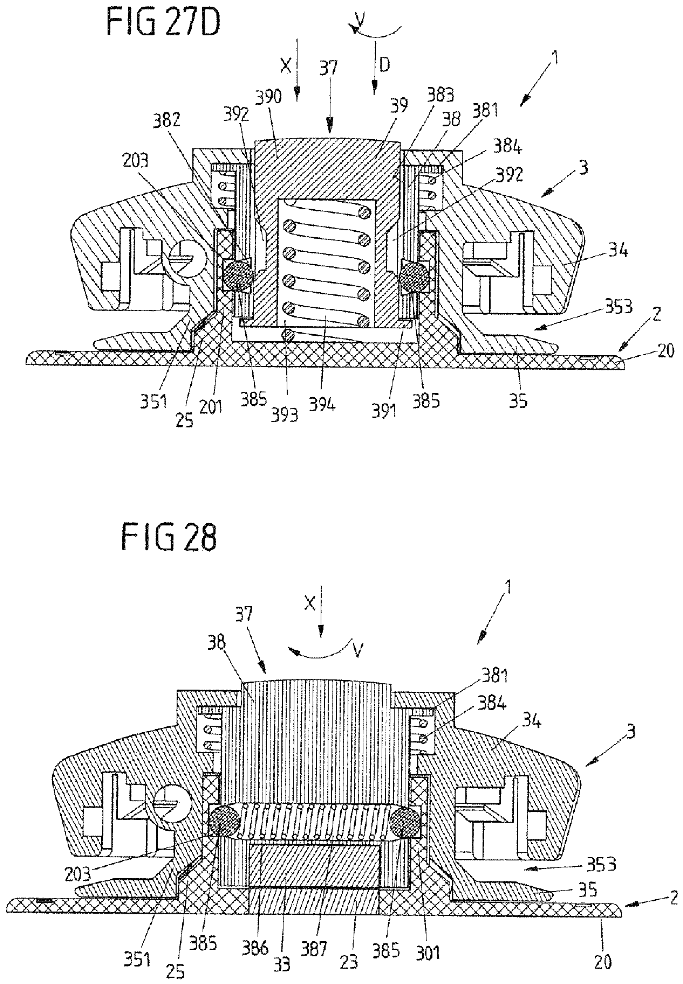

Die Rasteinrichtung 37 weist ein Eingriffselement 38 auf, das mit einem Ringbund 381 axial beweglich in einem Aufnahmeraum innerhalb des Betätigungselements 34 aufgenommen und über ein Federelement 384 in Form einer Spiralfeder gegenüber dem Betätigungselement 34 federvorgespannt ist. An einem sich von dem Ringbund 381 erstreckenden, zylindrischen Körper 380 sind diametral gegenüberliegend Aufnahmeöffnungen 382 geformt, in denen Rastelemente 385 in Form von Kugeln aufgenommen sind, die dazu dienen, in der Schließstellung der Verschlussvorrichtung 1 (Fig. 27D) rastend in einen Rasteingriff 203 in Form einer innenseitig des Zylinderbunds 201 des ersten Verschlussteils 2 umlaufenden Ringnut einzugreifen, sodass darüber, wie dies aus Fig. 27D ersichtlich ist, eine Verrastung zwischen dem in der Schließstellung in eine des Zylinderbunds 201 eingreifenden Eingriffselement 38 und dem Zylinderbund 201 besteht und darüber die Verschlussteile 2, 3 miteinander verrastet sind.

Innerhalb einer zentralen Öffnung 383 des Eingriffselements 38 ist ein Bedienelement 39 axial entlang der Schließrichtung X geführt. Das Bedienelement 39 weist eine zylindrische Gestalt auf, ist über ein in einer Öffnung 393 in Form eines Sacklochs einliegendes Federelement 394 in der Schließstellung an dem ersten Verschlussteil 2 elastisch abgestützt und zudem über einen endseitigen Ringbund 391 mit dem Eingriffselement 38 wegbegrenzend in Anlage, wenn es nicht gegenüber dem Eingriffselement 38 betätigt ist.

Wie aus Fig. 27D ersichtlich, weist das Bedienelement 39 an seiner äußeren Umfangsfläche zwei diametral gegenüberliegende Entsperröffnungen 392 auf, die in der Schließstellung auf einer axial unterschiedlichen Höhe zu den Rastelementen 385 angeordnet sind, sodass die Rastelemente 385 mit der äußeren Umfangsfläche des zylindrischen Körpers 390 des Bedienelements 39 in Anlage sind und darüber in rastendem Eingriff mit dem Rasteingriff 203 innenseitig des Zylinderbunds 201 gehalten werden.

In der Schließstellung (Fig. 27D) wird das Betätigungselement 34 aufgrund der Federvorspannung des Federelements 384 in Richtung des ersten Verschlussteils 2 gedrückt, und durch Anlage der Basisfläche 340 an dem Ringbund 354 des Wickelelements 35 wird das Wickelelement 35 in Eingriff mit der Verzahnung 25 des ersten Verschlussteils 2 gedrückt. In der Schließstellung ist das zweite Verschlussteil 3 somit rastend an dem ersten Verschlussteil 2 gehalten, bei Verzahnungseingriff zwischen dem Wickelelement 35 und dem ersten Verschlussteil 2.

Wird in der Schließstellung der Verschlussvorrichtung 1 das Betätigungselement 34 und somit das Wickelelement 35 zusammen mit dem Betätigungselement 34 in die Wickelrichtung V verdreht, so gleiten die Verzahnungen 25, 351 übereinander, was eine (geringfügige) axiale Bewegung des Wickelelements 35 und darüber des Betätigungselements 34 bewirkt. Weil dabei das Eingriffselement 38 der Rasteinrichtung 37 über die Rastelemente 385 axial zu dem Zylinderbund 201 des ersten Verschlussteils 2 festgehalten wird, erfolgt die axiale Auslenkung des Wickelelements 35 und des Betätigungselements 34 entgegen der Federvorspannung des Federelements 384.

Nach erfolgter Verdrehung des Betätigungselements 34 und des Wickelelements 35 gelangen die Verzahnungen 25, 351 dann, aufgrund der Federvorspannung des Federelements 384, wiederum in Eingriff miteinander.

Zum Lösen der Verschlussteile 2, 3 voneinander kann ein Nutzer das Bedienelement 39 in eine Betätigungsrichtung D in das Eingriffselement 38 hineindrücken. Dadurch wird der Körper 390 des Bedienelements 39 axial entlang der Schließrichtung X innerhalb des Eingriffselements 38 verstellt, sodass die Entsperröffnungen 392 auf die axial gleiche Höhe wie die Rastelemente 385 gelangen und die Rastelemente 385 somit radial nach innen ausweichen können. Auf diese Weise wird die sperrende Verrastung zwischen dem Eingriffselement 38 und dem Zylinderbund 201 aufgehoben, sodass die Verschlussteile 2, 3 entgegen der Schließrichtung X voneinander abgenommen werden können.

Zum erneuten Schließen der Verschlussvorrichtung 1 kann das zweite Verschlussteil 3 in die Schließrichtung X wiederum an das erste Verschlussteil 2 angesetzt werden, wodurch das Eingriffselement 38 in Eingriff mit der Öffnung des Zylinderbunds 201 gelangt und die Rastelemente 385 rastend in den Rasteingriff 203 in Form der umlaufenden Nut innerhalb des Zylinderbunds 201 eingreifen. Dadurch gelangen auch die Verzahnungen 25, 351 in Verzahnungseingriff miteinander, und die Verschlussvorrichtung 1 nimmt die in Fig. 27D dargestellte Schließstellung ein.

Fig. 28 zeigt ein gegenüber dem Ausführungsbeispiel gemäß Fig. 27A-27D modifiziertes Ausführungsbeispiel, das in seiner äußeren Ansicht dem Ausführungsbeispiel gemäß Fig. 27A-27D gleicht. Bei dem Ausführungsbeispiel gemäß Fig. 28 weist die Rasteinrichtung 37 ein Eingriffselement 38 auf, an dem eine durchgehende, quer zur Schließrichtung X erstreckte Bohrung 387 gebildet ist, in der zwei kugelförmige Rastelemente 385 angeordnet und über ein Federelement 386 gegeneinander vorgespannt sind. Bei Ansetzen der Verschlussteile 2, 3 aneinander gelangt das Eingriffselement 38 in Eingriff mit dem Zylinderbund 201, und die Rastelemente 385 verrasten mit dem Rasteingriff 203 innenseitig der Öffnung des Zylinderbunds 201, wie dies aus Fig. 28 ersichtlich ist.

Die Verrastung wird hierbei aufgrund der Federvorspannung über das Federelement 386 in der Schließstellung gehalten. Soll die Verrastung gelöst werden, so kann das zweite Verschlussteil 3 entgegen der Schließrichtung X bei hinreichender Kraftwirkung von dem ersten Verschlussteil 2 abgezogen werden, wodurch die Rastelemente 385 selbsttätig, durch Auflaufen auf die obere Kante des nutförmigen Rasteingriffs 203, radial nach innen versetzt werden und somit außer Eingriff von dem Rasteingriff 203 gelangen. Die Verrastung zwischen den Verschlussteilen 2, 3 kann somit ohne gesonderte Betätigung des Eingriffselements 38 gelöst werden.

Das Ausführungsbeispiel gemäß Fig. 28 ist magnetisch ausgebildet dadurch, dass ein Magnetelement 23 an dem Körper 20 des ersten Verschlussteils 2 und zudem ein Magnetelement 33 an dem Körper 380 des Eingriffselements 38 angeordnet ist, wie dies aus der Schnittansicht gemäß Fig. 28 ersichtlich ist. Die Verschlussteile 2, 3 wirken somit (auch) magnetisch zusammen, was das Ansetzen der Verschlussteile 2, 3 erleichtert.

Das Ausführungsbeispiel gemäß Fig. 28 kann aber auch rein mechanisch ohne Magnetelemente ausgebildet sein.

Bei den vorangehend beschriebenen Ausführungsbeispielen können die Verzahnungseinrichtungen 25, 351 auf Seiten des ersten Verschlussteils 2 und des zweiten Verschlussteils 3 grundsätzlich ganz unterschiedlich ausgeführt sein, um in der Schließstellung einen (zumindest bis zu einem gewissen Grenzdrehmoment belastbaren) formschlüssigen Halt zwischen den Verschlussteilen 2, 3 herzustellen.

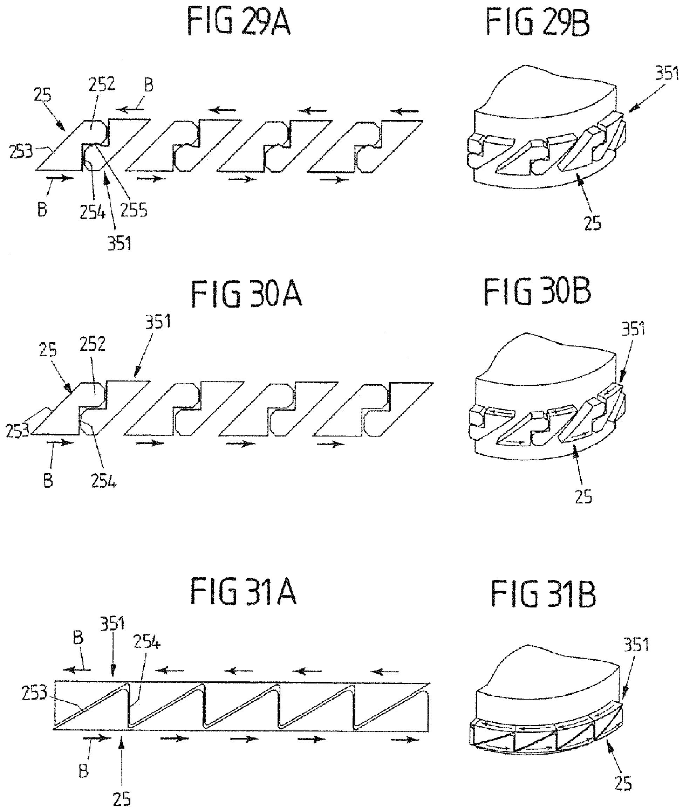

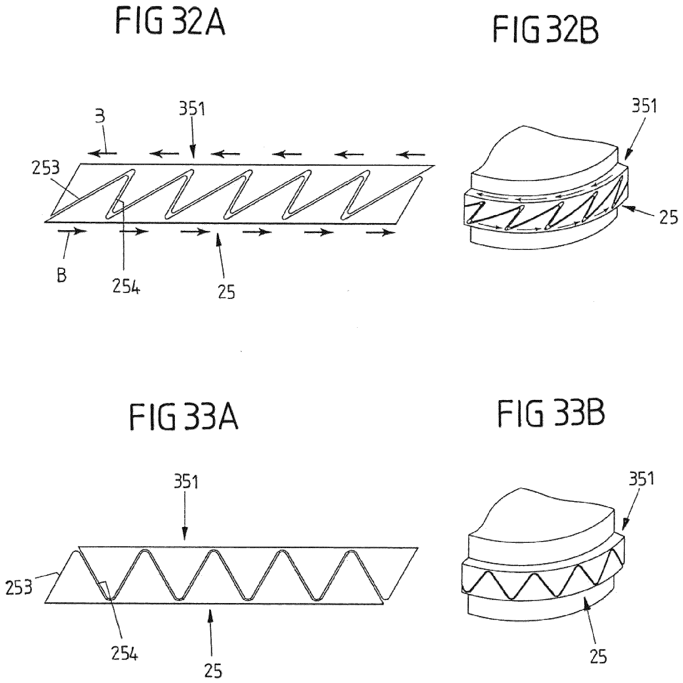

Fig. 29A, 29B bis 33A, 33B zeigen unterschiedliche Ausführungsformen von Verzahnungseinrichtungen 25, 351, die sich in der Geometrie ihrer Zähne unterscheiden und bei einer Verschlussvorrichtung 1 der hier beschriebenen Art zum Einsatz kommen können.

So weisen bei dem Ausführungsbeispiel gemäß Fig. 29A, 29B die Zähne einer jeden Verzahnungseinrichtung 25, 351 eine schräg zur Schließrichtung X erstreckte Zahnflanke in Form einer Auflaufschräge 253 sowie eine näherungsweise senkrecht erstreckte Zahnflanke 254 mit einem daran ausgebildeten, entlang der Wickelrichtung V vorspringenden Vorsprungselement 252 auf. Bei einer der Wickelrichtung V entgegengesetzten Belastung in eine Belastungsrichtung B gelangen die Vorsprungselemente 252 der Zähne der Verzahnungseinrichtungen 25, 351 miteinander in Eingriff, sodass eine Bewegung der Verzahnungseinrichtungen 25, 351 in die Belastungsrichtung B (entgegen der Wickelrichtung V) gesperrt ist. Das Wickelelement 35 kann somit nicht in die Belastungsrichtung B entgegen der Wickelrichtung V zu dem ersten Verschlussteil 2 verdreht werden, wenn sich die Verschlussrichtung 1 in ihrer Schließstellung befindet.

Bei dem Ausführungsbeispiel gemäß Fig. 29A, 29B ist zusätzlich an dem Vorsprungselement 252 eines jeden Zahns (oder zumindest eines Teils der Zähne) der Verzahnungseinrichtung 351 eine Rastnase 255 ausgebildet, die zum Beispiel rastend mit einer zugehörigen Rastausnehmung an dem Vorsprungselement 252 eines zugeordneten Zahns der Verzahnungseinrichtung 25 in Eingriff gelangen kann, um den sperrenden Eingriff der Zähne der Verzahnungseinrichtungen 25, 351 bei Belastung in die Belastungsrichtung B zusätzlich zu sperren.

Das Ausführungsbeispiel gemäß Fig. 30A, 30B gleicht dem Ausführungsbeispiel gemäß Fig. 29A, 29B, abgesehen davon, dass bei dem Ausführungsbeispiel gemäß Fig. 30A, 30B an den Vorsprungselementen 252 der Zähne der Verzahnungseinrichtung 351 keine Rastnasen 255 wie bei dem Ausführungsbeispiel gemäß Fig. 29A, 29B vorgesehen sind.

Bei dem Ausführungsbeispiel gemäß Fig. 31A, 31B sind die Verzahnungseinrichtungen 25, 351 als Sägezahn-Verzahnungen ausgebildet. Die Zähne der Verzahnungseinrichtungen 25, 351 weisen hierbei jeweils eine Zahnflanke in Form einer Auflaufschräge 253 sowie eine senkrecht erstreckte Zahnflanke 254 auf. Eine Belastung der Verzahnungseinrichtungen 25, 351 in eine der Wickelrichtung V entgegengesetzte Belastungsrichtung B ist gesperrt.

Bei einem in Fig. 32A, 32B dargestellten Ausführungsbeispiel sind die Zähne der Verzahnungseinrichtungen 25, 351, im Vergleich zum Ausführungsbeispiel gemäß Fig. 31A, 31B, an den Zahnflanken 254 hinterschnitten, sind also schräg zur Schließrichtung X gestellt. Bei einer Belastung der Verzahnungseinrichtungen 25, 351 in eine der Wickelrichtung V entgegengesetzte Belastungsrichtung B sind die Verzahnungseinrichtungen 25, 351 zueinander gesperrt.

Bei den Ausführungsbeispielen gemäß Fig. 29A, 29B, 30A, 30B und 32A, 32B ist der Eingriff der Verzahnungseinrichtungen 25, 351 selbstverstärkend bei Belastung in die Belastungsrichtung B. Die Verschlussteile 2, 3 werden bei Belastung somit in die Schließrichtung X zueinander hin gezogen. Aufgrund des Formschlusses über die Vorsprungselemente 252 bzw. aufgrund des Hinterschnitts ist die Verschlussvorrichtung 1 zudem auch gegen ein Öffnen entgegen der Schließrichtung X gesperrt. Es können insbesondere auch Belastungskräfte aufgenommen und abgeleitet werden, die nicht rein tangential, sondern mit einer Komponente entgegen der Schließrichtung X wirken.

Bei dem Ausführungsbeispiel gemäß Fig. 33A, 33B sind die Zähne der Verzahnungseinrichtungen 25, 351 an beiden Zahnflanke 253, 254 so schräg gestellt, dass sie eine Auflaufschräge ausbilden und die Verzahnungseinrichtungen 25, 351, bei hinreichender Kraftwirkung zwischen den Verzahnungseinrichtungen 25, 351, in die Wickelrichtung V und auch entgegen der Wickelrichtung V übereinander gleiten können, bei axialem Ausweichen der Verzahnungseinrichtungen 25, 351 zueinander. Bei dem Ausführungsbeispiel gemäß Fig. 33A, 33B sind die Verzahnungseinrichtungen 25, 351 somit weder in die Wickelrichtung V noch entgegen der Wickelrichtung V zueinander gesperrt, sondern können ratschend übereinander gleiten, wenn das zwischen den Verzahnungseinrichtungen 25, 351 wirkende Drehmoment hinreichend groß ist.

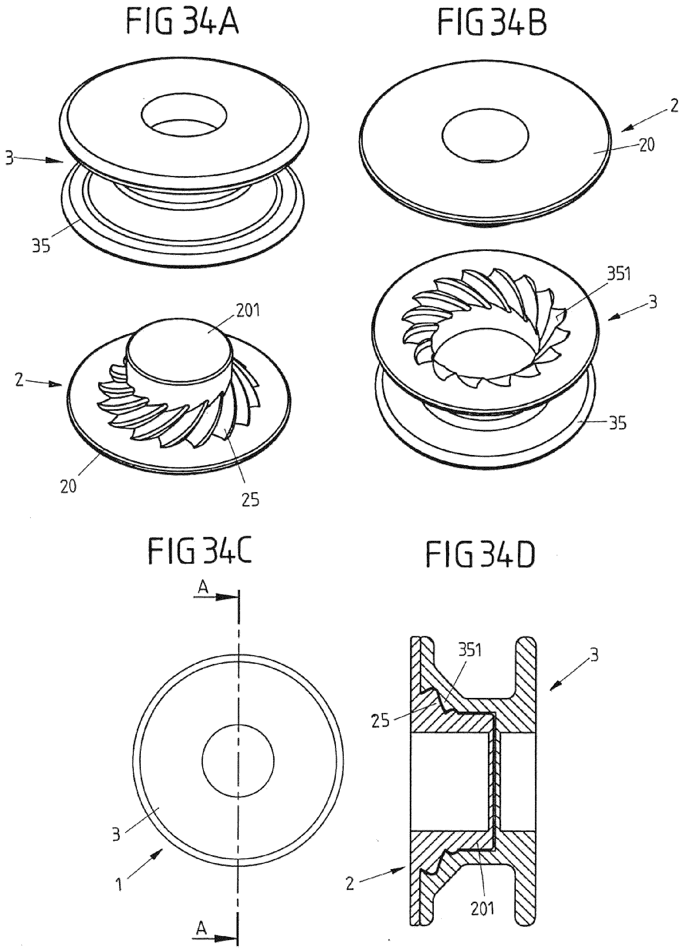

Bei einem in Fig. 34A-34D dargestellten Ausführungsbeispiel sind die Zähne der Verzahnungseinrichtungen 25, 351 wie bei einem Kegelzahnrad an ihren Zahnrücken schräg zur Wickelrichtung V und auch zur Schließrichtung X erstreckt, was die bei Belastung entgegen der Wickelrichtung V miteinander in Eingriff liegenden, zueinander abgestützten Flächen der Zähne der Verzahnungseinrichtungen 25, 351 vergrößern kann.

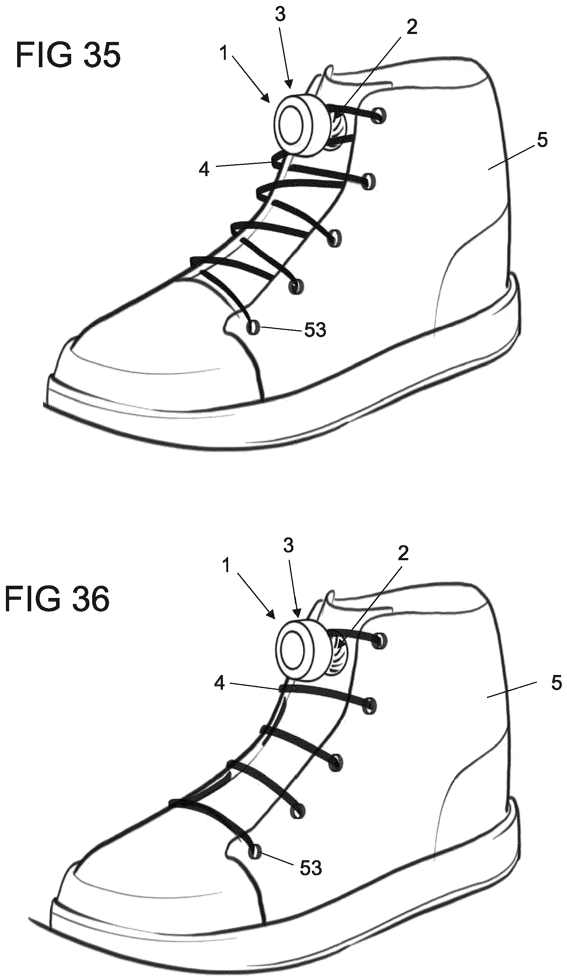

Fig. 35 bis 68 zeigen unterschiedliche Ausführungsbeispiele von Anwendungen einer Verschlussvorrichtung 1, die nach einem Ausführungsbeispiel der vorangehend beschriebenen Art ausgebildet sein kann.

Bei dem Ausführungsbeispiel gemäß Fig. 35 dient die Verschlussvorrichtung 1 zum Schließen und Spannen eines Schuhs 5. Das Verschlussteil 3 kann hierzu an das Verschlussteil 2 an einer Lasche des Schuhs 5 angesetzt werden, um ein Zugelement 4 in Form eines Schnürsenkels zu spannen.

Fig. 36 zeigt ein anderes Ausführungsbeispiel eines Schuhs 5, das sich in der Wicklung des Zugelements 4 in Form des Schnürsenkels von dem Ausführungsbeispiel gemäß Fig. 35 unterscheidet.

Bei dem Ausführungsbeispiel gemäß Fig. 35 und 36 ist ein Ende des Zugelements 4 in Form des Schnürsenkels an dem Verschlussteil 3 der Verschlussvorrichtung 1 festgelegt und kann durch Verdrehen des Betätigungselements 34 zusammen mit dem Wickelelement 35 relativ zu dem Verschlussteil 2 gespannt werden. Das Spannen ist hierbei auch händisch möglich, indem vor Festlegen des Verschlussteils 3 an dem Verschlussteil 2 an dem Verschlussteil 3 gezogen und das Zugelement 4 in Form des Schnürsenkels dadurch gespannt wird.

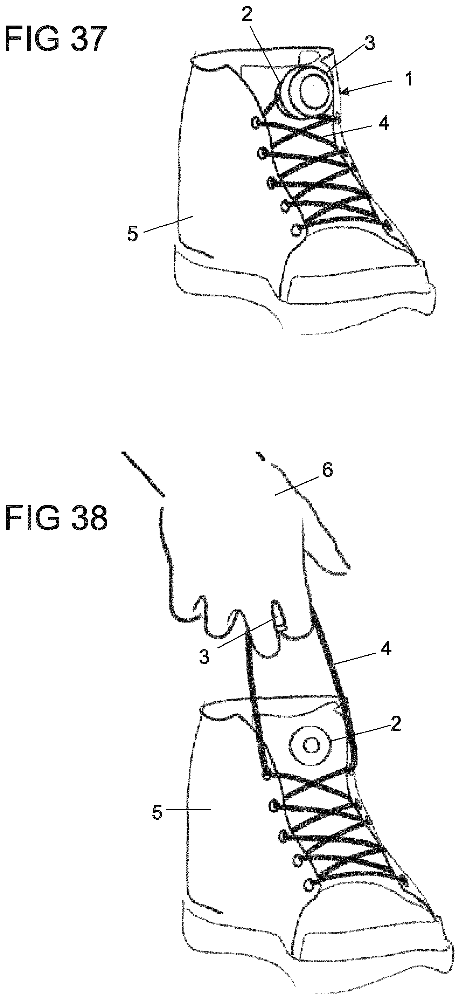

Bei dem Ausführungsbeispiel gemäß Fig. 37 und 38 sind zwei Enden des in diesem Fall als Schlaufe gewickelten Zugelements 4 in Form des Schnürsenkels mit dem Verschlussteil 3 der Verschlussvorrichtung 1 verbunden, sodass durch Verdrehen des Verschlussteils 3 relativ zum Verschlussteil 2 das Zugelement 4 in Form des Schnürsenkels mit zwei Enden aufgewickelt und somit gespannt werden kann. Wie in Fig. 38 dargestellt ist vor Anordnen des Verschlussteils 3 an dem Verschlussteil 2 ein händisches Spannen des Zugelements 4 durch Ziehen an dem Zugelement 4 mit einer Hand 6 möglich.

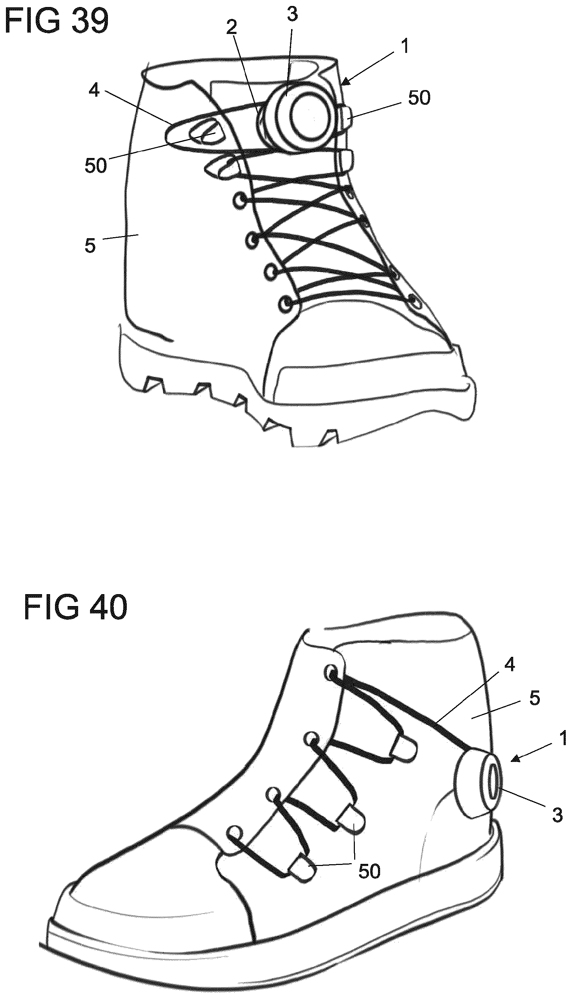

Bei dem Ausführungsbeispiel gemäß Fig. 39 sind wiederum zwei Enden des Zugelements 4 in Form des Schnürsenkels mit dem Verschlussteil 3 der Verschlussvorrichtung 1 verbunden und können somit durch Verdrehen des Verschlussteils 3 relativ zum Verschlussteils 2 zum Schließen und Spannen des Schuhs 5 aufgewickelt werden. Das Zugelement 4 in Form des Schnürsenkels ist hierbei um Spannelemente 50 herum verlegt, sodass der Schuh 5 durch Aufwickeln des Zugelements 4 auf das Wickelelement 35 des Verschlussteils 3 gespannt werden kann.

Fig. 40 zeigt ein anderes Ausführungsbeispiel, bei dem das Zugelement 4 um Spannelemente 50 herumverlegt und mit einem Ende an dem Verschlussteil 3 der Verschlussvorrichtung 1 angeordnet ist und somit über die Verschlussvorrichtung 1 gespannt werden kann.

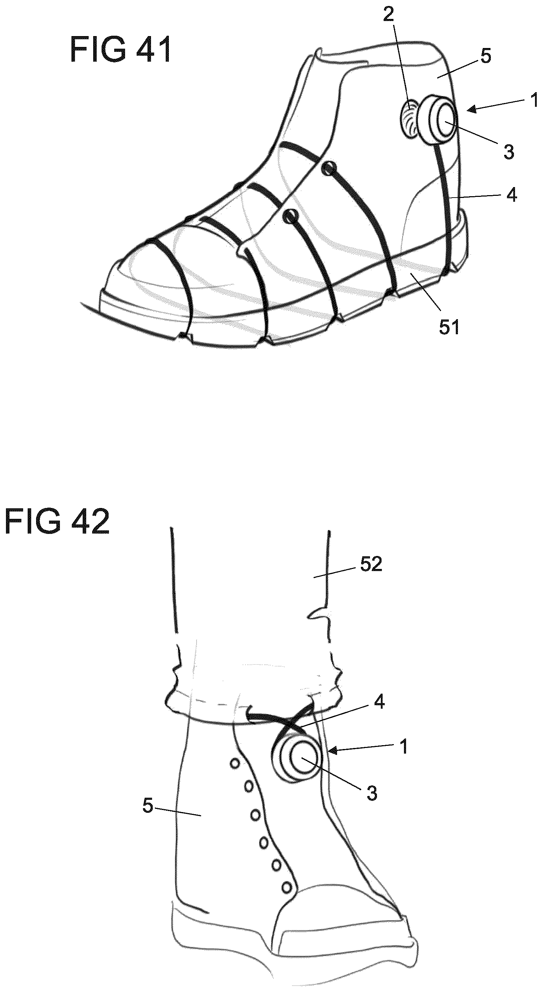

Bei dem Ausführungsbeispiel gemäß Fig. 41 ist das Zugelement 4 in Form des Schnürsenkels (auch) um die Sohle 51 des Schuhs 5 herum verlegt und mit einem Ende mit dem Verschlussteil 3 der Verschlussvorrichtung 1 verbunden, sodass der Schuh 5 über die Verschlussvorrichtung 1 geschlossen und gespannt werden kann.

Bei dem in Fig. 42 dargestellten Ausführungsbeispiel dient die Verschlussvorrichtung 1 zum Verspannen eines Hosenbeins 52 über ein Zugelement 4 in Form eines Bands mit einem Schuh 5. Das Zugelement 4 erstreckt sich hierzu um das Hosenbein 52 herum und ist über die Verschlussvorrichtung 1 an dem Schuh 5 festgelegt, sodass durch Verdrehen des Verschlussteils 3 gegenüber dem Verschlussteil 2 das Hosenbein 52 gegenüber dem Schuh 5 verspannt werden kann.

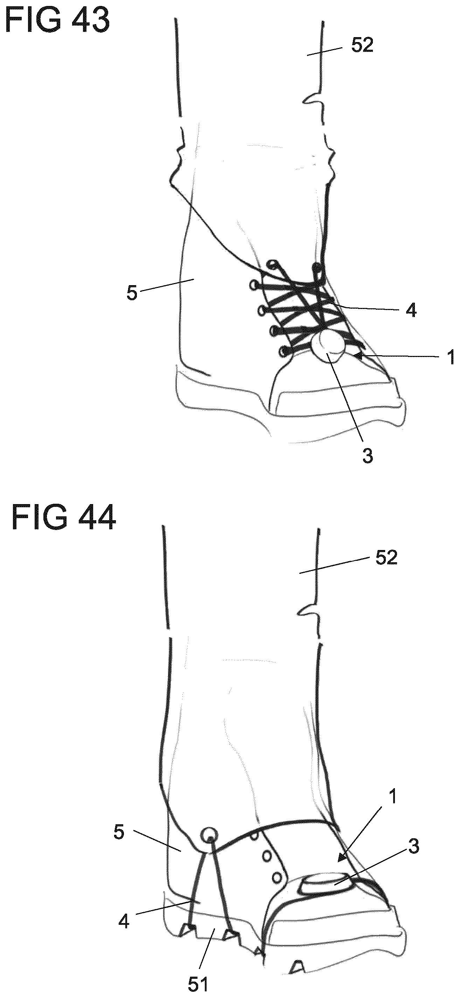

Ein anderes Ausführungsbeispiel zeigt Fig. 43. Bei diesem Ausführungsbeispiel kann ein Hosenbein 52 wiederum gegenüber einem Schuh verspannt werden, indem ein Zugelement 4, das sich durch Ösen im Hosenbein 52 hindurch erstreckt, über die Verschlussvorrichtung 1 gegenüber dem Schuh 5 verspannt werden kann.

Auch bei dem Ausführungsbeispiel gemäß Fig. 44 kann ein Hosenbein 52 gegenüber einem Schuh verspannt werden, wobei sich in diesem Fall das Zugelement 4 in Form eines Bands um die Sohle 51 des Schuhs 5 herum erstreckt und über die Verschlussvorrichtung 1 an dem Schuh 5 verspannt werden kann.

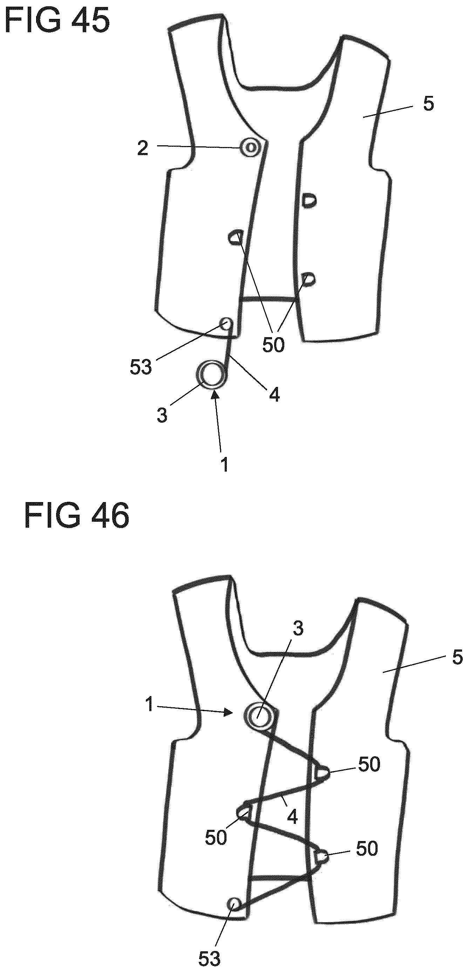

Bei dem Ausführungsbeispiel gemäß Fig. 45 und 46 dient die Verschlussvorrichtung 1 zum Schließen und Spannen eines Bekleidungsstücks 5, zum Beispiel einer Weste oder einer Jacke. Das Zugelement 4 in Form eines Bands oder eines Seils ist an einer Befestigungsstelle 53 an dem Bekleidungsstück 5 festgelegt und kann um Spannelemente 50 beidseits eines Öffnungsschlitzes des Bekleidungsstücks 5 herum verlegt werden, um auf diese Weise das Bekleidungsstück 5 zu schließen, wie dies in Fig. 46 dargestellt ist. Durch Anordnung des Verschlussteils 3 an dem Verschlussteil 2 und durch Verdrehen des Verschlussteils 3 relativ zum Verschlussteil 2 kann sodann das Bekleidungsstück 5 gespannt werden.

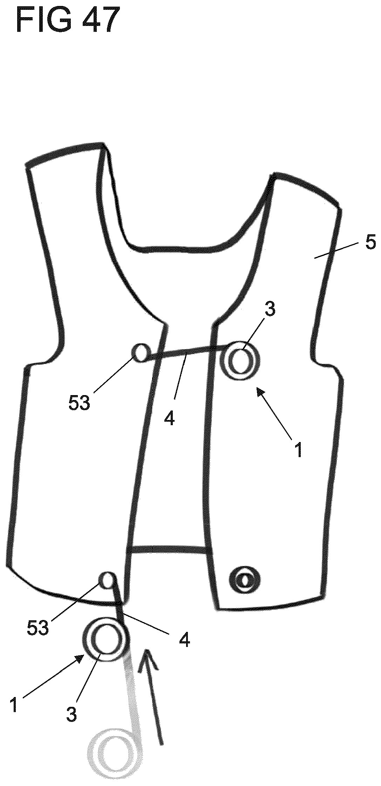

Auch bei dem Ausführungsbeispiel gemäß Fig. 47 dient die Verschlussvorrichtung 1 zum Verschließen eines Bekleidungsstücks 5, wobei in diesem Fall zwei Verschlussvorrichtungen 1 zum Spannen zweier Zugelemente 4 vorgesehen sind. Die Zugelemente 4 sind jeweils auf einer Seite des Öffnungsschlitzes des Bekleidungsstücks 5 an einer Befestigungsstelle 53 festgelegt und können durch Anordnung des jeweiligen Verschlussteils 3 an dem zugeordneten Verschlussteil 2 der Verschlussvorrichtung 1 auf der anderen Seite des Öffnungsschlitzes des Bekleidungsstücks 5 gespannt werden.

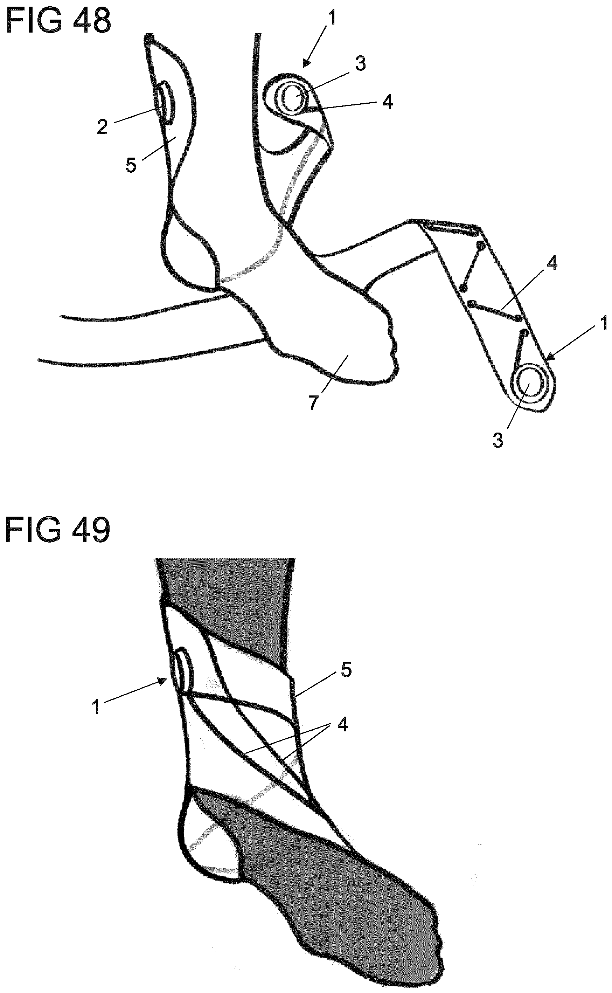

Bei dem Ausführungsbeispiel gemäß Fig. 48 und 49 dient die Verschlussvorrichtung 1 zum Schließen und Spannen eines medizinischen Hilfsmittels 5 in Form einer Knöchelbinde an einem Fuß 7. Durch Anordnen des mit einem Ende der Knöchelbinde verbundenen Verschlussteils 3 an dem mit einem anderen Ende der Knöchelbinde verbundenen Verschlussteil 2 und durch Spannen des an der Knöchelbinde erstreckten Zugelements 4 kann die Knöchelbinde geschlossen und gespannt werden.

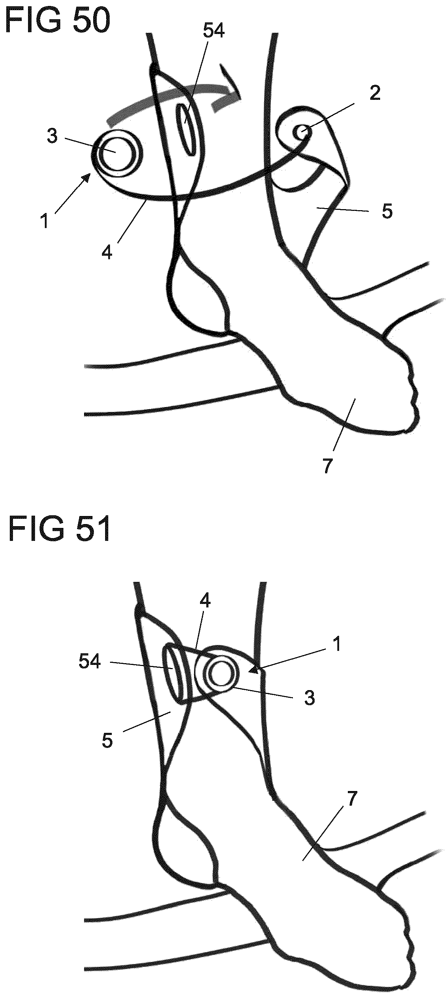

Fig. 50 und 51 zeigen ein anderes Ausführungsbeispiel eines medizinischen Hilfsmittels 5 in Form einer Knöchelbinde, bei der das an einem Ende mit dem Verschlussteil 2 und an einem anderen Ende mit dem Verschlussteil 3 verbundene Zugelement 4 um eine Umlenkung 54 herumverlegt werden kann und durch Ansetzen des Verschlussteils 3 an das Verschlussteil 2 somit eine Schlaufe des Zugelements 4 gebildet wird, die durch Verdrehen des Verschlussteils 3 zum Verschlussteil 2 zum Schließen und Spannen der Knöchelbinde verspannt werden kann.

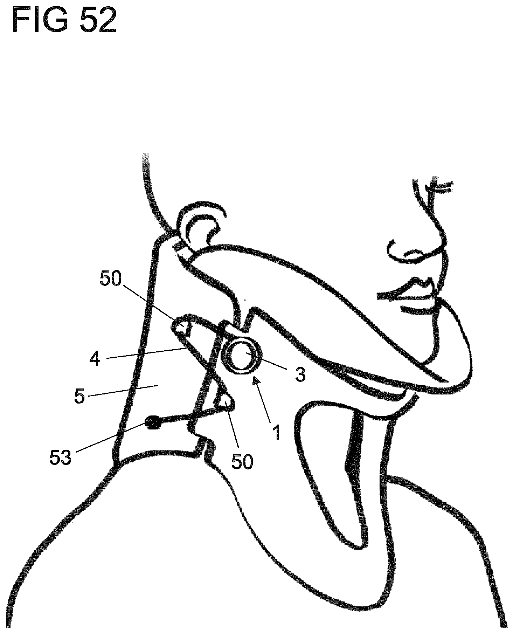

Fig. 52 zeigt ein Ausführungsbeispiel eines medizinischen Hilfsmittels 5 in Form einer als Halskrause ausgebildeten Orthese, bei der die Verschlussvorrichtung 1 zum Schließen und Spannen dient. Ein mit dem Verschlussteil 3 verbundenes Zugelement 4 kann, ausgehend von einer Befestigungsstelle 53, um Spannhaken 50 herumverlegt und durch Verdrehen des Verschlussteils 3 relativ zu dem Verschlussteil 2 gespannt werden.

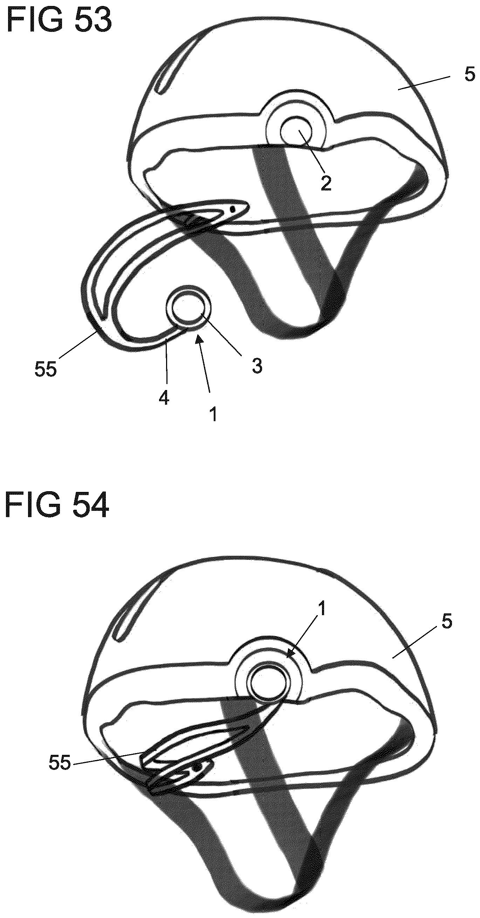

Fig. 53 und 54 zeigen ein Ausführungsbeispiel eines Helms 5, zum Beispiel eines Fahrradhelms, bei dem die Verschlussvorrichtung 1 zum Schließen und Spannen eines Riemens 55 dient. Das Zugelement 4 ist Bestandteil des Riemens 55 und kann auf das Verschlussteil 3, nämlich das Wickelelement 35 des Verschlussteils 3, aufgewickelt werden, um auf diese Weise den Riemen 55 zu spannen.

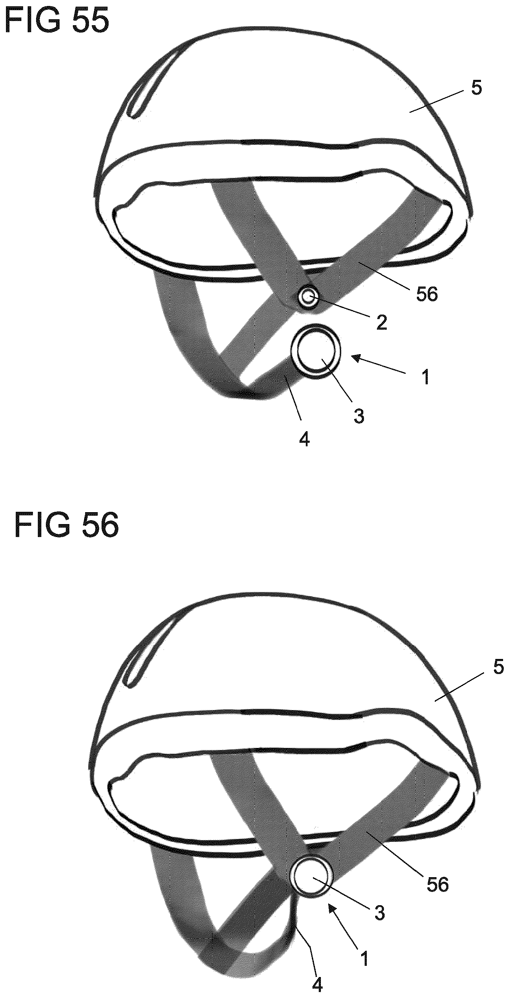

Bei dem Ausführungsbeispiel gemäß Fig. 55 und 56 dient die Verschlussvorrichtung 1 zum Spannen eines Kinngurts 56 eines Helms 5, zum Beispiel eines Fahrradhelms. Das Verschlussteil 3 der Verschlussvorrichtung 1 kann hierbei an dem Verschlussteil 2 angeordnet und zu dem Verschlussteil 2 verdreht werden, um auf diese Weise den Gurt 56 zu spannen.

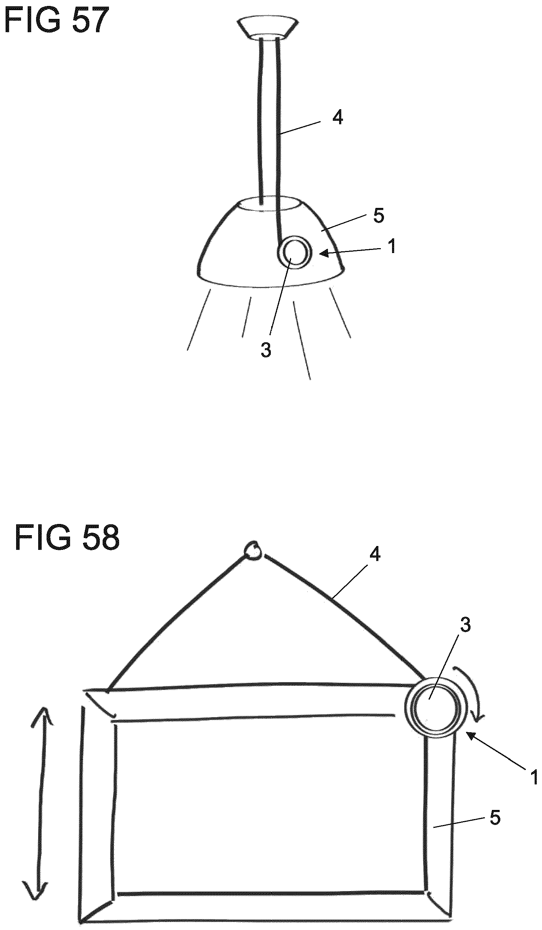

Beim Ausführungsbeispiel gemäß Fig. 57 dient die Verschlussvorrichtung 1 zum Höhenverstellen einer Baugruppe 5 in Form einer Lampe. Das Zugelement 4 dient hierbei zum Aufhängen der Lampe. Durch Verdrehen des Verschlussteils 3 der Verschlussvorrichtung 1 kann die Länge des Zugelements 4 verändert und dadurch die Höhe der Lampe eingestellt werden.

Bei dem Ausführungsbeispiel gemäß Fig. 58 dient die Verschlussvorrichtung 1 zum Aufhängen einer Baugruppe 5 in Form eines Bildes. Durch Verdrehen des Verschlussteils 3 kann die Länge des Zugelements 4 verändert und dadurch die Aufhängung des Bildes angepasst werden.

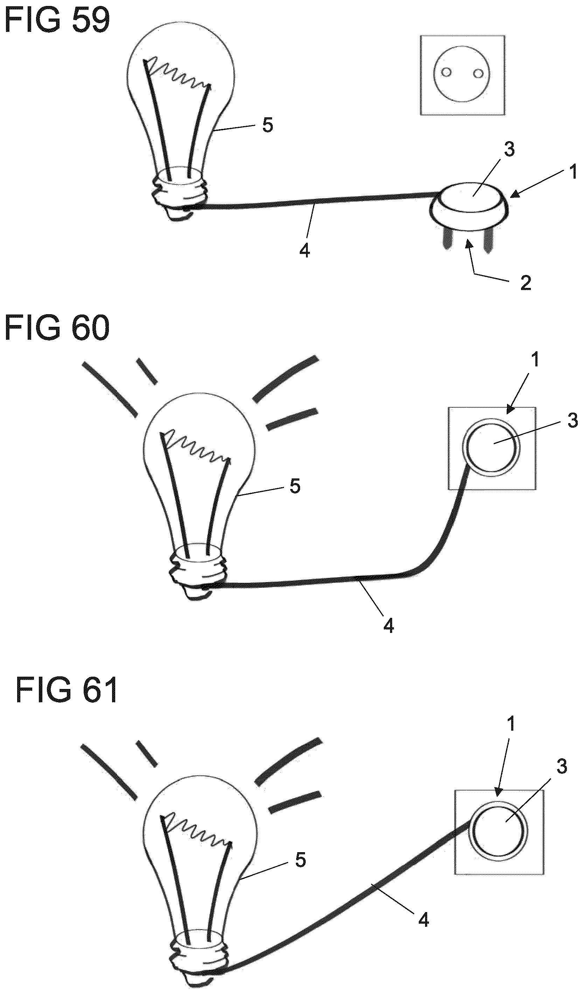

Bei dem in Fig. 59 bis 61 dargestellten Ausführungsbeispiel dient die Verschlussvorrichtung 1 zum elektrischen Anschließen einer Baugruppe in Form einer Lampe 5. Das Verschlussteil 2 ist hierzu als Stecker ausgebildet. Das Verschlussteil 3 kann zu dem Verschlussteil 2 in Form des Steckers verdreht werden, um auf diese Weise die frei erstreckte Länge des das Zugelement 4 verwirklichenden elektrischen Kabels zu verändern.

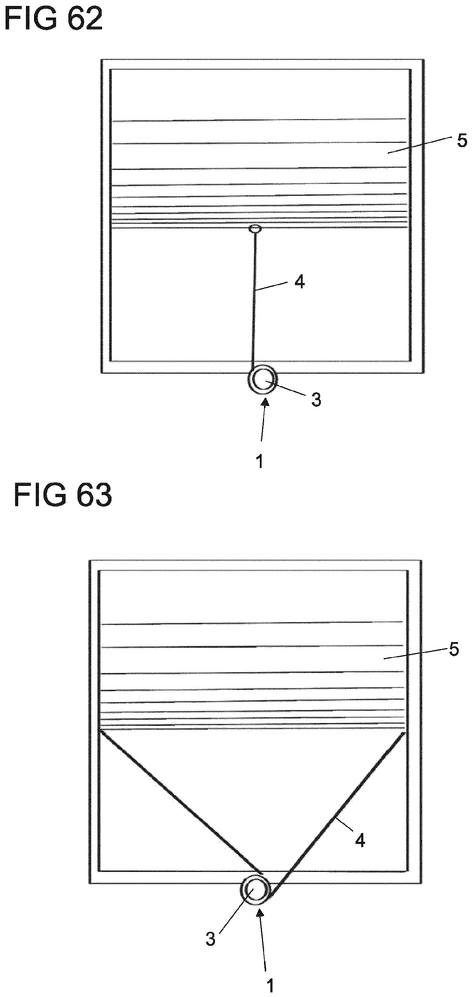

Bei dem Ausführungsbeispiel gemäß Fig. 62 dient die Verschlussvorrichtung 1 zum Spannen einer Baugruppe 5 in Form eines Rollos. Über das Zugelement 4 ist das Verschlussteil 3 mit dem Rollo verbunden. Durch Anordnen an dem zum Beispiel an einem Rahmen angeordneten Verschlussteil 2 und durch Verdrehen des Verschlussteils 3 kann das Zugelement 4 aufgewickelt und das Rollo somit verstellt werden.

Bei dem Ausführungsbeispiel gemäß Fig. 63 sind, im Unterschied zum Ausführungsbeispiel gemäß Fig. 62, zwei Enden des Zugelements 4 mit dem Verschlussteil 3 verbunden, sodass durch Verdrehen des Verschlussteils 3 die zwei Enden des Zugelements 4 gleichzeitig auf das Wickelelement 35 des Verschlussteils 3 aufgewickelt werden können.

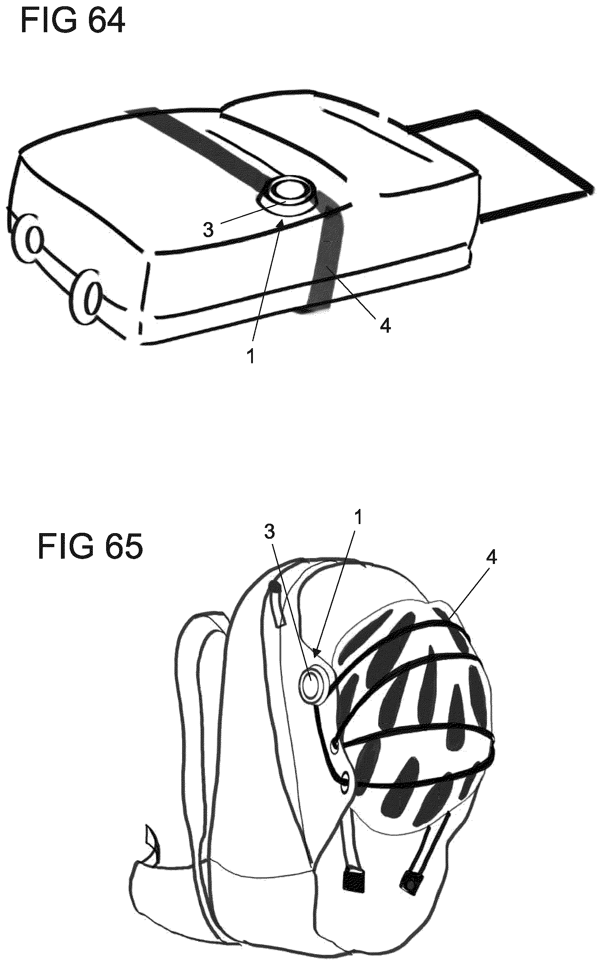

Bei einem in Fig. 64 dargestellten Ausführungsbeispiel dient die Verschlussvorrichtung 1 zum Spannen eines Zugelements 4 in Form eines Gurts an einem Gepäckstück.

Bei einem in Fig. 65 dargestellten Ausführungsbeispiel dient die Verschlussvorrichtung 1 zum Spannen eines Zugelements 4 in Form eines Bands oder eines Seils an einem Gepäckstück in Form eines Rucksacks, um auf diese Weise Gegenstände an dem Gepäckstück zu befestigen.

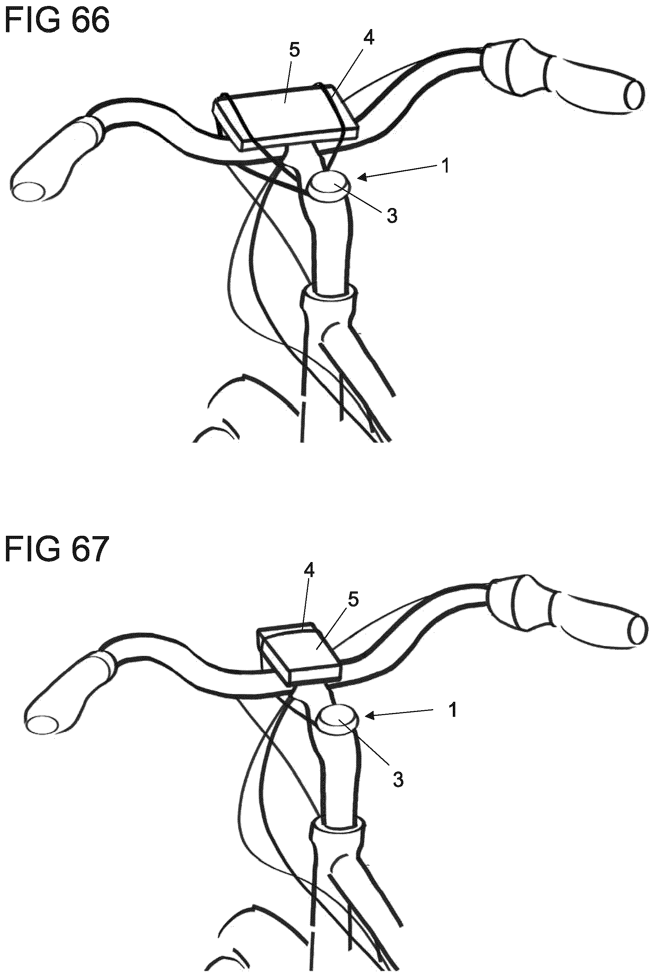

Bei einem in Fig. 66 dargestellten Ausführungsbeispiel dient die Verschlussvorrichtung 1 zum Befestigen eines Gegenstands 5, zum Beispiel eines Mobiltelefons oder dergleichen, an einem Fahrrad, insbesondere einem Lenker eines Fahrrads. Das Zugelement 4 ist hierbei um den Gegenstand 5 herumgeschlungen und kann durch abschnittweises Aufwickeln auf das Wickelelement 35 des Verschlussteils 3 gespannt werden.

Fig. 67 zeigt ein anderes Ausführungsbeispiel mit anderer Wicklung des Zugelements 4 zum Befestigen eines Gegenstands 5, zum Beispiel eines Mobiltelefons, an einem Fahrrad.

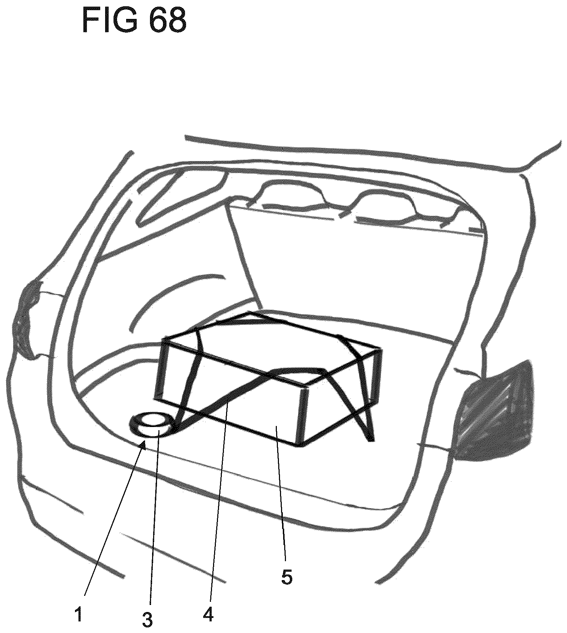

Bei einem in Fig. 68 dargestellten Ausführungsbeispiel dient die Verschlussvorrichtung 1 zum Befestigen eines Gegenstands, zum Beispiel eines Gepäckstücks, in einem Laderaum eines Fahrzeugs. Das Zugelement 4 ist hierbei über das Gepäckstück hinweg verlegt und hält auf diese Weise das Gepäckstück ortsfest am Boden des Laderaums. Über die Verschlussvorrichtung 1 kann das Zugelement 4 zum Befestigen des Gepäckstücks gespannt werden.

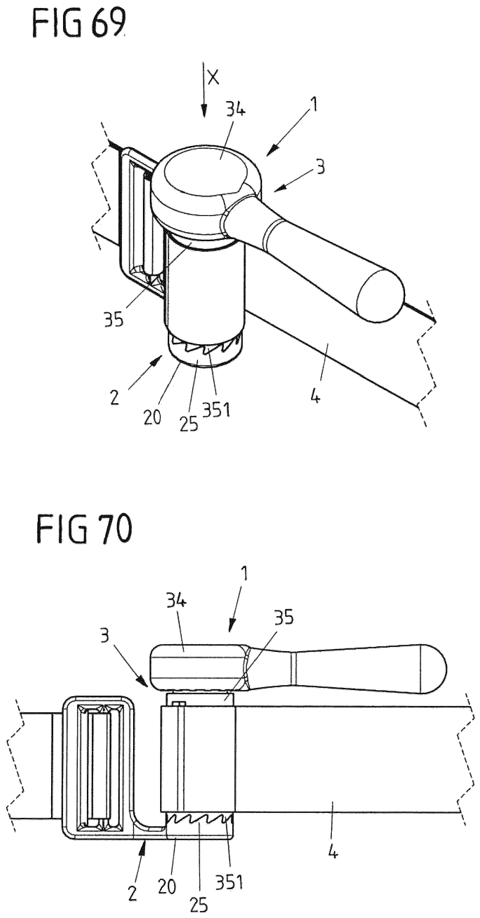

Fig. 69 bis 73 zeigen ein weiteres Ausführungsbeispiel einer Verschlussvorrichtung 1, bei der Verschlussteile 2, 3 entlang einer Schließrichtung X aneinander angesetzt werden können und in einer Schließstellung aneinandergehalten sind.

Bei dem Ausführungsbeispiel gemäß Fig. 69 bis 73 ist das Zugelement 4 als Gurt ausgebildet, der auf ein Wickelelement 35 in Form einer Hülse des Verschlussteils 3 aufgewickelt werden kann. Das Verschlussteil 3 kann an das Verschlussteile 2 angesetzt werden, das beispielsweise eine Gurtschnalle aufweist, sodass über die Verschlussvorrichtung 1 Enden des Zugelements 4 miteinander verbunden und zueinander gespannt werden können.

Das Verschlussteil 3 weist ein Betätigungselement 34 mit einem daran angeformten Handhebel auf, der über eine Eingriffseinrichtung 345 mit einer Eingriffseinrichtung 355 des Wickelelements 35 in Eingriff gebracht werden kann, sodass über das Betätigungselement 34 das Wickelelement 35 in eine Wickelrichtung V gegenüber dem Verschlussteil 2 verdreht werden kann.

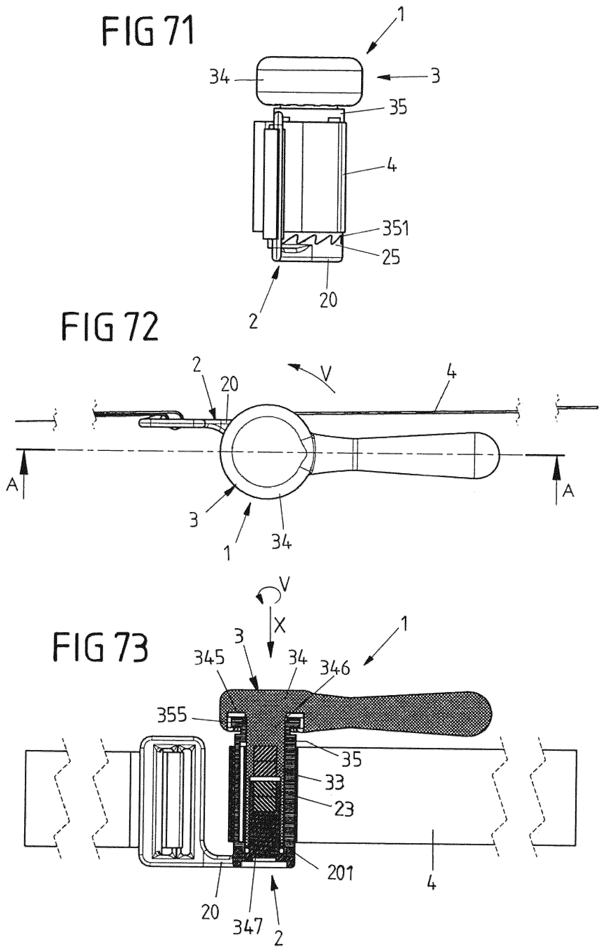

Wie insbesondere aus der Schnittansicht gemäß Fig. 73 ersichtlich, weist das Betätigungselement 34 einen Zapfen 346 mit einer Eingriffsöffnung 347 auf, mit dem das Betätigungselement 34 in die Schließrichtung X auf einen Zylinderabschnitt 201 an einem Körper 20 des Verschlussteils 2 aufgesetzt werden kann, um die Verschlussteile 2, 3 miteinander zu verbinden.

Beispielsweise aufgrund einer Federvorspannung zwischen dem Betätigungselement 34 und dem Wickelelement 35 sind die Eingriffseinrichtungen 345, 355 nicht miteinander in Eingriff, wenn die Verschlussteile 2, 3 voneinander getrennt sind. Das Wickelelement 35 kann somit frei gegenüber dem Betätigungselement 34 verdreht werden, sodass das Zugelement 4 beispielsweise von dem Wickelelement 35 abgewickelt werden kann. Zum Schließen wird das Verschlussteil 3 derart an das Verschlussteil 3 angesetzt, dass die Eingriffsöffnung 347 an dem Zapfen 346 des Betätigungselements 34 mit dem Zylinderabschnitt 201 des Verschlussteils 2 in Eingriff gelangt, wobei das Wickelelement 35 an dem Körper 20 des Verschlussteils 2 abgestützt wird und aufgrund einer magnetischen Anziehung von Magnetelementen 23, 33 an dem Zylinderabschnitt 201 einerseits und dem Zapfen 346 des Betätigungselements 34 andererseits (siehe Fig. 73) das Betätigungselement 34 hin zu dem Wickelelement 35 gezogen und dadurch die Eingriffseinrichtungen 345, 355 formschlüssig miteinander in Eingriff gelangen. Auf diese Weise wird eine Wirkverbindung zwischen dem Betätigungselement 34 und dem Wickelelement 35 hergestellt, sodass bei miteinander verbundenen Verschlussteilen 2, 3 das Betätigungselement 34 und das Wickelelement 35 gemeinsam verdreht werden können, um auf diese Weise das Zugelement 4 auf das Wickelelement 35 aufzuwickeln und dadurch das Zugelement 4 zu spannen.

An einem dem Körper 20 zugewandten Ende weist das Wickelelement 35 eine Verzahnungseinrichtung 351 in Form einer sägezahnförmigen Verzahnung auf, die bei miteinander verbundenen Verschlussteilen 2, 3 mit einer Verzahnungseinrichtung 25 an dem Körper 20 in Eingriff steht. Der Eingriff der Verzahnungseinrichtungen 25, 351 bewirkt, dass die Verschlussteile 2, 3 in die Wickelrichtung V zueinander verdreht werden können, um das Zugelement 4 an dem Wickelelement 35 zu spannen, nicht aber entgegen der Wickelrichtung V.

Zum Lösen der Verschlussvorrichtung 1 können die Verschlussteile 2, 3 entgegen der Schließrichtung X voneinander abgezogen werden, sodass auf diese Weise die Verschlussteile 2, 3 voneinander getrennt werden.

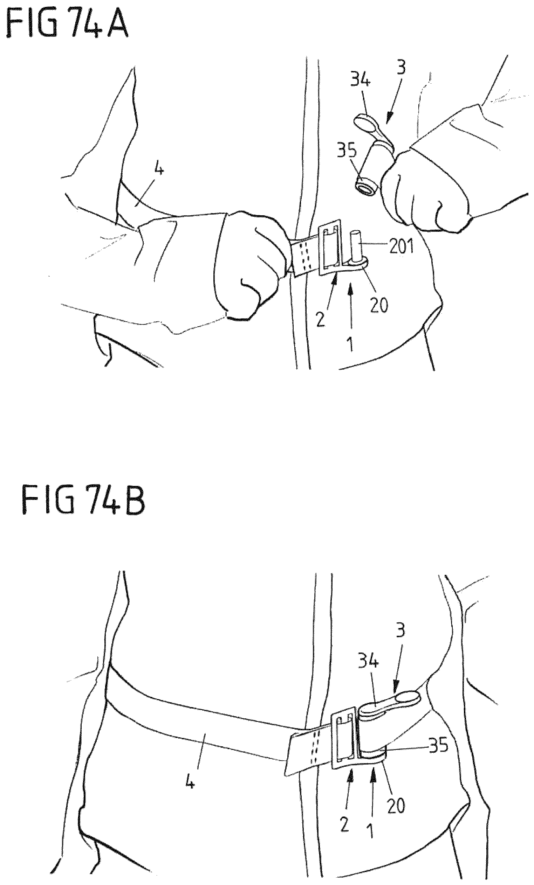

Fig. 74A und 74B zeigen die Verschlussvorrichtung 1 bei getrennten Verschlussteilen 2, 3 (Fig. 74A) und bei geschlossener Verschlussvorrichtung 1 (Fig. 74B). Die Verschlussvorrichtung 1 kann beispielsweise zum Spannen eines Gurts in Form eines Gürtels dienen.

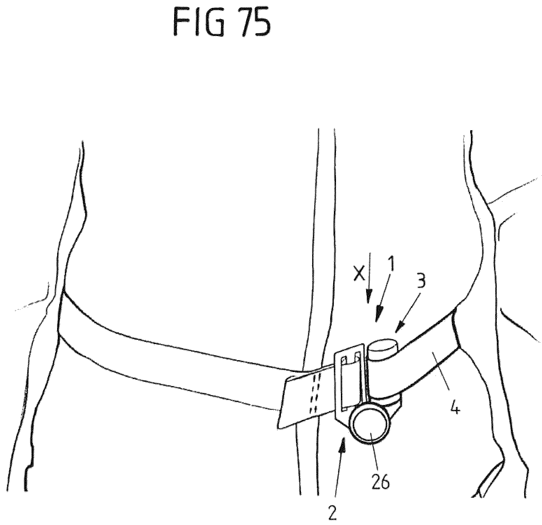

Fig. 75 ein weiteres Ausführungsbeispiel einer Verschlussvorrichtung 1, bei der Verschlussteile 2, 3 in eine Schließrichtung X aneinander angesetzt werden können, analog wie dies vorangehend beschrieben worden ist.

Bei dem Ausführungsbeispiel gemäß Fig. 75 ist ein Getriebe 26 an dem Verschlussteil 2 vorgesehen, das beispielsweise die Gestalt eines Kegelgetriebes oder Schneckengetriebes aufweisen kann und dazu dient, über die Verzahnungseinrichtung 25 (die bei miteinander verbundenen Verschlussteilen 2, 3 mit der Verzahnungseinrichtung 351 des Verschlussteils 3 in Eingriff steht, wie dies zum Beispiel aus Fig. 69 ersichtlich ist) das Verschlussteil 3 relativ zum Verschlussteil 2 zu verdrehen, um auf diese Weise das Zugelement 4 zu spannen. Bei dem Ausführungsbeispiel gemäß Fig. 75 erfolgt ein Verdrehen der Verschlussteile 2, 3 zueinander somit über ein an dem Verschlussteil 2 vorgesehenes Getriebe, das zum Beispiel händisch betätigt werden kann.

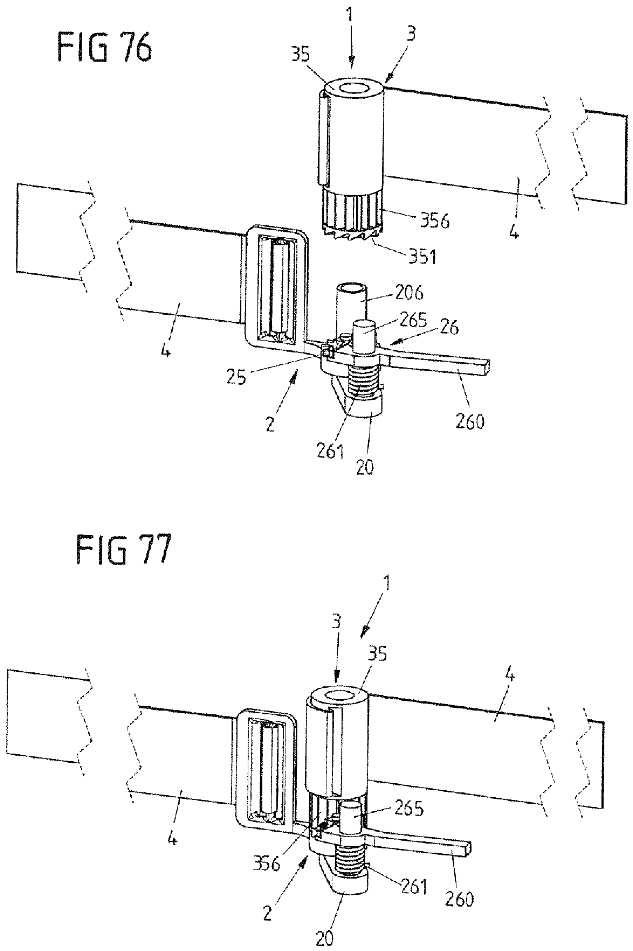

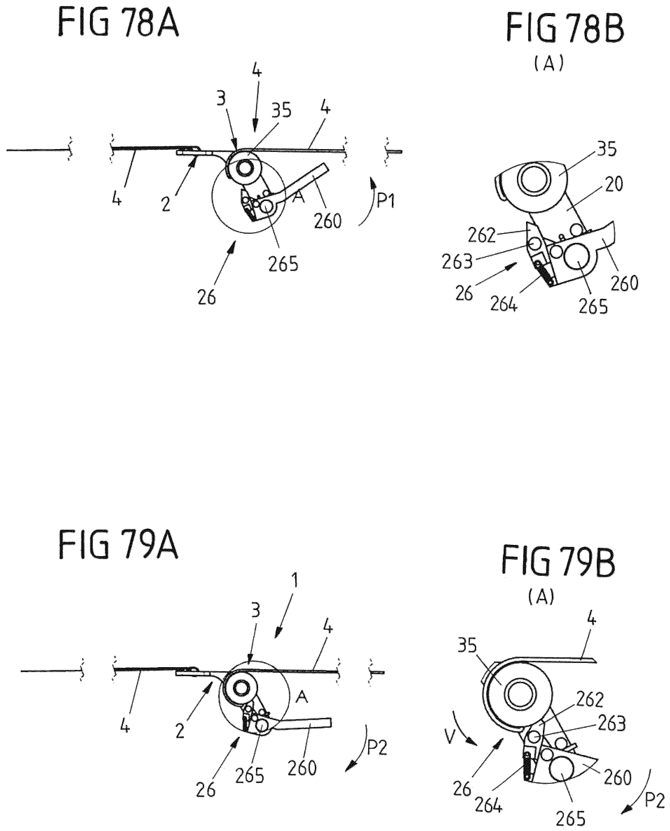

Fig. 76 bis 79A, 79B zeigen ein weiteres Ausführungsbeispiel, bei dem eben ein solches Getriebe 26 zum Spannen des Wickelelements 35 umgesetzt ist.

Das Getriebe 26 ist bei dem Ausführungsbeispiel gemäß Fig. 76 bis 79A, 79B als Spanngetriebe ausgebildet, bei dem ein Spannhebel 260 um eine Schwenkachse 265 verschwenkbar an dem Körper 20 des Verschlussteils 2 gelagert und über ein Federelement 261 gegenüber dem Körper 20 in eine Grundstellung (dargestellt in Fig. 79A, 79B) vorgespannt ist.

Bei dem Ausführungsbeispiel kann das Verschlussteil 3 mit einem Wickelelement 35 auf einen Zylinderabschnitt 201 des Körpers 20 aufgesetzt werden und gelangt, in verbundener Stellung, über eine Verzahnungseinrichtung 351 mit einer zugeordneten Verzahnungseinrichtung 25 des Körpers 20 in Eingriff, wie dies auch vorangehend beschrieben worden ist. An dem Wickelelement 35 ist ein Spanneingriff 356 in Form einer Verzahnung ausgebildet, die um das Wickelelement 35 umläuft und zum Wechselwirken mit dem Getriebe 26 dient.

An dem Spannhebel 260 des Getriebes 26 ist, um eine Schwenkachse 263 schwenkbar, ein Eingriffshebel 262 angeordnet, der zum Eingriff in den Spanneingriff 356 des Wickelelements 35 dient.

Zum Ansetzen der Verschlussteile 2, 3 aneinander kann der Spannhebel 260, wie dies in Fig. 78A, 78B dargestellt ist, aus der Grundstellung in eine Schwenkrichtung P1 ausgelenkt werden, um auf diese Weise den Eingriffshebel 262 aus einem Bereich zu entfernen, den das Wickelelement 35 bei miteinander verbundenen Verschlussteilen 2, 3 einnimmt. Die Verschlussteile 2, 3 können somit ohne weiteres, ohne Behinderung durch das Getriebe 26, aneinander angesetzt werden.

Wird der Spannhebel 260 wieder losgelassen, gelangt das Getriebe 26 mit seinem Spannhebel 260 in die Grundstellung gemäß Fig. 79A, 79B, in der der Eingriffshebel 262 mit dem Spanneingriff 256 in Eingriff steht.

Wird nunmehr der Spannhebel 260 in eine Schwenkrichtung P2 ausgelenkt, so wird der Eingriffshebel 262 mitbewegt und verdreht das Wickelelement 35 in die Wickelrichtung V, sodass das Zugelement 4 auf das Wickelelement 35 aufgewickelt wird. Das Zugelement 4 wird somit gespannt.

Das Spannen erfolgt hierbei schrittweise. Aufgrund des Federelements 261 und der dadurch bereitgestellten Federvorspannung wird der Spannhebel 260 nach einer Betätigung selbsttätig in die Grundstellung zurückgestellt, wobei der Eingriffshebel 262 über den Spanneingriff 356 hinweg begleitet, unter elastischer Auslenkung des Federelements 264, über das der Eingriffshebel 262 zu dem Spannhebel 260 elastisch vorgespannt ist. Das Spannen des Zugelements 4 erfolgt somit schrittweise durch wiederholte Betätigung des Spannhebels 260.

Über das Federelement 264 wird auch ein Freilauf bereitgestellt. Das Wickelelement 35 kann auch per Hand in die Wickelrichtung V verdreht werden. Hierbei gleitet der Eingriff 262 unter elastischer Auslenkung des Federelements 264 über den Spanneingriff 356 des Wickelelements 35.

Der der Erfindung zugrunde liegende Gedanke ist nicht auf die vorangehend geschilderten Ausführungsbeispiele beschränkt, sondern lässt sich auch in gänzlich anders gearteter Weise verwirklichen.

Eine Verschlussvorrichtung der hier beschriebenen Art kombiniert einen mechanischen Verschluss und eine Wickeleinrichtung. Verschlussteile können aneinander angesetzt werden und sind in einer Schließstellung aneinander gehalten. Über ein Wickelelement kann hierbei ein Zugelement aufgewickelt und damit gespannt werden, sodass Baugruppen miteinander verbunden und zueinander gespannt werden können.

Das Zugelement ist generell zum Beispiel als biegeschlaffes Element, das (ausschließlich) zum Übertragen von Zugkräften geeignet ist, ausgebildet. Das Zugelement kann beispielsweise ein Seil, Gurt, Riemen oder Band sein.

Verschlussvorrichtungen der hier beschriebenen Art können als rein mechanische Verschlussvorrichtungen ohne Verwendung von Magnetelementen ausgestaltet sein. Das Verwenden von Magnetelementen kann jedoch vorteilhaft sein, um zum einen eine einfache, intuitive Handhabbarkeit zu erreichen und zum anderen einen Halt zwischen den Verschlussteilen zu verbessern.

Die Verschlussvorrichtung kann grundsätzlich ganz unterschiedliche magnetisch-mechanische Rastverschlüsse verwirklichen. Beispielsweise kann die Verschlussvorrichtung - mit Bezug auf ihre magnetisch-mechanische Rastfunktion - so ausgestaltet sein wie in der WO 2008/006357 A2, der WO 2008/006354 A2, der WO 2009/092368 A2, der WO 2010/006594 A2, der WO 2008/006356 A2, der WO 2009/010049 A2, der WO 2009/127196 A2, der WO 2014/090926 A1 und der internationalen Anmeldung mit dem Aktenzeichen PCT/EP 2013/060762 beschrieben.

### Bezugszeichenliste

- 1: Verschlussvorrichtung
- 2: Verschlussteil
- 20: Grundkörper
- 200: Seitenwandung
- 201: Zylinderbund
- 202: Aussparungen
- 203: Rasteingriff (Rastnut)
- 21: Hinterschnittelement
- 22: Hinterschnittelement
- 23: Magnetelement
- 24: Blockierelement
- 240: Achselement
- 241: Federelement
- 25: Verzahnungseinrichtung
- 250: Verzahnungselement
- 251: Ring
- 252: Vorsprungselement
- 253: Auflaufschräge
- 254: Zahnflanke
- 255: Rastnase
- 26: Getriebeeinrichtung
- 260: Spannhebel
- 261: Federelement
- 262: Eingriffshebel
- 263: Schwenkachse
- 264: Federelement
- 265: Schwenkachse
- 3: Verschlussteil
- 30: Gehäuseelement
- 300: Zylinderbund
- 301: Innenraum
- 302: Verzahnung
- 303: Dom
- 304: Öffnung
- 305: Rastnut
- 31: Hinterschnittelement
- 32: Hinterschnittelement
- 33: Magnetelement
- 34: Betätigungselement
- 340: Körper
- 341: Verzahnungseinrichtung
- 342: Zapfen
- 343: Sperrelement
- 344: Federelement
- 345: Eingriffseinrichtung
- 346: Zapfen
- 347: Öffnung
- 35: Wickelelement
- 350: Wickelkörper
- 351: Verzahnungseinrichtung
- 352: Öffnung
- 353: Rille
- 355: Eingriffseinrichtung
- 356: Spanneingriff
- 36: Rastelement
- 360: Rastvorsprung
- 37: Rasteinrichtung
- 370: Rastelement
- 371: Rastvorsprung
- 372: Auflaufelement
- 373: Vorspannelement (Ring)
- 374: Federelement
- 375: Bedienelement
- 376: Auflaufelement
- 38: Eingriffselement
- 380: Körper
- 381: Ringbund
- 382: Aufnahmeöffnung
- 383: Öffnung
- 384: Federelement
- 385: Rastelement
- 386: Federelement
- 387: Öffnung
- 39: Bedienelement
- 390: Körper
- 391: Ringbund
- 392: Entsperröffnung
- 393: Öffnung
- 394: Federelement
- 4: Zugelement
- 5: Baugruppe
- 50: Spannhaken
- 51: Sohle
- 52: Hosenbein
- 53: Befestigungsstelle
- 54: Umlenkung
- 55: Riemen
- 56: Gurt
- 6: Hand
- 7: Fuß
- B: Belastungsrichtung
- D: Betätigungsrichtung
- E: Eingriffsrichtung
- L: Löserichtung
- P1, P2: Schwenkrichtung
- V: Wickelrichtung
- X: Schließrichtung
- Y: Öffnungsrichtung

## Patentansprüche

1. Verschlussvorrichtung (1), mit einem ersten Verschlussteil (2) und einem zweiten Verschlussteil (3), die entlang einer Schließrichtung (X) aneinander ansetzbar, in einer Schließstellung aneinander gehalten und zum Öffnen der Verschlussvorrichtung (1) voneinander lösbar sind,
wobei das zweite Verschlussteil (3) ein Wickelelement (35) aufweist, an dem ein Zugelement (4) anordbar ist und das relativ zum ersten Verschlussteil (2) zum Aufwickeln des Zugelements (4) auf das Wickelelement (35) in eine Wickelrichtung (V) verdrehbar ist,
wobei eines der Verschlussteile (2, 3) einen Zylinderbund (201) oder einen Zylinderabschnitt (201) aufweist, der in das andere der Verschlussteile (3, 2) zum drehbaren Lagern der Verschlussteile (2, 3) aneinander eingreift,
die Verschlussvorrichtung ist **dadurch gekennzeichnet dass** das erste Verschlussteil (2) und das zweite Verschlussteil (3) jeweils zumindest ein Magnetelement (23, 33) zum Bereitstellen einer magnetischen Anziehungskraft beim Ansetzen der Verschlussteile (2, 3) aneinander aufweisen.

2. Verschlussvorrichtung (1) nach Anspruch 1, **dadurch gekennzeichnet, dass** das Wickelelement (35) um die Schließrichtung (X) relativ zum ersten Verschlussteil (2) verdrehbar ist.

3. Verschlussvorrichtung (1) nach Anspruch 1 oder 2, **dadurch gekennzeichnet, dass** das Wickelelement (35) in der Schließstellung relativ zum ersten Verschlussteil (2) verdrehbar ist.

4. Verschlussvorrichtung (1) nach einem der Ansprüche 1 bis 3, **dadurch gekennzeichnet, dass** das Wickelelement (35) in der Schließstellung in die Wickelrichtung (V), nicht aber entgegen der Wickelrichtung (V) relativ zum ersten Verschlussteil (2) verdrehbar ist.

5. Verschlussvorrichtung (1) nach Anspruch 1 oder 2, **dadurch gekennzeichnet, dass** das Wickelelement (35) in der Schließstellung nicht gegenüber dem ersten Verschlussteil (2) verdrehbar ist.

6. Verschlussvorrichtung (1) nach einem der vorangehenden Ansprüche, **dadurch gekennzeichnet, dass** das Wickelelement (35) eine Verzahnungseinrichtung (351) aufweist, die in der Schließstellung mit einer Verzahnungseinrichtung (25) des ersten Verschlussteils (2) in Eingriff steht.

7. Verschlussvorrichtung (1) nach Anspruch 6, **dadurch gekennzeichnet, dass** die Verzahnungseinrichtung (351) des Wickelelements (35) in der Schließstellung in die Wickelrichtung (V) zu der Verzahnungseinrichtung (25) des ersten Verschlussteils (2) bewegbar ist, eine Bewegung entgegen der Wickelrichtung (V) jedoch gesperrt ist.

8. Verschlussvorrichtung (1) nach Anspruch 6 oder 7, **dadurch gekennzeichnet, dass** zumindest eine der Verzahnungseinrichtungen (351) zumindest ein Verzahnungselement (250) aufweist, das bei Verdrehen des Wickelelements (35) in die Wickelrichtung (V) quer zur Wickelrichtung (V) beiseite drängbar ist.

9. Verschlussvorrichtung (1) nach einem der vorangehenden Ansprüche, **dadurch gekennzeichnet, dass** das erste Verschlussteil (2) und das zweite Verschlussteil (3) in der Schließstellung zum Halten der Verschlussteile (2, 3) entgegen der Schließrichtung (X) aneinander mechanisch miteinander verrastet sind.

10. Verschlussvorrichtung (1) nach Anspruch 9, **dadurch gekennzeichnet, dass** eines der Verschlussteile (2, 3) eine Rasteinrichtung (37) mit zumindest einem beweglichen Rastelement (371) aufweist, das in einer verrasteten Stellung in einen Rasteingriff (203) des anderen Verschlussteils (3, 2) eingreift und dadurch die Verschlussteile (2, 3) entgegen der Schließrichtung (X) aneinander hält.

11. Verschlussvorrichtung (1) nach Anspruch 10, **dadurch gekennzeichnet, dass** die Rasteinrichtung (37) bei Ansetzen der Verschlussteile (2, 3) selbsttätig in die verrastete Stellung gelangt.

12. Verschlussvorrichtung (1) nach Anspruch 10 oder 11, **dadurch gekennzeichnet, dass** das zumindest eine verstellbare Rastelement (371) in Richtung der verrasteten Stellung federvorgespannt ist.

13. Verschlussvorrichtung (1) nach einem der Ansprüche 10 bis 12, **dadurch gekennzeichnet, dass** die Rasteinrichtung (37) ein Bedienelement (375) aufweist, das betätigbar ist, um das zumindest eine Rastelement (371) außer Eingriff von dem Rasteingriff zu bringen.

14. Verschlussvorrichtung (1) nach Anspruch 13, **dadurch gekennzeichnet, dass** das Bedienelement (375) ein Auflaufelement (376) aufweist, das ausgebildet ist, bei Betätigung des Bedienelements (375) auf ein Auflaufelement (372) an einem das zumindest eine Rastelement (371) in Richtung der verrasteten Stellung vorspannenden Vorspannelement (373) aufzulaufen, um das zumindest eine Rastelement (371) dadurch zu verstellen.

## Claims

1. A closure device (1), comprising a first closure part (2) and a second closure part (3) which can be attached to each other along a closing direction (X), are held at each other in a closed position, and can be released from each other for opening the closure device (1),
wherein the second closure part (3) includes a winding element (35) on which a traction element (4) can be arranged and which can be rotated relative to the first closure part (2) in a winding direction (V) for winding the traction element (4) onto the winding element (35),
wherein one of the closure parts (2, 3) includes a cylindrical collar (201) or a cylindrical portion (201) which engages into the other one of the closure parts (3, 2) for rotatably mounting the closure parts (2, 3) on each other,
the closure device is **characterized in that** the first closure part (2) and the second closure part (3) each include at least one magnetic element (23, 33) for providing a force of magnetic attraction on attachment of the closure parts (2, 3) to each other.

2. The closure device (1) according to claim 1, **characterized in that** the winding element (35) can be rotated relative to the first closure part (2) about the closing direction (X).

3. The closure device (1) according to claim 1 or 2, **characterized in that** in the closed position the winding element (35) can be rotated relative to the first closure part (2).

4. The closure device (1) according to any of claims 1 to 3, **characterized in that** in the closed position the winding element (35) can be rotated relative to the first closure part (2) in the winding direction (V), but not against the winding direction (V).

5. The closure device (1) according to claim 1 or 2, **characterized in that** in the closed position the winding element (35) cannot be rotated with respect to the first closure part (2).

6. The closure device (1) according to any of the preceding claims, **characterized in that** the winding element (35) includes a gearing device (351) which in the closed position is in engagement with a gearing device (25) of the first closure part (2).

7. The closure device (1) according to claim 6, **characterized in that** in the closed position the gearing device (351) of the winding element (35) can be moved in the winding direction (V) to the gearing device (25) of the first closure part (2), but a movement against the winding direction (V) is blocked.

8. The closure device (1) according to claim 6 or 7, **characterized in that** at least one of the gearing devices (351) includes at least one gearing element (250) which on rotation of the winding element (35) in the winding direction (V) can be urged aside transversely to the winding direction (V).

9. The closure device (1) according to any of the preceding claims, **characterized in that** in the closed position the first closure part (2) and the second closure part (3) are mechanically latched with each other to hold the closure parts (2, 3) at each other against the closing direction (X).

10. The closure device (1) according to claim 9, **characterized in that** one of the closure parts (2, 3) includes a latching device (37) with at least one movable latching element (371) which in a latched position engages into a latching receptacle (203) of the other closure part (3, 2) and thereby holds the closure parts (2, 3) at each other against the closing direction (X).

11. The closure device (1) according to claim 10, **characterized in that** on attachment of the closure parts (2, 3) the latching device (37) automatically gets into the latched position.

12. The closure device (1) according to claim 10 or 11, **characterized in that** the at least one adjustable latching element (371) is spring-biased in the direction of the latched position.

13. The closure device (1) according to any of claims 10 to 12, **characterized in that** the latching device (37) includes an operating element (375) which can be actuated in order to bring the at least one latching element (371) out of engagement with the latching receptacle.

14. The closure device (1) according claim 13, **characterized in that** the operating element (375) includes a run-up element (376) which on actuation of the operating element (375) is configured to run up on a run-up element (372) on a pretensioning element (373) pretensioning the at least one latching element (371) in the direction of the latched position, in order to thereby adjust the at least one latching element (371).

## Revendications

1. Dispositif de fermeture (1), comportant une première partie de fermeture (2) et une deuxième partie de fermeture (3), qui peuvent être appliquées l'une contre l'autre le long d'une direction de fermeture (X), sont maintenues l'une contre l'autre dans une position fermée et peuvent être détachées l'une de l'autre pour ouvrir le dispositif de fermeture (1),
la deuxième partie de fermeture (3) présentant un élément d'enroulement (35) sur lequel peut être agencé un élément de traction (4) et qui peut être tourné dans une direction d'enroulement (V) par rapport à la première partie de fermeture (2) pour enrouler l'élément de traction (4) sur l'élément d'enroulement (35),
l'une des parties de fermeture (2, 3) présentant un collet cylindrique (201) ou une partie cylindrique (201) qui s'engage dans l'autre des parties de fermeture (3, 2) pour le montage rotatif des parties de fermeture (2, 3) l'une sur l'autre,
le dispositif de fermeture est **caractérisé en ce que** la première partie de fermeture (2) et la deuxième partie de fermeture (3) présentent chacune au moins un élément magnétique (23, 33) pour fournir une force d'attraction magnétique lors de la mise en place des parties de fermeture (2, 3) l'une contre l'autre.

2. Dispositif de fermeture (1) selon la revendication 1, **caractérisé en ce que** l'élément d'enroulement (35) peut être tourné par rapport à la première partie de fermeture (2) autour de la direction de fermeture (X).

3. Dispositif de fermeture (1) selon la revendication 1 ou 2, **caractérisé en ce que** l'élément d'enroulement (35) peut être tourné par rapport à la première partie de fermeture (2) dans la position fermée.

4. Dispositif de fermeture (1) selon l'une quelconque des revendications 1 à 3, **caractérisé en ce que** dans la position fermée l'élément d'enroulement (35) peut être tourné par rapport à la première partie de fermeture (2) dans la direction d'enroulement (V), mais pas contrairement à la direction d'enroulement (V).

5. Dispositif de fermeture (1) selon la revendication 1 ou 2, **caractérisé en ce que** dans la position fermée l'élément d'enroulement (35) ne peut pas être tourné par rapport à la première partie de fermeture (2).

6. Dispositif de fermeture (1) selon l'une quelconque de revendications précédentes, **caractérisé en ce que** l'élément d'enroulement (35) présente un dispositif d'engrènement (351) qui, dans la position fermée, est en prise avec un dispositif d'engrènement (25) de la première partie de fermeture (2).

7. Dispositif de fermeture (1) selon la revendication 6, **caractérisé en ce que** le dispositif d'engrènement (351) de l'élément d'enroulement (35) peut, dans la position fermée, se déplacer dans la direction d'enroulement (V) vers le dispositif d'engrènement (25) de la première partie de fermeture (2), mais qu'un mouvement dans la direction opposée à la direction d'enroulement (V) est bloqué.

8. Dispositif de fermeture (1) selon la revendication 6 ou 7, **caractérisé en ce qu'**au moins l'un des dispositifs d'engrènement (351) comporte au moins un élément d'engrènement (250) qui, lors de la rotation de l'élément d'enroulement (35) dans la direction d'enroulement (V), peut être repoussé sur le côté, transversalement à la direction d'enroulement (V).

9. Dispositif de fermeture (1) selon l'une quelconque des revendications précédentes, **caractérisé en ce que** dans la position fermée la première partie de fermeture (2) et la deuxième partie de fermeture (3) sont encliquetées mécaniquement l'une avec l'autre pour maintenir les parties de fermeture (2, 3) l'une á l'autre contre la direction de fermeture (X).

10. Dispositif de fermeture (1) selon la revendication 9, **caractérisé en ce que** l'une des parties de fermeture (2, 3) présente un dispositif d'encliquetage (37) avec au moins un élément d'encliquetage mobile (371) qui, dans une position verrouillée, est en prise avec un logement d'encliquetage (203) de l'autre partie de fermeture (3, 2) et maintient ainsi les parties de fermeture (2, 3) l'une à l'autre contre la direction de fermeture (X).

11. Dispositif de fermeture (1) selon la revendication 10, **caractérisé en ce que** le dispositif d'encliquetage (37) se déplace automatiquement dans la position verrouillée lorsque les parties de fermeture (2, 3) sont attachées.

12. Dispositif de fermeture (1) selon la revendication 10 ou 11, **caractérisé en ce que** l'au moins un élément d'encliquetage (371) ajustable est précontraint par ressort en direction de la position verrouillée.

13. Dispositif de fermeture (1) selon l'une quelconque des revendications 10 à 12, **caractérisé en ce que** le dispositif de verrouillage (37) présente un élément de commande (375) qui peut être actionné pour désengager l'au moins un élément de verrouillage (371) du logement d'encliquetage.

14. Dispositif de fermeture (1) selon la revendication 13, **caractérisé en ce que** l'élément de commande (375) comporte un élément d'appui (376) qui est conçu pour venir buter, lors de l'actionnement de l'élément de commande (375), contre un élément d'appui (372) situé sur un élément de précontrainte (373) précontraignant ledit au moins un élément de verrouillage (371) en direction de la position verrouillée, afin de déplacer ainsi ledit au moins un élément de verrouillage (371).
